# EUROPEAN PATENT SPECIFICATION

(11) **EP 3 939 594 B1**
(45) Date of publication and mention of the grant of the patent: **12.08.2026**
(21) Application number: 21183202.7
(22) Date of filing: 12.09.2014
(51) Int. Cl.: A61K 31/519, C07D 471/14, A61P 35/00

(54) **7-BENZYL-4-(2-METHYLBENZYL)-2,4,6,7,8,9-HEXAHYDROIMIDAZO[1,2-A]PYRIDO[3,4-E]PYRIMIDIN-5(1H)-ONE FOR USE IN THE TREATMENT OF CANCER**
7-BENZYL-4-(2-METHYLBENZYL)-2,4,6,7,8,9-HEXAHYDROIMIDAZO[1,2-A]PYRIDO[3,4-E]PYRIMIDIN-5(1H)-ON ZUR VERWENDUNG IN DER BEHANDLUNG VON KREBS
7-BENZYL-4-(2-MÉTHYLBENZYL)-2,4,6,7,8,9-HEXAHYDROIMIDAZO[1,2-A]PYRIDO[3,4-E]PYRIMIDIN-5(1H)-ONE POUR UTILISATION DANS LE TRAITEMENT DU CANCER

(30) Priority: 15.11.2013 US 201361904718 P; 13.03.2014 US 201414208657; 25.07.2014 US 201414341392; 25.07.2014 WO PCT/US2014/048241
(43) Date of publication of application: 19.01.2022
(60) Divisional application: 26190229.0
(62) Document number(s) of the earlier application(s) in accordance with Art. 76 EPC: 19158371.5 / 3 546 461; 14861931.5 / 3 068 401
(73) Proprietor: Oncoceutics, Inc., Philadelphia, PA 19104 (US)
(72) Inventor: STOGNIEW, Martin, Blue Pell, 19422 (US); ALLEN, Joshua E., New Haven, 06511 (US); POTTORF, Richard S., Indianapolis, 46526 (US); NALLAGANCHU, Bhaskara Rao, Hillsborough, 08852 (US); OLSON, Gary L., Mountainside, 07092 (US)
(74) Representative: Potter Clarkson

(56) References cited:
- WO-A1-2015/153468
- WO-A2-2012/149546
- DE-A1- 2 150 062
- WO 2016/123571, ONCOCEUTICS, INC., 4 August 2016 (2016-08-04)
- WO 2017/132661, ONCOCEUTICS, INC., 3 August 2017 (2017-08-03)
- ALLEN, JOSHUA E. ET AL: "Dual inactivation of Akt and ERK by TIC10 signals Foxo3a nuclear translocation, TRAIL gene induction, and potent antitumor effects", SCIENCE TRANSLATIONAL MEDICINE , 5(171), 171RA17, 14 PP. CODEN: STMCBQ; ISSN: 1946-6242, 6 February 2013 (2013-02-06), XP002761508, DOI: 10.1126/SCITRANSLMED.3004828
- NICHOLAS T. JACOB ET AL: "Pharmacophore Reassignment for Induction of the Immunosurveillance Cytokine TRAIL", ANGEWANDTE CHEMIE INTERNATIONAL EDITION, vol. 53, no. 26, 23 June 2014 (2014-06-23), DE, pages 6628 - 6631, XP055259380, ISSN: 1433-7851, DOI: 10.1002/anie.201402133

## Description

### BACKGROUND OF THE INVENTION

TNF-related apoptosis-inducing ligand (TRAIL; Apo2L) is an endogenous protein that selectively induces apoptosis in cancer cells. TRAIL is a powerful inducer of apoptosis in a wide range of human cancer cell lines via pro-apoptotic death receptor 4 (DR4; TRAIL-R1) and death receptor 5 (DR5; TRAIL-R2) at the cell surface through engagement of the extrinsic or intrinsic apoptotic pathways. TRAIL plays a direct role in tumor suppression during immune surveillance but this anti-tumor mechanism is lost during the disease progression. The ability of TRAIL to initiate apoptosis selectively in cancer cells has led to ongoing clinical trials with administration of recombinant TRAIL and the longer-lived TRAIL-agonist antibodies targeting either of its two pro-apoptotic death receptors.

Despite its potency, recombinant TRAIL has efficacy-limiting properties such as short serum half-life, stability, cost, and delivery. Delivery of recombinant TRAIL or TRAIL-agonist antibodies to the brain is limited by inability of recombinant TRAIL and TRAIL-agonist antibodies to cross the blood-brain barrier. Accordingly, there is a continuing need for anti-cancer compositions and methods.
N. T. Jacob et al., Angew. Chem. Int. Ed., 2014, 53, 6628-6631 describes a pharmacophore reassignment for induction of the immunosurveillance cytokike TRAIL.

### BRIEF SUMMARY OF THE INVENTION

The present invention provides compound (1): 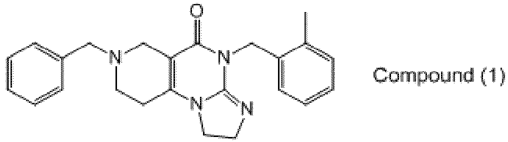 di-HCl for use in the treatment of one or more diseases selected from the group consisting of brain cancer, glioblastoma, astrocytoma, atypical teratoid tumors, atypical rhabdoid tumors, brain tumors, spinal cord tumors, central nervous system atypical teratoid tumors, central nervous system atypical rhabdoid tumors, leptomeningeal disease, central nervous system embryonal tumors, central nervous system lymphoma, chordoma, craniopharyngioma, embryonal tumors of central nervous system, ependymoblastoma, ependymoma, extracranial germ cell tumors, extragonadal germ cell tumors, germ cell tumors, glioma, primary central nervous system lymphoma, medulloblastoma, medulloepithelioma, neuroblastoma, pineal parenchymal tumors of intermediate differentiation, pineoblastoma, supratentorial primitive neuroectodermal tumors, pituitary tumors, and primary central nervous system lymphoma.

In one embodiment of the present invention, the subject undergoing treatment has, or is at risk of having, a cancer defined in the claims. In one embodiment, the cancer is selected from the group consisting of brain cancer, glioblastoma, astrocytoma, atypical teratoid tumors, atypical rhabdoid tumors, brain tumors, craniopharyngioma, ependymoblastoma, glioma, and medulloblastoma.

In one embodiment of the present invention, the subject is at risk of having a cancer defined in the claims.

### BRIEF DESCRIPTION OF THE DRAWINGS

The foregoing summary, as well as the following detailed description of embodiments of the present invention, will be better understood when read in conjunction with the appended drawings of an exemplary embodiment. It should be understood, however, that the invention is not limited to the precise arrangements and instrumentalities shown.

In the drawings:
Figure 1 illustrates a dose response relation showing effects of various concentrations of an exemplary compound of the present invention, compound (1), on viability of tumor and normal cells; and
Figure 2 illustrates cell viability assay in human fetal lung fibroblast (MRC-5) cells following 72 hour treatment with an exemplary compound of the present invention, compound (1).

### DETAILED DESCRIPTION OF THE INVENTION

Scientific and technical terms used herein are intended to have the meanings commonly understood by those of ordinary skill in the art. Such terms are found defined and used in context in various standard references illustratively including J. Sambrook and D. W. Russell, Molecular Cloning: A Laboratory Manual, Cold Spring Harbor Laboratory Press; 3rd Ed., 2001; F. M. Ausubel, Ed., Short Protocols in Molecular Biology, Current Protocols; 5th Ed., 2002; B. Alberts et al., Molecular Biology of the Cell, 4th Ed., Garland, 2002; D. L. Nelson and M. M. Cox, Lehninger Principles of Biochemistry, 4th Ed., W.H. Freeman & Company, 2004; Engelke, D. R., RNA Interference (RNAi): Nuts and Bolts of RNAi Technology, DNA Press LLC, Eagleville, Pa., 2003; Herdewijn, P. (Ed.), Oligonucleotide Synthesis: Methods and Applications, Methods in Molecular Biology, Humana Press, 2004; A. Nagy, M. Gertsenstein, K. Vintersten, R. Behringer, Manipulating the Mouse Embryo: A Laboratory Manual, 3rd edition, Cold Spring Harbor Laboratory Press; Dec. 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 9780470151808, as well as U.S. Patent Application Publication No. 20120276088.

The singular terms "a," "an," and "the" are not intended to be limiting and include plural referents unless explicitly state or the context clearly indicates otherwise.

### I. COMPOSITIONS

The present invention provides a a compound selected from the group consisting of compound (1): 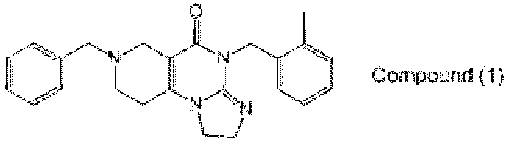 di-HClfor use in the treatment of one or more of the diseases defined in the claims. In one embodiment, the pharmaceutical composition comprises compound (1), a hydrochloride di-salt thereof (e.g., di-hydrochloride salt).

Compound (1) has the same chemical structure that would be revealed by structural analysis (e.g. NMR, X-ray diffraction) of compound NSC 350625, available from the National Cancer Institute's Developmental Therapeutics Program Repository.

In one embodiment, the compound of present invention is a di-hydrochloride salt of compound (1).

Salts (e.g., di-salts or tri-salts) of a compound defined in the claims can be prepared from a compound defined in the claims, which can be obtained commercially or synthesized using standard chemical synthetic methodology known to one of ordinary skill in the art.

In one embodiment, a pharmaceutical composition comprsing a compound defined in the claims includes at least one pharmaceutically acceptable carrier. Suitable pharmaceutically acceptable carriers, included those found in Handbook of Pharmaceutical Excipients, 7th Edition, edited by Raymond C. Rowe et al., American Pharmaceutical Association, Washington, USA and Pharmaceutical Press, London; and earlier editions.

Exemplary pharmaceutically acceptable carriers, methods for making pharmaceutical compositions and various dosage forms, as well as modes of administration are well-known in the art, for example as detailed in Pharmaceutical Dosage Forms: Tablets, edited by Larry L. Augsburger and Stephen W. Hoag., London: Informa Healthcare, 2008; and in L. V. Allen, Jr. et al., Ansel's Pharmaceutical Dosage Forms and Drug Delivery Systems, 8th Ed., Philadelphia, Pa.: Lippincott, Williams & Wilkins, 2004; A. R. Gennaro, Remington: The Science and Practice of Pharmacy, Lippincott Williams & Wilkins, 21st ed., 2005, particularly chapter 89; and J. G. Hardman et al., Goodman & Gilman's The Pharmacological Basis of Therapeutics, McGraw-Hill Professional, 10th ed., 2001.

In some embodiments, the pharmacuetical composition is formulated for ocular administration. In some embodiments, pharmaceutical compositions are formulated for topical ocular administration. In some embodiments, the pharmaceutical compositions are formulated as ointments, drops, or liquids. In some embodiments, the pharmaceutical composition include conventional pharmaceutical carriers such as aqueous, powdery or oily bases, thickeners or the like.

In some embodiments, the pharmaceutical composition of is formulated as intravenous formulation. In one embodiment, the intravenous formulation comprises compound (1) di-HCl dissolved in a solvent. In one embodiment, the solvent comprises water. In one such embodiment, the intravenous formulation comprises compound (1) di-HCl dissolved in water at a concentration of 25 mg/ml. In some embodiments, the intravenous formulation includes a higher or a lower concentration of compound (1) di-HCl. In one embodiment, the intravenous formulation includes compound (1) di-HCl in a concentration of from about 5 mg/ml to about 100 mg/ml. In one embodiment, the intravenous formulation includes compound (1) di-HCl in a concentration of about 50 mg/ml. In one embodiment, the intravenous formulation includes compound (1) di-HCl in a concentration of about 5 mg/ml. In one embodiment, the intravenous formulation includes from about 0.5 % to about 10 % of compound (1) di-HCl. In one embodiment, the intravenous formulation includes from about 5 % of compound (1) di-HCl.

In some embodiments, the intravenous formulation has pH of about 3. In one embodiment, pH of the intravenous formulation is adjusted to pH 3 with a phosphate buffer. In some embodiments, the intravenous formulation includes dextrose or sodium chloride. In one embodiment, the intravenous formulation including compound (1) di-HCl in a concentration of about 5 mg/ml and pH 3 forms a stable solution. In one embodiment, the intravenous formulation includes compound (1) di-HCl in a concentration of about 5 mg/ml and pH < 5 and forms a stable solution. In one embodiment, the intravenous formulation includes compound (1) di-HCl and one or more antioxidants. In one embodiment, the intravenous formulation includes a mixture of mono- and di-hydrochloride salt of compound (1). In one embodiment, the intravenous formulation includes compound (1) di-HCl as a 1 % solution having compound (1) di-HCl in a concentration of about 10 mg/ml. In one such embodiment, the intravenous formulation is a solution having a pH of about 3.3. In one embodiment, the pH is less than 4.0.

In one embodiment, a pharmaceutical composition according to the invention comprises about 0.1-99% of a salt of compound (1) di-HCl. In one such embodiment, the pharmaceutical composition further includes a pharmaceutically acceptable carrier. In one embodiment, a suitable pharmaceutically acceptable carrier includes an oil. In one embodiment, a suitable pharmaceutically acceptable carrier includes a sterile water. In one embodiment, a suitable pharmaceutically acceptable carrier includes an aqueous carrier.

In some embodiments, the intravenous formulation includes dextrose and/or sodium.

In one embodiment, the intravenous formulation comprises a di-hydrochloride salt of compound (1) dissolved in water at 25 mg/ml. In one such embodiment, the intravenous formulation is adjusted to pH 3 with phosphate buffer. In one such embodiment, the intravenous formulation includes dextrose or sodium chloride. In one such embodiment, the intravenous formulation includes a higher or a lower increase or decrease the concentration of the di-hydrochloride salt of compound (1). In one embodiment, the intravenous formulation includes a di-hydrochloride salt of compound (1) in a concentration of about 5 mg/ml. In one embodiment, the intravenous formulation including a di-hydrochloride salt of compound (1) in a concentration of about 5 mg/ml and pH 3 forms a stable solution. In one embodiment, the intravenous formulation includes a di-hydrochloride salt of compound (1) in a concentration of about 5 mg/ml and pH < 5 and forms a stable solution. In one embodiment, the intravenous formulation includes a di-hydrochloride salt of compound (1) and one or more antioxidants. In one embodiment, the intravenous formulation includes a mixture of mono- and di-hydrochloride salt of compound (1). In one embodiment, the intravenous formulation includes a di-hydrochloride salt of compound (1) as a 1 % solution having the di-hydrochloride salt of compound (1) in a concentration of about 10 mg/ml. In one such embodiment, the intravenous formulation is a solution having a pH of about 3.33. In one embodiment, the pH is less than 4.0.

In one embodiment, the intravenous formulation includes from about 0.5 % to about 10 % (or from about 5 mg/ml to about 100 mg/ml) of a di-HCl salt of compound (1). In one embodiment, the intravenous formulation includes from about 5 % (or about 50 mg/ml) of a di-HCl salt of compound (1). In one embodiment, the intravenous infusion rate may be slowed to decrease side effects of a di-HCl salt of compound (1).

In one embodiment, a pharmaceutical composition according to the invention comprises about 0.1-99% of a di-HCl salt of compound (1); and a pharmaceutically acceptable carrier, e.g., an oil or a sterile water or other aqueous carriers. In one embodiment, a pharmaceutical composition according to the invention comprises a di-HCl salt of compound (1) in a range of from about 5% to about 50 % for oral dosage forms.

In some embodiments, a pharmaceutical composition of the present invention includes an antioxidant. Suitable antioxidants include: ascorbic acid derivatives such as ascorbic acid, erythorbic acid, sodium ascorbate, thiol derivatives such as thioglycerol, cysteine, acetylcysteine, cystine, dithioerythreitol, dithiothreitol, glutathione, tocopherols, butylated hydroxyanisole (BHA), butylated hydroxytoluene (BHT), sulfurous acid salts such as sodium sulfate, sodium bisulfite, acetone sodium bisulfite, sodium metabisulfite, sodium sulfite, sodium formaldehyde sulfoxylate, and sodium thiosulfate, nordihydroguaiaretic acid. It should be noted that antioxidants used for aqueous formulations typically include: sodium sulphite, sodium metabisulphite, sodium formaldehyde sulphoxylate and ascorbic acid and combinations thereof, whereas antioxidants used in oil-based solutions, organic solvents, include butylated hydroxytoluene (BHT), butylated hydroxyanisole (BHA) and propyl gallate and combinations thereof. In yet other embodiments, an antioxidant can be one or more of a flavanoid, an isoflavone, monothioglycerol, L-cysteine, thioglycolic acid, α-tocopherol, ascorbic acid 6-palmitate, dihydrolipoic acid, butylated hydroxytoluene (BHT), butylated hydroxyanisole (BHA), vitamin E, propyl gallate, β-carotene, ascorbic acid. Antioxidants can typically be used in about 0.1% to 1.0% by weight, more typically about 0.2%.

In one embodiment, the pharmaceutical composition includes compound (1) di-HCl and at least one other therapeutic agent. In one such embodiment, the at least one other therapeutic agent is selected from the group consisting of hormone analogues and antihormones, aromatase inhibitors, LHRH agonists and antagonists, inhibitors of growth factors, growth factor antibodies, growth factor receptor antibodies, tyrosine kinase inhibitors; antimetabolites; antitumour antibiotics; platinum derivatives; alkylation agents; antimitotic agents; tubuline inhibitors; PARP inhibitors, topoisomerase inhibitors, serine/threonine kinase inhibitors, tyrosine kinase inhibitors, protein protein interaction inhibitors, RAF inhibitors, MEK inhibitors, ERK inhibitors, IGF-1R inhibitors, ErbB receptor inhibitors, rapamycin analogs, BTK inhibitors, CRM1 inhibitors (e.g., KPT185), P53 modulators (e.g., Nutlins), antiangiogenics (e.g., axitinib, aflibercept, sorafenib, and regorafenib), amifostin, anagrelid, clodronat, filgrastin, interferon, interferon alpha, leucovorin,rituximab, procarbazine, levamisole, mesna, mitotane, pamidronate and porfimer, 2-chlorodesoxyadenosine, 2-fluorodesoxy-cytidine, 2-methoxyoestradiol, 2C4,3-alethine, 131-1-TM-601, 3CPA, 7-ethyl-10-hydroxycamptothecin, 16-aza-epothilone B, A 105972, A 204197, abiraterone, aldesleukin, alitretinoin, allovectin-7, altretamine, alvocidib, amonafide, anthrapyrazole, AG-2037, AP-5280, apaziquone, apomine, aranose, arglabin, arzoxifene, atamestane, atrasentan, auristatin PE, AVLB, AZ10992, ABX-EGF, AMG-479 (ganitumab), ARRY 162, ARRY 438162, ARRY-300, ARRY-142886/AZD-6244 (selumetinib), ARRY-704/AZD-8330, AR-12, AR-42, AS-703988, AXL-1717, AZD-8055, AZD-5363, AZD-6244, ARQ-736, ARQ 680, AS-703026 (primasertib), avastin, AZD-2014, azacytidine, azaepothilone B, azonafide, BAY-43-9006, BAY 80-6946, BBR-3464, BBR-3576, bevacizumab, BEZ-235, biricodar dicitrate, BCX-1777, BKM-120, bleocin, BLP-25, BMS-184476, BMS-247550, BMS-188797, BMS-275291, BMS-663513, BMS-754807, BNP-1350, BNP-7787, BIBW 2992 (afatinib, tomtovok), BIBF 1120 (vargatef), BI 836845, BI 2536, BI 6727, BI 836845, BI 847325, BI 853520, BUB-022, bleomycinic acid, bleomycin A, bleomycin B, brivanib, bryostatin-1, bortezomib, brostallicin, busulphan, BYL-719, CA-4 prodrug, CA-4, CapCell, calcitriol, canertinib, canfosfamide, capecitabine, carboxyphthalatoplatin, CCl-779, CC-115, CC-223, CEP-701, CEP-751, CBT-1 cefixime, ceflatonin, ceftriaxone, celecoxib, celmoleukin, cemadotin, CH4987655/RO-4987655, chlorotrianisene, cilengitide, ciclosporin, CDA-II, CDC-394, CKD-602, CKI-27, clofarabin, colchicin, combretastatin A4, COT inhibitors, CHS-828, CH-5132799, CLL-Thera, CMT-3 cryptophycin 52, CTP-37, CTLA-4 monoclonal antibodies, CP-461, CV-247, cyanomorpholinodoxorubicin, cytarabine, D 24851, decitabine, deoxorubicin, deoxyrubicin, deoxycoformycin, depsipeptide, desoxyepothilone B, dexamethasone, dexrazoxanet, diethylstilbestrol, diflomotecan, didox, DMDC, dolastatin 10, doranidazole, DS-7423, E7010, E-6201, edatrexat, edotreotide, efaproxiral, eflornithine, EGFR inhibitors, EKB-569, EKB-509, enzastaurin, enzalutamide, elsamitrucin, epothilone B, epratuzumab, ER-86526, erlotinib, ET-18-0CH3, ethynylcytidine, ethynyloestradiol, exatecan, exatecan mesylate, exemestane, exisulind, fenretinide, figitumumab, floxuridine, folic acid, FOLFOX, FOLFOX4, FOLFIRI, formestane, fotemustine, galarubicin, gallium maltolate, gefinitib, gemtuzumab, gimatecan, glufosfamide, GCS-100, GDC-0623, GDC-0941 (pictrelisib), GDC-0980, GDC-0032, GDC-0068, GDC-0349, GDC-0879, G17DT immunogen, GMK, GPX-100, gp100-peptide vaccines, GSK-5126766, GSK-690693, GSK-1120212 (trametinib), GSK-2118436 (dabrafenib), GSK-2126458, GSK-2132231A, GSK-2334470, GSK-2110183, GSK-2141795, GW2016, granisetron, herceptine, hexamethylmelamine, histamine, homoharringtonine, hyaluronic acid, hydroxyurea, hydroxyprogesterone caproate, ibandronate, ibrutinib, ibritumomab, idatrexate, idenestrol, IDN-5109, IGF-1R inhibitors, IMC-1C11, IMC-A12 (cixutumumab), immunol, indisulam, interferon alpha-2a, interferon alpha-2b, pegylated interferon alpha-2b, interleukin-2, INK-1117, INK-128, INSM-18, ionafarnib, ipilimumab, iproplatin, irofulven, isohomohalichondrin-B, isoflavone, isotretinoin, ixabepilone, JRX-2, JSF-154, J-107088, conjugated oestrogens, kahalid F, ketoconazole, KW-2170, KW-2450, lobaplatin, leflunomide, lenograstim, leuprolide, leuporelin, lexidronam, LGD-1550, linezolid, lutetium texaphyrin, lometrexol, losoxantrone, LU 223651, lurtotecan, LY-S6AKT1, LY-2780301, mafosfamide, marimastat, mechloroethamine, MEK inhibitors, MEK-162, methyltestosteron, methylprednisolone, MEDI-573, MEN-10755, MDX-H210, MDX-447, MDX-1379, MGV, midostaurin, minodronic acid, mitomycin, mivobulin, MK-2206, MK-0646 (dalotuzumab), MLN518, motexaf in gadolinium, MS-209, MS-275, MX6, neridronate, neratinib, Nexavar, neovastat, nilotinib, nimesulide, nitroglycerin, nolatrexed, norelin, N-acetylcysteine, 06-benzylguanine, oblimersen, omeprazole, oncophage, oncoVEXGM-CSF, ormiplatin, ortataxel, OX44 antibodies, OSI-027, OSI-906 (linsitinib), 4-1BB antibodies, oxantrazole, oestrogen, panitumumab, patupilone, pegfilgrastim, PCK-3145, pegfilgrastim, PBI-1402, PBI-05204, PDO325901, PD-1 antibodies, PEG-paclitaxel, albumin-stabilized paclitaxel, PEP-005, PF-05197281, PF-05212384, PF-04691502, PHT-427, P-04, PKC412, P54, PI-88, pelitinib, pemetrexed, pentrix, perifosine, perillylalcohol, pertuzumab, PI3K inhibitors, PI3K/mTOR inhibitors, PG-TXL, PG2, PLX-4032/RO-5185426 (vemurafenib), PLX-3603/RO-5212054, PT-100, PWT-33597, PX-866, picoplatin, pivaloyloxymethylbutyrate, pixantrone, phenoxodiol O, PKI166, plevitrexed, plicamycin, polyprenic acid, porfiromycin, prednisone, prednisolone, quinamed, quinupristin, R115777, RAF-265, ramosetron, ranpirnase, RDEA-119/BAY 869766, RDEA-436, rebeccamycin analogues, receptor tyrosine kinase (RTK) inhibitors, regorafenib, revimid, RG-7167, RG-7304, RG-7421, RG-7321, RG 7440, rhizoxin, rhu-MAb, rinfabate, risedronate, rituximab, robatumumab, rofecoxib, RO-31-7453, RO-5126766, RO-5068760, RPR 109881A, rubidazone, rubitecan, R-flurbiprofen, RX-0201, S-9788, sabarubicin, SAHA, sargramostim, satraplatin, SB 408075, Se-015/Ve-015, SU5416, SU6668, SDX-101, semustin, seocalcitol, SM-11355, SN-38, SN-4071, SR-27897, SR-31747, SR-13668, SRL-172, sorafenib, spiroplatin, squalamine, suberanilohydroxamic acid, sutent, T 900607, T 138067, TAK-733, TAS-103, tacedinaline, talaporf in, Tarceva, tariquitar, tasisulam, taxotere, taxoprexin, tazarotene, tegafur, temozolamide, tesmilifene, testosterone, testosterone propionate, tesmilifene, tetraplatin, tetrodotoxin, tezacitabine, thalidomide, theralux, therarubicin, thymalfasin, thymectacin, tiazofurin, tipifarnib, tirapazamine, tocladesine, tomudex, toremofin, trabectedin, TransMID-107, transretinic acid, traszutumab, tremelimumab, tretinoin, triacetyluridine, triapine, triciribine, trimetrexate, TLK-286TXD 258, tykerb/tyverb, urocidin, valrubicin, vatalanib, vincristine, vinflunine, virulizin, WX-UK1, WX-554, vectibix, xeloda, XELOX, XL-147, XL-228, XL-281, XL-518/R-7420/GDC-0973, XL-765, YM-511, YM-598, ZD-4190, ZD-6474, ZD-4054, ZD-0473, ZD-6126, ZD-9331, ZD1839, ZSTK-474, zoledronat, zosuquidar, and combinations thereof.

In one embodiment, the at least one other therapeutic agent comprises one or more hormone analogues and/or antihormones are selected from the group consisting of tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxy-progesterone, octreotide, and combinations thereof. In one embodiment, the at least one other therapeutic agent comprises one or more LHRH agonists and/or antagonists selected from the group consisting of goserelin acetate, luprolide acetate, triptorelin pamoate and combinations thereof and wherein the LHRH antagonists are selected from the group consisting of Degarelix, Cetrorelix, Abarelix, Ozarelix, Degarelix combinations thereof. In one embodiment, the at least one other therapeutic agent comprises one or more growth factor inhibitors selected from the group consisting of inhibitors of: platelet derived growth factor (PDGF), fibroblast growth factor (FGF), vascular endothelial growth factor (VEGF), epidermal growth factor (EGF), insuline-like growth factors (IGF), human epidermal growth factor (HER) and hepatocyte growth factor (HGF). In one embodiment, the at least one other therapeutic agent comprises one or more inhibitors of the human epidermal growth factor selected from the group consisting of HER2, HER3, and HER4. In one embodiment, the at least one other therapeutic agent comprises one or more tyrosine kinase inhibitors selected from the group consisting of cetuximab, gefitinib, imatinib, lapatinib and trastuzumab, and combinations thereof. In one embodiment, the at least one other therapeutic agent comprises one or more aromatase inhibitors selected from the group consisting of anastrozole, letrozole, liarozole, vorozole, exemestane, atamestane, and combinations thereof. In one embodiment, the at least one other therapeutic agent comprises one or more antimetabolites which are antifolates selected from the group consisting of methotrexate, raltitrexed, and pyrimidine analogues. In one embodiment, the at least one other therapeutic agent comprises one or more antimetabolites which are pyrimidine analogues selected from the group consisting of 5-fluorouracil, capecitabin and gemcitabin. In one embodiment, the at least one other therapeutic agent comprises one or more antimetabolites which are purine and/or adenosine analogues selected from the group consisting of mercaptopurine, thioguanine, cladribine and pentostatin, cytarabine, fludarabine, and combinations thereof. In one embodiment, the at least one other therapeutic agent comprises one or more antitumour antibiotics selected from the group consisting of anthracyclins, doxorubicin, daunorubicin, epirubicin and idarubicin, mitomycin-C, bleomycin, dactinomycin, plicamycin, streptozocin and combinations thereof. In one embodiment, the at least one other therapeutic agent comprises one or more platinum derivatives selected from the group consisting of cisplatin, oxaliplatin, carboplatin and combinations thereof. In one embodiment, the at least one other therapeutic agent comprises one or more alkylation agents selected from the group consisting of estramustin, meclorethamine, melphalan, chlorambucil, busulphan, dacarbazin, cyclophosphamide, ifosfamide, temozolomide, nitrosoureas, and combinations thereof. In one embodiment, the at least one other therapeutic agent comprises nitrosoureas selected from the group consisting of carmustin, lomustin, thiotepa, and combinations thereof. In one embodiment, the at least one other therapeutic agent comprises antimitotic agents selected from the group consisting of Vinca alkaloids and taxanes. In one embodiment, the at least one other therapeutic agent comprises one or more taxanes selected from the group consisting of paclitaxel, docetaxel, and combinations thereof. In one embodiment, the at least one other therapeutic agent comprises one or more Vinca alkaloids selected from the group consisting of vinblastine, vindesin, vinorelbin, vincristine, and combinations thereof. In one embodiment, the at least one other therapeutic agent comprises one or more topoisomerase inhibitors which are epipodophyllotoxins. In one embodiment, the at least one other therapeutic agent comprises one or more epipodophyllotoxins selected from the group consisting of etoposide and etopophos, teniposide, amsacrin, topotecan, irinotecan, mitoxantron, and combinations thereof. In one embodiment, the at least one other therapeutic agent comprises one or more serine/threonine kinase inhibitors selected from the group consisting of PDK 1 inhibitors, B-Raf inhibitors, mTOR inhibitors, mTORC1 inhibitors, PI3K inhibitors, dual mTOR/PI3K inhibitors, STK 33 inhibitors, AKT inhibitors, PLK 1 inhibitors, inhibitors of CDKs, Aurora kinase inhibitors, and combinations thereof. In one embodiment, the at least one other therapeutic agent comprises one or more tyrosine kinase inhibitors which are PTK2/FAK inhibitors. In one embodiment, the at least one other therapeutic agent comprises one or more protein protein interaction inhibitors selected from the group consisting of IAP, Mcl-1, MDM2/MDMX and combinations thereof. In one embodiment, the at least one other therapeutic agent comprises one or more rapamycin analogs selected from the group consisting of everolimus, temsirolimus, ridaforolimus, sirolimus, and combinations thereof. In one embodiment, the at least one other therapeutic agent comprises one or more therapeutic agents selected from the group consisting of amifostin, anagrelid, clodronat, filgrastin, interferon, interferon alpha, leucovorin,rituximab, procarbazine, levamisole, mesna, mitotane, pamidronate and porfimer, and combinations thereof. In one embodiment, the at least one other therapeutic agent comprises one or more therapeutic agents selected from the group consisting of 2-chlorodesoxyadenosine, 2-fluorodesoxy-cytidine, 2-methoxyoestradiol, 2C4,3-alethine, 131-1-TM-601, 3CPA, 7-ethyl-10-hydroxycamptothecin, 16-aza-epothilone B, A 105972, A 204197, abiraterone, aldesleukin, alitretinoin, allovectin-7, altretamine, alvocidib, amonafide, anthrapyrazole, AG-2037, AP-5280, apaziquone, apomine, aranose, arglabin, arzoxifene, atamestane, atrasentan, auristatin PE, AVLB, AZ10992, ABX-EGF, AMG-479 (ganitumab), ARRY 162, ARRY 438162, ARRY-300, ARRY-142886/AZD-6244 (selumetinib), ARRY-704/AZD-8330, AR-12, AR-42, AS-703988, AXL-1717, AZD-8055, AZD-5363, AZD-6244, ARQ-736, ARQ 680, AS-703026 (primasertib), avastin, AZD-2014, azacytidine, azaepothilone B, azonafide, BAY-43-9006, BAY 80-6946, BBR-3464, BBR-3576, bevacizumab, BEZ-235, biricodar dicitrate, BCX-1777, BKM-120, bleocin, BLP-25, BMS-184476, BMS-247550, BMS-188797, BMS-275291, BMS-663513, BMS-754807, BNP-1350, BNP-7787, BIBW 2992 (afatinib, tomtovok), BIBF 1120 (vargatef), BI 836845, BI 2536, BI 6727, BI 836845, BI 847325, BI 853520, BUB-022, bleomycinic acid, bleomycin A, bleomycin B, brivanib, bryostatin-1, bortezomib, brostallicin, busulphan, BYL-719, CA-4 prodrug, CA-4, CapCell, calcitriol, canertinib, canfosfamide, capecitabine, carboxyphthalatoplatin, CCl-779, CC-115, CC-223, CEP-701, CEP-751, CBT-1 cefixime, ceflatonin, ceftriaxone, celecoxib, celmoleukin, cemadotin, CH4987655/RO-4987655, chlorotrianisene, cilengitide, ciclosporin, CDA-II, CDC-394, CKD-602, CKI-27, clofarabin, colchicin, combretastatin A4, COT inhibitors, CHS-828, CH-5132799, CLL-Thera, CMT-3 cryptophycin 52, CTP-37, CTLA-4 monoclonal antibodies, CP-461, CV-247, cyanomorpholinodoxorubicin, cytarabine, D 24851, decitabine, deoxorubicin, deoxyrubicin, deoxycoformycin, depsipeptide, desoxyepothilone B, dexamethasone, dexrazoxanet, diethylstilbestrol, diflomotecan, didox, DMDC, dolastatin 10, doranidazole, DS-7423, E7010, E-6201, edatrexat, edotreotide, efaproxiral, eflornithine, EGFR inhibitors, EKB-569, EKB-509, enzastaurin, enzalutamide, elsamitrucin, epothilone B, epratuzumab, ER-86526, erlotinib, ET-18-0CH3, ethynylcytidine, ethynyloestradiol, exatecan, exatecan mesylate, exemestane, exisulind, fenretinide, figitumumab, floxuridine, folic acid, FOLFOX, FOLFOX4, FOLFIRI, formestane, fotemustine, galarubicin, gallium maltolate, gefinitib, gemtuzumab, gimatecan, glufosfamide, GCS-100, GDC-0623, GDC-0941 (pictrelisib), GDC-0980, GDC-0032, GDC-0068, GDC-0349, GDC-0879, G17DT immunogen, GMK, GPX-100, gp100-peptide vaccines, GSK-5126766, GSK-690693, GSK-1120212 (trametinib), GSK-2118436 (dabrafenib), GSK-2126458, GSK-2132231A, GSK-2334470, GSK-2110183, GSK-2141795, GW2016, granisetron, herceptine, hexamethylmelamine, histamine, homoharringtonine, hyaluronic acid, hydroxyurea, hydroxyprogesterone caproate, ibandronate, ibrutinib, ibritumomab, idatrexate, idenestrol, IDN-5109, IGF-1R inhibitors, IMC-1C11, IMC-A12 (cixutumumab), immunol, indisulam, interferon alpha-2a, interferon alpha-2b, pegylated interferon alpha-2b, interleukin-2, INK-1117, INK-128, INSM-18, ionafarnib, ipilimumab, iproplatin, irofulven, isohomohalichondrin-B, isoflavone, isotretinoin, ixabepilone, JRX-2, JSF-154, J-107088, conjugated oestrogens, kahalid F, ketoconazole, KW-2170, KW-2450, lobaplatin, leflunomide, lenograstim, leuprolide, leuporelin, lexidronam, LGD-1550, linezolid, lutetium texaphyrin, lometrexol, losoxantrone, LU 223651, lurtotecan, LY-S6AKT1, LY-2780301, mafosfamide, marimastat, mechloroethamine, MEK inhibitors, MEK-162, methyltestosteron, methylprednisolone, MEDI-573, MEN-10755, MDX-H210, MDX-447, MDX-1379, MGV, midostaurin, minodronic acid, mitomycin, mivobulin, MK-2206, MK-0646 (dalotuzumab), MLN518, motexaf in gadolinium, MS-209, MS-275, MX6, neridronate, neratinib, Nexavar, neovastat, nilotinib, nimesulide, nitroglycerin, nolatrexed, norelin, N-acetylcysteine, 06-benzylguanine, oblimersen, omeprazole, oncophage, oncoVEXGM-CSF, ormiplatin, ortataxel, OX44 antibodies, OSI-027, OSI-906 (linsitinib), 4-1BB antibodies, oxantrazole, oestrogen, panitumumab, patupilone, pegfilgrastim, PCK-3145, pegfilgrastim, PBI-1402, PBI-05204, PDO325901, PD-1 antibodies, PEG-paclitaxel, albumin-stabilized paclitaxel, PEP-005, PF-05197281, PF-05212384, PF-04691502, PHT-427, P-04, PKC412, P54, PI-88, pelitinib, pemetrexed, pentrix, perifosine, perillylalcohol, pertuzumab, PI3K inhibitors, PI3K/mTOR inhibitors, PG-TXL, PG2, PLX-4032/RO-5185426 (vemurafenib), PLX-3603/RO-5212054, PT-100, PWT-33597, PX-866, picoplatin, pivaloyloxymethylbutyrate, pixantrone, phenoxodiol O, PKI166, plevitrexed, plicamycin, polyprenic acid, porfiromycin, prednisone, prednisolone, quinamed, quinupristin, R115777, RAF-265, ramosetron, ranpirnase, RDEA-119/BAY 869766, RDEA-436, rebeccamycin analogues, receptor tyrosine kinase (RTK) inhibitors, revimid, RG-7167, RG-7304, RG-7421, RG-7321, RG 7440, rhizoxin, rhu-MAb, rinfabate, risedronate, rituximab, robatumumab, rofecoxib, RO-31-7453, RO-5126766, RO-5068760, RPR 109881A, rubidazone, rubitecan, R-flurbiprofen, RX-0201, S-9788, sabarubicin, SAHA, sargramostim, satraplatin, SB 408075, Se-015/Ve-015, SU5416, SU6668, SDX-101, semustin, seocalcitol, SM-11355, SN-38, SN-4071, SR-27897, SR-31747, SR-13668, SRL-172, sorafenib, spiroplatin, squalamine, suberanilohydroxamic acid, sutent, T 900607, T 138067, TAK-733, TAS-103, tacedinaline, talaporf in, Tarceva, tariquitar, tasisulam, taxotere, taxoprexin, tazarotene, tegafur, temozolamide, tesmilifene, testosterone, testosterone propionate, tesmilifene, tetraplatin, tetrodotoxin, tezacitabine, thalidomide, theralux, therarubicin, thymalfasin, thymectacin, tiazofurin, tipifarnib, tirapazamine, tocladesine, tomudex, toremofin, trabectedin, TransMID-107, transretinic acid, traszutumab, tremelimumab, tretinoin, triacetyluridine, triapine, triciribine, trimetrexate, TLK-286TXD 258, tykerb/tyverb, urocidin, valrubicin, vatalanib, vincristine, vinflunine, virulizin, WX-UK1, WX-554, vectibix, xeloda, XELOX, XL-147, XL-228, XL-281, XL-518/R-7420/GDC-0973, XL-765, YM-511, YM-598, ZD-4190, ZD-6474, ZD-4054, ZD-0473, ZD-6126, ZD-9331, ZD1839, ZSTK-474, zoledronat, zosuquidar, and combinations thereof.

In some embodiments, the at least one other therapeutic agent comprises a steroid. Steroids include dexamethasone, prednisolone, methyl prednisolone, prednisone, hydrocortisone, triamcinolone, betamethasone, and cortivazol. In some embodiments, the at least one other therapeutic agent comprises an anti-emetic. Anti-emetics include 5-HT3 receptor agonists (such as dolasetron, granisetron, ondansetron, tropisetron, palonosetron, and mirtazapine), dopamine agonists (such as domperidone, olanzapine, droperidol, haloperidol, chlorpromazine, prochlorperazine, alizapride, prochlorperazine, and metoclopramide), NK1 receptor antagonists (such as aprepitant and casopitant), antihistamines (such as cyclizine, diphenhydramine, dimenhydrinate, doxylamine, meclizine, promethazine, hydroxyzine), cannabinoids (such as cannabis, dronabinol, nabilone, and sativex), benzodiazepines (such as midazolam and lorazepam), anticholinergics (such as hyoscine), trimethobenzamide, ginger, emetrol, propofol, peppermint, muscimol, and ajwain.

In some embodiments, the at least one other therapeutic agent comprises anti-cancer agent which includes a mitotic inhibitor. In one embodiment, the mitotic inhibitor includes a taxane. In one embodiment, the mitotic inhibitor includes a taxane selected from the group consisting of paclitaxel and docetaxel.

In one embodiment, the pharmaceutical composition includes compound (1) di-HCl and at least one anti-cancer agent, wherein the anti-cancer agent includes, without limitation, one or more of acivicin, aclarubicin, acodazole, acronine, adozelesin, aldesleukin, alitretinoin, allopurinol, altretamine, ambomycin, ametantrone, amifostine, aminoglutethimide, amsacrine, anastrozole, anthramycin, arsenic trioxide, asparaginase, asperlin, azacitidine, azetepa, azotomycin, batimastat, benzodepa, bevacizumab, bicalutamide, bisantrene, bisnafide dimesylate, bizelesin, bleomycin, brequinar, bropirimine, busulfan, cactinomycin, calusterone, capecitabine, caracemide, carbetimer, carboplatin, carmustine, carubicin, carzelesin, cedefingol, celecoxib, chlorambucil, cirolemycin, cisplatin, cladribine, crisnatol mesylate, cyclophosphamide, cytarabine, dacarbazine, dactinomycin, daunorubicin, decitabine, dexormaplatin, dezaguanine, dezaguanine mesylate, diaziquone, docetaxel, doxorubicin, droloxifene, dromostanolone, duazomycin, edatrexate, eflomithine, elsamitrucin, enloplatin, enpromate, epipropidine, epirubicin, erbulozole, esorubicin, estramustine, etanidazole, etoposide, etoprine, fadrozole, fazarabine, fenretinide, floxuridine, fludarabine, fluorouracil, flurocitabine, fosquidone, fostriecin, fulvestrant, gemcitabine, hydroxyurea, idarubicin, ifosfamide, ilmofosine, interleukin II (IL-2, including recombinant interleukin II or rIL2), interferon alfa-2a, interferon alfa-2b, interferon alfa-n1, interferon alfa-n3, interferon beta-Ia, interferon gamma-Ib, iproplatin, irinotecan, lanreotide, letrozole, leuprolide, liarozole, lometrexol, lomustine, losoxantrone, masoprocol, maytansine, mechlorethamine hydrochlride, megestrol, melengestrol acetate, melphalan, menogaril, mercaptopurine, methotrexate, metoprine, meturedepa, mitindomide, mitocarcin, mitocromin, mitogillin, mitomalcin, mitomycin, mitosper, mitotane, mitoxantrone, mycophenolic acid, nelarabine, nocodazole, nogalamycin, ormnaplatin, oxisuran, paclitaxel, pegaspargase, peliomycin, pentamustine, peplomycin, perfosfamide, pipobroman, piposulfan, piroxantrone hydrochloride, plicamycin, plomestane, porfimer, porfiromycin, prednimustine, procarbazine, puromycin, pyrazofurin, riboprine, rogletimide, safingol, semustine, simtrazene, sparfosate, sparsomycin, spirogermanium, spiromustine, spiroplatin, streptonigrin, streptozocin, sulofenur, talisomycin, tamoxifen, tecogalan, tegafur, teloxantrone, temoporfin, teniposide, teroxirone, testolactone, thiamiprine, thioguanine, thiotepa, tiazofurin, tirapazamine, topotecan, toremifene, trestolone, triciribine, trimetrexate, triptorelin, tubulozole, uracil mustard, uredepa, vapreotide, verteporfin, vinblastine, vincristine sulfate, vindesine, vinepidine, vinglycinate, vinleurosine, vinorelbine, vinrosidine, vinzolidine, vorozole, zeniplatin, zinostatin, zoledronate, zorubicin and combinations thereof.

Examples of suitable anti-cancer agents include those described Goodman and Gilman's The Pharmacological Basis of Therapeutics, 12th Ed., edited by Laurence Brunton, Bruce Chabner, Bjorn Knollman, McGraw Hill Professional, 2010.

In some exemplary embodiments, the pharmaceutical composition includes compound (1) di-HCl and at least one other therapeutic agent, wherein the at least one other therapeutic agent comprises an anti-angiogenic agent. In one such embodiment, the anti-angiogenic agent is bevacizumab. In one embodiment, the anti-angiogenic agent is selected from the group consisting of aflibercept, axitinib, angiostatin, endostatin, 16kDa prolactin fragment, laminin peptides, fibronectin peptides, tissue metalloproteinase inhibitors (TIMP 1, 2, 3, 4), plasminogen activator, inhibitors (PAI-1, -2), tumor necrosis factor α, (high dose, invitro), TGF-β1, interferons (IFN-α, -β, γ), ELR-CXC Chemokines:, IL-12; SDF-1; MIG; platelet factor 4 (PF-4); IP-10, thrombospondin (TSP), SPARC, 2-methoxyoestradiol, proliferin-related protein, suramin, sorafenib, regorafenib, thalidomide, cortisone, linomide, fumagillin (AGM-1470; TNP-470), tamoxifen, retinoids, CM101, dexamethasone, leukemia inhibitoryfactor (LIF), hedgehog inhibitor and combinations thereof.

The pharmaceutical combination in accordance with the present invention can include the first and second therapeutic agents in any desired proportions provided that the synergistic or cooperative effect still occurs. The synergistic pharmaceutical combination in accordance with the present invention preferably contains the first and second therapeutic agents in a ratio of from about 1:9 to about 9:1. In one embodiment, the synergistic pharmaceutical combination pontains the first and second therapeutic agents in a ratio of from about 1:8 to about 8:1. In one embodiment, the synergistic pharmaceutical combination pontains the first and second therapeutic agents in a ratio of from about 1:7 to about 7:1. In one embodiment, the synergistic pharmaceutical combination pontains the first and second therapeutic agents in a ratio of from about 1:6 to about 6:1. In one embodiment, the synergistic pharmaceutical combination pontains the first and second therapeutic agents in a ratio of from about 1:5 to about 5:1. In one embodiment, the synergistic pharmaceutical combination pontains the first and second therapeutic agents in a ratio of from about 1:4 to about 4: 1. In one embodiment, the synergistic pharmaceutical combination pontains the first and second therapeutic agents in a ratio of from about 1:3 to about 3: 1. In one embodiment, the synergistic pharmaceutical combination pontains the first and second therapeutic agents in a ratio of from about 1:2 to about 2: 1. In one embodiment, the synergistic pharmaceutical combination pontains the first and second therapeutic agents in a ratio of approximately 1: 1.

In some prefered embodiments, the second therapeutic agent is selected from the group consisting of Allopurinol, Arsenic Trioxide, Azacitidine, Bortezomib, Bevacizumab, Capecitabine, Carboplatin, Celecoxib, Chlorambucil, Clofarabine, Cytarabine, Dacarbazine, Daunorubicin HCl, Docetaxel, Doxorubicin HCl, Floxuridine, Gemcitabine HCl, Hydroxyurea, Ifosfamide, Imatinib Mesylate, Ixabepilone, Lenalidomide, Megestrol acetate, Methotrexate, Mitotane, Mitoxantrone HCl, Oxaliplatin, Paclitaxel, Pralatrexate, Romidepsin, Sorafenib, Streptozocin, Tamoxifen Citrate, Topotecan HCl, Tretinoin, Vandetanib, Vismodegib, Vorinostat, and combinations thereof.

In some prefered embodiments, the second therapeutic agent comprises a small molecule multi-kinase inhibitor. In one embodiment, the small molecule multi-kinase inhibitor comprises sorafenib or regorafenib. In some prefered embodiments, the second therapeutic agent comprises a Hedgehog Pathway Inhibitor. In one prefered embodiment, the Hedgehog Pathway Inhibitor comprises vismodegib.

In some prefered embodiments, the second therapeutic agent include members of the classes drugs listed in the following table 1.

**Table 1: Classes Of Drugs That Have Demonstrated Synergy**

| **Classes of drugs** | **Examples** |
|---|---|
| Purine analogs | Examples include allopurinol, oxypurinol, clofarabine, and tisopurine |
| Pyrimidine analogs | Examples include 5-fluorouracil, Floxuridine (FUDR), capecitabine, cytarabine, 6-azauracil (6-AU), and gemcitabine (Gemzar). |
| Proteasome inhibitors | Examples include bortezomib, carfilzomib, cediranib, disulfiram, epigallocatechin-3-gallate, salinosporamide A, ONCX 0912, CEP-18770, MLN9708, epoxomicin, and MG132. |
| Anti-angiogenic | Examples include bevacizumab, aflibercept, sunitinib, sorafenib, pazopanib, vandetanib, cabozantinib, axitinib, |
| | ponatinib, regorafenib, ranibizumab, lapatinib, and vandetanib. |
| Platinum-based antineoplastic drugs | Examples include cisplatin, carboplatin, oxaliplatin, satraplatin, picoplatin, nedaplatin, and triplatin. |
| COX-2 inhibitors | Examples include celecoxib, valdecoxib (Bextra), parecoxib (Dynastat), lumiracoxib, etoricoxib, and rofecoxib. |
| Nitrogen mustards | Examples include cyclophosphamide, chlorambucil, uramustine, ifosfamide, melphalan, bendamustine, and mustine. |
| Alkylating agents | Examples include cyclophosphamide, mechlorethamine or mustine (HN2) (trade name Mustardgen), uramustine or uracil mustard, melphalan, chlorambucil, ifosfamide, bendamustine, carmustine, lomustine, streptozocin, and busulfan. |
| Anthracyclines | Examples include Daunorubicin (Daunomycin), Daunorubicin (liposomal), Doxorubicin (Adriamycin), Doxorubicin (liposomal), Epirubicin, Idarubicin, Valrubicin, and Mitoxantrone. |
| Taxanes | Examples include Paclitaxel (Taxol), Docetaxel (Taxotere), and albumin-bound paclitaxel (Abraxane). |
| Nucleotide synthesis inhibitor | Examples include methotrexate, pralatrexate, hydroxyurea, and 5-fluorodeoxyuridine, 3,4-dihydroxybenzylamine. |
| Bcr-abl inhibitors | Examples include imatinib, nilotinib, dasatinib, bosutinib and ponatinib. |
| Other | Examples include arsenic trioxide, thalidomide, revlimid, and mitotane. |
| Topoisomerase inhibitor | Examples include amsacrine, etoposide, etoposide phosphate, teniposide, doxorubicin, Topotecan (Hycamtin), Irinotecan (CPT-11, Camptosar), Exatecan, Lurtotecan, ST 1481, CKD 602, ICRF-193, and genistein. |
| HDAC inhibitors | Examples include Vorinostat (SAHA), Romidepsin (Istodax), Panobinostat (LBH589), Valproic acid (as Mg valproate), Belinostat (PXD101), Mocetinostat (MGCD0103), Abexinostat (PCI-24781), Entinostat (MS-275), SB939, Resminostat (4SC-201), Givinostat, Quisinostat (JNJ-26481585), CUDC-101, AR-42, CHR-2845, CHR-3996, 4SC-202, CG200745, ACY-1215, ME-344, sulforaphane, Kevetrin, and ATRA. |
| Multi-kinase inhibitors | Examples include sorafenib, regorafenib, and vandetanib. |
| Hormone therapies | Examples include tamoxifen, toremifene, Arimidex (anastrozole), Aromasin (exemestane), Femara (letrozole), and Fulvestrant (Faslodex). |
| Hedgehog signaling Inhibitors | Examples include vismodegib, BMS-833923, IPI-926, LDE-225, PF-04449913, LEQ 506, and TAK-441. |

In some embodiments, the second therapeutic agent includes drugs that target tumor necrosis factor-related apoptosis-inducing ligand (TRAIL) receptors. In one embodiment, the second therapeutic agent includes a recombinant TRAIL or an agonistic antibody that activates one or more TRAIL receptors. In one embodiment, the second therapeutic agent includes one or more antibodies or recombinant TRAIL that activate signaling by DR4 and/or DR5. In one embodiment, the second therapeutic agent includes one or more of mapatumumab, lexatumumab, Apomab, AMG-655, LBY-135 and rhApo2L/TRAEL. In one embodiment, the second therapeutic agent includes an active agent selected from the group consisting of Camptothecin, 5-FU, capecitabine, cisplatin, doxorubicin, irinotecan, paclitaxel, cisplatin, bortezomib, BH3I-2, rituximab, radiation, triterpenoids, sorafenib, gemcitabine, HDAC inhibitors, carboplatin, T-101 (a gossypol derivate), ABT-263, ABT-737, and GX-15-070 (obatoclax), vorinostat, cetuximab, panitumumab, bevacizumab, ganitumab, interferon gamma, sorafenib, XIAP antagonists, Bcl-2 antagonists, and Smac mimetics.

### II. DOSE

In one embodiment, a pharmaceutical composition comprises compound (1) di-HCl in a dose ranging from about 100 mg to about 2000 mg, where the weight can, in certain embodiments be based on compound (**1**) in its free base form. In one embodiment, a pharmaceutical composition according to the invention comprises compound (**1**) di-HCl in a dose ranging from about 40 mg to about 2000 mg, where the weight can, in certain embodiments be based on compound (**1**) in its free base form. In one embodiment, a pharmaceutical composition according to the invention comprises compound (**1**) di-HCl in a dose ranging from about 50 mg to about 2000 mg, where the weight can, in certain embodiments be based on compound (**1**) in its free base form. In one embodiment, a pharmaceutical composition according to the invention comprises compound (**1**) di-HCl in a dose ranging from about 60 mg to about 2000 mg, where the weight can, in certain embodiments be based on compound (**1**) in its free base form. In one embodiment, a pharmaceutical composition according to the invention comprises compound (**1**) di-HCl in a dose level selected from the group consisting of from about 50 mg to about 200 mg, from about 50 mg to about 300 mg, from about 50 mg to about 400 mg, from about 50 mg to about 500 mg, from about 50 mg to about 600 mg, from about 50 mg to about 700 mg, from about 50 mg to about 800 mg, from about 50 mg to about 900 mg, from about 50 mg to about 1000 mg, from about 50 mg to about 1100 mg, from about 50 mg to about 1200 mg, from about 50 mg to about 1300 mg, from about 50 mg to about 1400 mg, from about 50 mg to about 1500 mg, from about 50 mg to about 1600 mg, from about 50 mg to about 1700 mg, from about 50 mg to about 1800 mg, and from about 50 mg to about 1900 mg, from 40 mg to 2000 mg. In one embodiment, a pharmaceutical composition comprises compound (**1**) di-HCl in a dose level selected from the group consisting of from about 40 mg to about 200 mg, from about 40 mg to about 300 mg, from about 40 mg to about 400 mg, from about 40 mg to about 500 mg, from about 40 mg to about 600 mg, from about 40 mg to about 700 mg, from about 40 mg to about 800 mg, from about 40 mg to about 900 mg, from about 40 mg to about 1000 mg, from about 40 mg to about 1100 mg, from about 40 mg to about 1200 mg, from about 40 mg to about 1300 mg, from about 40 mg to about 1400 mg, from about 40 mg to about 1500 mg, from about 40 mg to about 1600 mg, from about 40 mg to about 1700 mg, from about 40 mg to about 1800 mg, and from about 40 mg to about 1900 mg, from 40 mg to 2000 mg. In one embodiment, a pharmaceutical composition according to the invention comprises compound (**1**) di-HCl in a dose level selected from the group consisting of from about 60 mg to about 200 mg, from about 60 mg to about 300 mg, from about 60 mg to about 400 mg, from about 60 mg to about 500 mg, from about 60 mg to about 600 mg, from about 60 mg to about 700 mg, from about 60 mg to about 800 mg, from about 60 mg to about 900 mg, from about 60 mg to about 1000 mg, from about 60 mg to about 1100 mg, from about 60 mg to about 1200 mg, from about 60 mg to about 1300 mg, from about 60 mg to about 1400 mg, from about 60 mg to about 1500 mg, from about 60 mg to about 1600 mg, from about 60 mg to about 1700 mg, from about 60 mg to about 1800 mg, and from about 60 mg to about 1900 mg, from 60 mg to 2000 mg. In one embodiment, a pharmaceutical composition according to the invention comprises compound (**1**) di-HCl in a dose level selected from the group consisting of from about 100 mg to about 200 mg, from about 100 mg to about 300 mg, from about 100 mg to about 400 mg, from about 100 mg to about 500 mg, from about 100 mg to about 600 mg, from about 100 mg to about 700 mg, from about 100 mg to about 800 mg, from about 100 mg to about 900 mg, from about 100 mg to about 1000 mg, from about 100 mg to about 1100 mg, from about 100 mg to about 1200 mg, from about 100 mg to about 1300 mg, from about 100 mg to about 1400 mg, from about 100 mg to about 1500 mg, from about 100 mg to about 1600 mg, from about 100 mg to about 1700 mg, from about 100 mg to about 1800 mg, and from about 100 mg to about 1900 mg, from 50 mg to 2000 mg and from 40 mg to 200 mg. In one embodiment, a pharmaceutical composition according to the invention comprises compound (**1**) di-HCl in a dose level selected from the group consisting of from about 200 mg to about 300 mg, from about 200 mg to about 400 mg, from about 200 mg to about 500 mg, from about 200 mg to about 600 mg, from about 200 mg to about 700 mg, from about 200 mg to about 800 mg, from about 200 mg to about 900 mg, from about 200 mg to about 1000 mg, from about 200 mg to about 1100 mg, from about 200 mg to about 1200 mg, from about 200 mg to about 1300 mg, from about 200 mg to about 1400 mg, from about 200 mg to about 1500 mg, from about 200 mg to about 1600 mg, from about 200 mg to about 1700 mg, from about 200 mg to about 1800 mg, and from about 200 mg to about 1900 mg based on compound (**1**) in its free base form. In one embodiment, a pharmaceutical composition comprises compound (**1**) di-HCl in a dose level selected from the group consisting of from about 400 mg to about 500 mg, from about 400 mg to about 600 mg, from about 400 mg to about 700 mg, from about 400 mg to about 800 mg, from about 400 mg to about 900 mg, from about 400 mg to about 1000 mg, from about 400 mg to about 1100 mg, from about 400 mg to about 1200 mg, from about 400 mg to about 1300 mg, from about 400 mg to about 1400 mg, from about 400 mg to about 1500 mg, from about 400 mg to about 1600 mg, from about 400 mg to about 1700 mg, from about 400 mg to about 1800 mg, and from about 400 mg to about 1900 mg based on compound (**1**) in its free base form. In one embodiment, a pharmaceutical composition comprises compound (**1**) di-HCl in a dose level selected from the group consisting of from about 50 mg to about 60 mg, from about 50 mg to about 70 mg, from about 50 mg to about 80 mg, from about 50 mg to about 90 mg, from about 50 mg to about 100 mg, from about 60 mg to about 70 mg, from about 60 mg to about 80 mg, from about 60 mg to about 90 mg, from about 60 mg to about 100 mg, from about 70 mg to about 80 mg, from about 70 mg to about 90 mg, from about 70 mg to about 100 mg, from about 80 mg to about 90 mg, from about 80 mg to about 100 mg, and from about 90 mg to about 100 mg

In one embodiment, a pharmaceutical composition comprises compound (**1**) di-HCl in a dose ranging from about 1 mg/kg to about 40 mg/kg. In one embodiment, a pharmaceutical composition comprises compound (**1**) di-HCl in a dose level selected from the group consisting of from about 1 mg/Kg to about 40 mg/Kg, from about 2 mg/Kg to about 40 mg/Kg, from about 3 mg/Kg to about 40 mg/Kg, from about 4 mg/Kg to about 40 mg/Kg, from about 5 mg/Kg to about 40 mg/Kg, from about 6 mg/Kg to about 40 mg/Kg, from about 7 mg/Kg to about 40 mg/Kg, from about 8 mg/Kg to about 40 mg/Kg, from about 9 mg/Kg to about 40 mg/Kg, from about 10 mg/Kg to about 40 mg/Kg, from about 11 mg/Kg to about 40 mg/Kg, from about 12 mg/Kg to about 40 mg/Kg, from about 13 mg/Kg to about 40 mg/Kg, from about 14 mg/Kg to about 40 mg/Kg, from about 15 mg/Kg to about 40 mg/Kg, from about 16 mg/Kg to about 40 mg/Kg, from about 17 mg/Kg to about 40 mg/Kg, from about 18 mg/Kg to about 40 mg/Kg, from about 19 mg/Kg to about 40 mg/Kg, from about 20 mg/Kg to about 40 mg/Kg, from about 21 mg/Kg to about 40 mg/Kg, from about 22 mg/Kg to about 40 mg/Kg, from about 23 mg/Kg to about 40 mg/Kg, from about 24 mg/Kg to about 40 mg/Kg, from about 25 mg/Kg to about 40 mg/Kg, from about 26 mg/Kg to about 40 mg/Kg, from about 27 mg/Kg to about 40 mg/Kg, from about 28 mg/Kg to about 40 mg/Kg, from about 29 mg/Kg to about 40 mg/Kg, from about 30 mg/Kg to about 40 mg/Kg, from about 31 mg/Kg to about 40 mg/Kg, from about 32 mg/Kg to about 40 mg/Kg, from about 33 mg/Kg to about 40 mg/Kg, from about 34 mg/Kg to about 40 mg/Kg, from about 35 mg/Kg to about 40 mg/Kg, from about 36 mg/Kg to about 40 mg/Kg, from about 37 mg/Kg to about 40 mg/Kg, from about 38 mg/Kg to about 40 mg/Kg, and from about 39 mg/Kg to about 40 mg/Kg.

In one embodiment, a pharmaceutical composition comprises compound (1) di-HCl in a dose level selected from the group consisting of from about 1 mg/Kg to about 30 mg/Kg, from about 2 mg/Kg to about 30 mg/Kg, from about 3 mg/Kg to about 30 mg/Kg, from about 4 mg/Kg to about 30 mg/Kg, from about 5 mg/Kg to about 30 mg/Kg, from about 6 mg/Kg to about 30 mg/Kg, from about 7 mg/Kg to about 30 mg/Kg, from about 8 mg/Kg to about 30 mg/Kg, from about 9 mg/Kg to about 30 mg/Kg, from about 10 mg/Kg to about 30 mg/Kg, from about 11 mg/Kg to about 30 mg/Kg, from about 12 mg/Kg to about 30 mg/Kg, from about 13 mg/Kg to about 30 mg/Kg, from about 14 mg/Kg to about 30 mg/Kg, from about 15 mg/Kg to about 30 mg/Kg, from about 16 mg/Kg to about 30 mg/Kg, from about 17 mg/Kg to about 30 mg/Kg, from about 18 mg/Kg to about 30 mg/Kg, from about 19 mg/Kg to about 30 mg/Kg, from about 20 mg/Kg to about 30 mg/Kg, from about 21 mg/Kg to about 30 mg/Kg, from about 22 mg/Kg to about 30 mg/Kg, from about 23 mg/Kg to about 30 mg/Kg, from about 24 mg/Kg to about 30 mg/Kg, from about 25 mg/Kg to about 30 mg/Kg, from about 26 mg/Kg to about 30 mg/Kg, from about 27 mg/Kg to about 30 mg/Kg, from about 28 mg/Kg to about 30 mg/Kg, and from about 29 mg/Kg to about 30 mg/Kg.

In one embodiment, a pharmaceutical composition comprises compound (1) di-HCl in a dose level selected from the group consisting of from about 1 mg/Kg to about 20 mg/Kg, from about 2 mg/Kg to about 20 mg/Kg, from about 3 mg/Kg to about 20 mg/Kg, from about 4 mg/Kg to about 20 mg/Kg, from about 5 mg/Kg to about 20 mg/Kg, from about 6 mg/Kg to about 20 mg/Kg, from about 7 mg/Kg to about 20 mg/Kg, from about 8 mg/Kg to about 20 mg/Kg, from about 9 mg/Kg to about 20 mg/Kg, from about 10 mg/Kg to about 20 mg/Kg, from about 11 mg/Kg to about 20 mg/Kg, from about 12 mg/Kg to about 20 mg/Kg, from about 13 mg/Kg to about 20 mg/Kg, from about 14 mg/Kg to about 20 mg/Kg, from about 15 mg/Kg to about 20 mg/Kg, from about 16 mg/Kg to about 20 mg/Kg, from about 17 mg/Kg to about 20 mg/Kg, from about 18 mg/Kg to about 20 mg/Kg, and from about 19 mg/Kg to about 20 mg/Kg.

In one embodiment, a pharmaceutical composition comprises compound (1) di-HCl in a dose level selected from the group consisting of from about 1 mg/Kg to about 10 mg/Kg, from about 2 mg/Kg to about 10 mg/Kg, from about 3 mg/Kg to about 10 mg/Kg, from about 4 mg/Kg to about 10 mg/Kg, from about 5 mg/Kg to about 10 mg/Kg, from about 6 mg/Kg to about 10 mg/Kg, from about 7 mg/Kg to about 10 mg/Kg, from about 8 mg/Kg to about 10 mg/Kg, and from about 9 mg/Kg to about 10 mg/Kg.

In one embodiment, a pharmaceutical composition comprises compound (1) di-HCl in a dose level ranging from about 37.5 mg/m² to about 1500 mg/m². In one embodiment, a pharmaceutical composition according to the invention comprises compound (1) di-HCl in a dose level selected from the group consisting of from about from about 40 mg/m² to about 1500 mg/m², from about 45 mg/m² to about 1500 mg/m², from about 50 mg/m² to about 1500 mg/m², from about 55 mg/m² to about 1500 mg/m², from about 60 mg/m² to about 1500 mg/m², from about 65 mg/m² to about 1500 mg/m², from about 70 mg/m² to about 1500 mg/m², from about 75 mg/m² to about 1500 mg/m², from about 80 mg/m² to about 1500 mg/m², from about 85 mg/m² to about 1500 mg/m², from about 90 mg/m² to about 1500 mg/m², from about 95 mg/m² to about 1500 mg/m², from about 100 mg/m² to about 1500 mg/m², from about 105 mg/m² to about 1500 mg/m², from about 110 mg/m² to about 1500 mg/m², from about 115 mg/m² to about 1500 mg/m², from about 120 mg/m² to about 1500 mg/m², from about 125 mg/m² to about 1500 mg/m², from about 130 mg/m² to about 1500 mg/m², from about 135 mg/m² to about 1500 mg/m², from about 140 mg/m² to about 1500 mg/m², from about 145 mg/m² to about 1500 mg/m², from about 150 mg/m² to about 1500 mg/m², from about 155 mg/m² to about 1500 mg/m², from about 160 mg/m² to about 1500 mg/m², from about 165 mg/m² to about 1500 mg/m², from about 170 mg/m² to about 1500 mg/m², from about 175 mg/m² to about 1500 mg/m², from about 180 mg/m² to about 1500 mg/m², from about 185 mg/m² to about 1500 mg/m², from about 190 mg/m² to about 1500 mg/m², from about 195 mg/m² to about 1500 mg/m², from about 200 mg/m² to about 1500 mg/m², from about 205 mg/m² to about 1500 mg/m², from about 210 mg/m² to about 1500 mg/m², from about 215 mg/m² to about 1500 mg/m², from about 220 mg/m² to about 1500 mg/m², from about 225 mg/m² to about 1500 mg/m², from about 230 mg/m² to about 1500 mg/m², from about 235 mg/m² to about 1500 mg/m², from about 240 mg/m² to about 1500 mg/m², from about 245 mg/m² to about 1500 mg/m², from about 250 mg/m² to about 1500 mg/m², from about 255 mg/m² to about 1500 mg/m², from about 260 mg/m² to about 1500 mg/m², from about 265 mg/m² to about 1500 mg/m², from about 270 mg/m² to about 1500 mg/m², from about 275 mg/m² to about 1500 mg/m², from about 280 mg/m² to about 1500 mg/m², from about 285 mg/m² to about 1500 mg/m², from about 290 mg/m² to about 1500 mg/m², from about 295 mg/m² to about 1500 mg/m², from about 300 mg/m² to about 1500 mg/m², from about 305 mg/m² to about 1500 mg/m², from about 310 mg/m² to about 1500 mg/m², from about 315 mg/m² to about 1500 mg/m², from about 320 mg/m² to about 1500 mg/m², from about 325 mg/m² to about 1500 mg/m², from about 330 mg/m² to about 1500 mg/m², from about 335 mg/m² to about 1500 mg/m², from about 340 mg/m² to about 1500 mg/m², from about 345 mg/m² to about 1500 mg/m², from about 350 mg/m² to about 1500 mg/m², from about 355 mg/m² to about 1500 mg/m², from about 360 mg/m² to about 1500 mg/m², from about 365 mg/m² to about 1500 mg/m², from about 370 mg/m² to about 1500 mg/m², from about 375 mg/m² to about 1500 mg/m², from about 380 mg/m² to about 1500 mg/m², from about 385 mg/m² to about 1500 mg/m², from about 390 mg/m² to about 1500 mg/m², from about 395 mg/m² to about 1500 mg/m², from about 400 mg/m² to about 1500 mg/m², from about 405 mg/m² to about 1500 mg/m², from about 410 mg/m² to about 1500 mg/m², from about 415 mg/m² to about 1500 mg/m², from about 420 mg/m² to about 1500 mg/m², from about 425 mg/m² to about 1500 mg/m², from about 430 mg/m² to about 1500 mg/m², from about 435 mg/m² to about 1500 mg/m², from about 440 mg/m² to about 1500 mg/m², from about 445 mg/m² to about 1500 mg/m², from about 450 mg/m² to about 1500 mg/m², from about 455 mg/m² to about 1500 mg/m², from about 460 mg/m² to about 1500 mg/m², from about 465 mg/m² to about 1500 mg/m², from about 470 mg/m² to about 1500 mg/m², from about 475 mg/m² to about 1500 mg/m², from about 480 mg/m² to about 1500 mg/m², from about 485 mg/m² to about 1500 mg/m², from about 490 mg/m² to about 1500 mg/m², from about 495 mg/m² to about 1500 mg/m², from about 500 mg/m² to about 1500 mg/m², from about 505 mg/m² to about 1500 mg/m², from about 510 mg/m² to about 1500 mg/m², from about 515 mg/m² to about 1500 mg/m², from about 520 mg/m² to about 1500 mg/m², from about 525 mg/m² to about 1500 mg/m², from about 530 mg/m² to about 1500 mg/m², from about 535 mg/m² to about 1500 mg/m², from about 540 mg/m² to about 1500 mg/m², from about 545 mg/m² to about 1500 mg/m², from about 550 mg/m² to about 1500 mg/m², from about 555 mg/m² to about 1500 mg/m², from about 560 mg/m² to about 1500 mg/m², from about 565 mg/m² to about 1500 mg/m², from about 570 mg/m² to about 1500 mg/m², from about 575 mg/m² to about 1500 mg/m², from about 580 mg/m² to about 1500 mg/m², from about 585 mg/m² to about 1500 mg/m², from about 590 mg/m² to about 1500 mg/m², from about 595 mg/m² to about 1500 mg/m², from about 600 mg/m² to about 1500 mg/m², from about 605 mg/m² to about 1500 mg/m², from about 610 mg/m² to about 1500 mg/m², from about 615 mg/m² to about 1500 mg/m², from about 620 mg/m² to about 1500 mg/m², from about 625 mg/m² to about 1500 mg/m², from about 630 mg/m² to about 1500 mg/m², from about 635 mg/m² to about 1500 mg/m², from about 640 mg/m² to about 1500 mg/m², from about 645 mg/m² to about 1500 mg/m², from about 650 mg/m² to about 1500 mg/m², from about 655 mg/m² to about 1500 mg/m², from about 660 mg/m² to about 1500 mg/m², from about 665 mg/m² to about 1500 mg/m², from about 670 mg/m² to about 1500 mg/m², from about 675 mg/m² to about 1500 mg/m², from about 680 mg/m² to about 1500 mg/m², from about 685 mg/m² to about 1500 mg/m², from about 690 mg/m² to about 1500 mg/m², from about 695 mg/m² to about 1500 mg/m², from about 700 mg/m² to about 1500 mg/m², from about 705 mg/m² to about 1500 mg/m², from about 710 mg/m² to about 1500 mg/m², from about 715 mg/m² to about 1500 mg/m², from about 720 mg/m² to about 1500 mg/m², from about 725 mg/m² to about 1500 mg/m², from about 730 mg/m² to about 1500 mg/m², from about 735 mg/m² to about 1500 mg/m², from about 740 mg/m² to about 1500 mg/m², from about 745 mg/m² to about 1500 mg/m², from about 750 mg/m² to about 1500 mg/m², from about 755 mg/m² to about 1500 mg/m², from about 760 mg/m² to about 1500 mg/m², from about 765 mg/m² to about 1500 mg/m², from about 770 mg/m² to about 1500 mg/m², from about 775 mg/m² to about 1500 mg/m², from about 780 mg/m² to about 1500 mg/m², from about 785 mg/m² to about 1500 mg/m², from about 790 mg/m² to about 1500 mg/m², from about 795 mg/m² to about 1500 mg/m², from about 800 mg/m² to about 1500 mg/m², from about 805 mg/m² to about 1500 mg/m², from about 810 mg/m² to about 1500 mg/m², from about 815 mg/m² to about 1500 mg/m², from about 820 mg/m² to about 1500 mg/m², from about 825 mg/m² to about 1500 mg/m², from about 830 mg/m² to about 1500 mg/m², from about 835 mg/m² to about 1500 mg/m², from about 840 mg/m² to about 1500 mg/m², from about 845 mg/m² to about 1500 mg/m², from about 850 mg/m² to about 1500 mg/m², from about 855 mg/m² to about 1500 mg/m², from about 860 mg/m² to about 1500 mg/m², from about 865 mg/m² to about 1500 mg/m², from about 870 mg/m² to about 1500 mg/m², from about 875 mg/m² to about 1500 mg/m², from about 880 mg/m² to about 1500 mg/m², from about 885 mg/m² to about 1500 mg/m², from about 890 mg/m² to about 1500 mg/m², from about 895 mg/m² to about 1500 mg/m², from about 900 mg/m² to about 1500 mg/m², from about 905 mg/m² to about 1500 mg/m², from about 910 mg/m² to about 1500 mg/m², from about 915 mg/m² to about 1500 mg/m², from about 920 mg/m² to about 1500 mg/m², from about 925 mg/m² to about 1500 mg/m², from about 930 mg/m² to about 1500 mg/m², from about 935 mg/m² to about 1500 mg/m², from about 940 mg/m² to about 1500 mg/m², from about 945 mg/m² to about 1500 mg/m², from about 950 mg/m² to about 1500 mg/m², from about 955 mg/m² to about 1500 mg/m², from about 960 mg/m² to about 1500 mg/m², from about 965 mg/m² to about 1500 mg/m², from about 970 mg/m² to about 1500 mg/m², from about 975 mg/m² to about 1500 mg/m², from about 980 mg/m² to about 1500 mg/m², from about 985 mg/m² to about 1500 mg/m², from about 990 mg/m² to about 1500 mg/m², from about 995 mg/m² to about 1500 mg/m², from about 1000 mg/m² to about 1500 mg/m², from about 1005 mg/m² to about 1500 mg/m², from about 1010 mg/m² to about 1500 mg/m², from about 1015 mg/m² to about 1500 mg/m², from about 1020 mg/m² to about 1500 mg/m², from about 1025 mg/m² to about 1500 mg/m², from about 1030 mg/m² to about 1500 mg/m², from about 1035 mg/m² to about 1500 mg/m², from about 1040 mg/m² to about 1500 mg/m², from about 1045 mg/m² to about 1500 mg/m², from about 1050 mg/m² to about 1500 mg/m², from about 1055 mg/m² to about 1500 mg/m², from about 1060 mg/m² to about 1500 mg/m², from about 1065 mg/m² to about 1500 mg/m², from about 1070 mg/m² to about 1500 mg/m², from about 1075 mg/m² to about 1500 mg/m², from about 1080 mg/m² to about 1500 mg/m², from about 1085 mg/m² to about 1500 mg/m², from about 1090 mg/m² to about 1500 mg/m², from about 1095 mg/m² to about 1500 mg/m², from about 1100 mg/m² to about 1500 mg/m², from about 1105 mg/m² to about 1500 mg/m², from about 1110 mg/m² to about 1500 mg/m², from about 1115 mg/m² to about 1500 mg/m², from about 1120 mg/m² to about 1500 mg/m², from about 1125 mg/m² to about 1500 mg/m², from about 1130 mg/m² to about 1500 mg/m², from about 1135 mg/m² to about 1500 mg/m², from about 1140 mg/m² to about 1500 mg/m², from about 1145 mg/m² to about 1500 mg/m², from about 1150 mg/m² to about 1500 mg/m², from about 1155 mg/m² to about 1500 mg/m², from about 1160 mg/m² to about 1500 mg/m², from about 1165 mg/m² to about 1500 mg/m², from about 1170 mg/m² to about 1500 mg/m², from about 1175 mg/m² to about 1500 mg/m², from about 1180 mg/m² to about 1500 mg/m², from about 1185 mg/m² to about 1500 mg/m², from about 1190 mg/m² to about 1500 mg/m², from about 1195 mg/m² to about 1500 mg/m², from about 1200 mg/m² to about 1500 mg/m², from about 1205 mg/m² to about 1500 mg/m², from about 1210 mg/m² to about 1500 mg/m², from about 1215 mg/m² to about 1500 mg/m², from about 1220 mg/m² to about 1500 mg/m², from about 1225 mg/m² to about 1500 mg/m², from about 1230 mg/m² to about 1500 mg/m², from about 1235 mg/m² to about 1500 mg/m², from about 1240 mg/m² to about 1500 mg/m², from about 1245 mg/m² to about 1500 mg/m², from about 1250 mg/m² to about 1500 mg/m², from about 1255 mg/m² to about 1500 mg/m², from about 1260 mg/m² to about 1500 mg/m², from about 1265 mg/m² to about 1500 mg/m², from about 1270 mg/m² to about 1500 mg/m², from about 1275 mg/m² to about 1500 mg/m², from about 1280 mg/m² to about 1500 mg/m², from about 1285 mg/m² to about 1500 mg/m², from about 1290 mg/m² to about 1500 mg/m², from about 1295 mg/m² to about 1500 mg/m², from about 1300 mg/m² to about 1500 mg/m², from about 1305 mg/m² to about 1500 mg/m², from about 1310 mg/m² to about 1500 mg/m², from about 1315 mg/m² to about 1500 mg/m², from about 1320 mg/m² to about 1500 mg/m², from about 1325 mg/m² to about 1500 mg/m², from about 1330 mg/m² to about 1500 mg/m², from about 1335 mg/m² to about 1500 mg/m², from about 1340 mg/m² to about 1500 mg/m², from about 1345 mg/m² to about 1500 mg/m², from about 1350 mg/m² to about 1500 mg/m², from about 1355 mg/m² to about 1500 mg/m², from about 1360 mg/m² to about 1500 mg/m², from about 1365 mg/m² to about 1500 mg/m², from about 1370 mg/m² to about 1500 mg/m², from about 1375 mg/m² to about 1500 mg/m², from about 1380 mg/m² to about 1500 mg/m², from about 1385 mg/m² to about 1500 mg/m², from about 1390 mg/m² to about 1500 mg/m², from about 1395 mg/m² to about 1500 mg/m², from about 1400 mg/m² to about 1500 mg/m², from about 1405 mg/m² to about 1500 mg/m², from about 1410 mg/m² to about 1500 mg/m², from about 1415 mg/m² to about 1500 mg/m², from about 1420 mg/m² to about 1500 mg/m², from about 1425 mg/m² to about 1500 mg/m², from about 1430 mg/m² to about 1500 mg/m², from about 1435 mg/m² to about 1500 mg/m², from about 1440 mg/m² to about 1500 mg/m², from about 1445 mg/m² to about 1500 mg/m², from about 1450 mg/m² to about 1500 mg/m², from about 1455 mg/m² to about 1500 mg/m², from about 1460 mg/m² to about 1500 mg/m², from about 1465 mg/m² to about 1500 mg/m², from about 1470 mg/m² to about 1500 mg/m², from about 1475 mg/m² to about 1500 mg/m², from about 1480 mg/m² to about 1500 mg/m², from about 1485 mg/m² to about 1500 mg/m², from about 1490 mg/m² to about 1500 mg/m², and from about 1495 mg/m² to about 1500 mg/m².

### III. DOSAGE FORMS

Suitable pharmaceutical compositions for use with the methods of the present invention can be formulated into any dosage form that can be administered to a patient. In one embodiment, the pharmaceutical composition is in the form of an oral dosage unit or parenteral dosage unit. In one embodiment, the pharmaceutical composition is in the form of an oral dosage unit. In some embodiments, an oral dosage unit is fractionated into several, smaller doses, which are administered to a subject over a predetermined period of time in order to reduce toxicity of the therapeutic agent being administered. In some embodiments, an oral dosage unit is administered by a tablet or capsule comprising a controlled release formulation that can include a plurality of particles, granules, pellets, minitablets or tablets. In one embodiment, the pharmaceutical composition is in the form of a parenteral dosage unit. In one embodiment, the pharmaceutical composition is in the form of a parenteral dosage unit, wherein the parenteral dosage unit is selected from the group consisting of intravenous (IV), subcutaneous (SC), and intramuscular (M), rectal (PR) and transdermal dosage units. In one embodiment, the pharmaceutical composition is in a dosage form selected from the group consisting of sterile solutions, suspensions, suppositories, tablets and capsules. In one embodiment, the composition is an oral dosage form selected from the group consisting of a tablet, caplet, capsule, lozenge, syrup, liquid, suspension and elixir. In one embodiment, the composition is in an oral dosage form selected from the group consisting of tablets, hard shell capsules, soft gelatin capsules, beads, granules, aggregates, powders, gels, solids and semi-solids.

In some embodiments, suitable forms of pharmaceutical compositions for use in the methods of the present invention include dermatological compositions adapted for cutaneous topical administration. In some such embodiments, dermatological compositions include a cosmetically or pharmaceutically acceptable medium. In some embodiments, the dermatological compositions for topical administration can include ointments, lotions, creams, gels, drops, suppositories, sprays, liquids and powders. In some embodiments, conventional pharmaceutical carriers, aqueous, powder or oily bases, thickeners, skin enhancers and the like can be necessary or desirable and therefore can be used. Examples of suitable enhancers include ethers such as diethylene glycol monoethyl ether (available commercially as Transcutol^{®}) and diethylene glycol monomethyl ether; surfactants such as sodium laurate, sodium lauryl sulfate, cetyltrimethylammonium bromide, benzalkonium chloride, Poloxamer (231, 182, 184), Tween (20, 40, 60, 80), and lecithin (U.S. Patent No. 4,783,450); alcohols such as ethanol, propanol, octanol, benzyl alcohol, and the like; polyethylene glycol and esters thereof such as polyethylene glycol monolaurate; amides and other nitrogenous compounds such as urea, dimethylacetamide (DMA), dimethylformamide (DMF), 2-pyrrolidone, 1-methyl-2-pyrrolidone, ethanolamine, diethanolamine and triethanolamine; terpenes; alkanones; and organic acids, particularly citric acid and succinic acid. Azone^{®} and sulfoxides such as DMSO and C 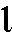 0MSO may also be used, but are less preferred.

In some embodiments, the pharmaceutical composition of the present invention is in a dosage form selected from the group consisting of sustained release forms, controlled release forms, delayed release forms and response release forms.

### IV. METHODS OF USE

All references to methods of treatment by therapy in this description are to be interpreted as references to the compounds and salts thereof of the present invention for use in those methods.

The compounds defined in the claims, and compositions comprising said compounds, are for use to treat one or more diseases selected from the group consisting of brain cancer, glioblastoma, astrocytoma, atypical teratoid tumors, atypical rhabdoid tumors, brain tumors, spinal cord tumors, central nervous system atypical teratoid tumors, central nervous system atypical rhabdoid tumors, leptomeningeal disease, central nervous system embryonal tumors, central nervous system lymphoma, chordoma, craniopharyngioma, embryonal tumors of central nervous system, ependymoblastoma, ependymoma, extracranial germ cell tumors, extragonadal germ cell tumors, germ cell tumors, glioma, primary central nervous system lymphoma, medulloblastoma, medulloepithelioma, neuroblastoma, pineal parenchymal tumors of intermediate differentiation, pineoblastoma, supratentorial primitive neuroectodermal tumors, pituitary tumors, and primary central nervous system lymphoma.

In some embodiments, the compound for use of the present invention are used as a first-line therapy (sometimes called primary therapy). In some embodiments, the compounds and methods of the present invention are used as a second-line therapy. In some embodiments, the compound for use and methods of the present invention are used as a third-line therapy. In some embodiments, the compound for use and methods of the present invention are used as a salvage therapy. The term "salvage therapy" as used herein means a therapeutic agent that can be taken with any regimen after a subject's initial treatment regimen has failed or after the subject's condition has not responded to an initial treatment. In some embodiments, the compound for use and methods of the present invention are used as a rescue therapy. In one embodiment of the rescue therapy, the compositions of the present invention are used as a rescue agent to counteract the action of an initial treatment. In one embodiment of the rescue therapy, the compound for use of the present invention are used as rescue agent which is administered to a subject who has developed resistance to a standard or an initial treatment. In some embodiments, the compound for use of the present invention are used as a neoadjuvant therapy. In one embodiment, the neoadjuvant therapy comprises administration of one or more of the therapeutic agents of the present invention to a subject before a main or first line treatment. In one embodiment, the neoadjuvant therapy reduces the size or extent of the cancer being treated before a main or first line treatment is administered to the subject undergoing treament. In some embodiments, the compound for use of the present invention are used as an adjuvant therapy. In one embodiment, the adjuvant therapy comprises administration of one or more therapeutic agents of the present invention to a subject, wherein the one or more therapeutic agent that modify the effect of other therapeutic agents that are already administered to the subject or are concurrently administered to the subject or subsequently administered to the subject.

In some embodiments, the compound for use of the present invention exhibit reduced chance of drug-drug interactions. In some embodiments, the compound for use, compound (**1**) di-HCl are eliminated from the patient's body before it can interact with another pharmaceutically active agent.

In some embodiments, the compound for use of the present invention, compound (1) di-HCl exhibit tonicity level that facilitates combinations with other pharamaceutical agents.

The utility of the compound for use of the present invention is not limited to any particular animal species. In one embodiment, a subject treated according to the present invention, can be mammalian or non-mammalian. In one embodiment, a mammalian subject can be any mammal including a human; a non-human primate; a rodent such as a mouse, rat, or guinea pig; a domesticated pet such as a cat or dog; a horse, cow, pig, sheep, goat, or rabbit. In one embodiment, a non-mammalian subject can be any non-mammal including a bird such as a duck, goose, chicken, or turkey. In one embodiment, subjects can be either gender and can be any age. The compound for usecan also be used to prevent one or more of the diseases recited in the claims.

The utility of the compound for use of the present invention is not limited to any particular age of the subject. In one embodiment, a subject treated according to methods and using compositions of the present invention can be over the age of 50 years, over the age of 55 years, over the age of 60 years, or over the age of 65 years. In one embodiment a subject treated according to methods and using compositions of the present invention can be under the age of 50 years, under the age of 55 years, under the age of 60 years, or under the age of 65 years.

In one embodiment the subject has received at least one prior therapeutic agent. In one embodiment the subject has received at least two, at least three, or at least four prior therapeutic agents. In one embodiment the prior therapeutic agent is ibrutinib, bortezomib, carfilzomib, temozolomide, bevacizumab, cyclophosphamide, hydroxydaunorubicin, vincristine, prednisone, cytarabine, cisplatin, rituximab, 5-fluorouracil, oxaliplatin, leucovorin, or lenalidomide.

In one embodiment the subject has been treated with radiation. In one embodiment the subject has been treated with surgery.

In some embodiments of the methods of treating a cancer, the cancer no longer responds to treatment with ibrutinib, bortezomib, carfilzomib, temozolomide, bevacizumab, cyclophosphamide, hydroxydaunorubicin, vincristine, prednisone, cytarabine, cisplatin, rituximab, 5-fluorouracil, oxaliplatin, leucovorin, lenalidomide, radiation, surgery, or a combination thereof.

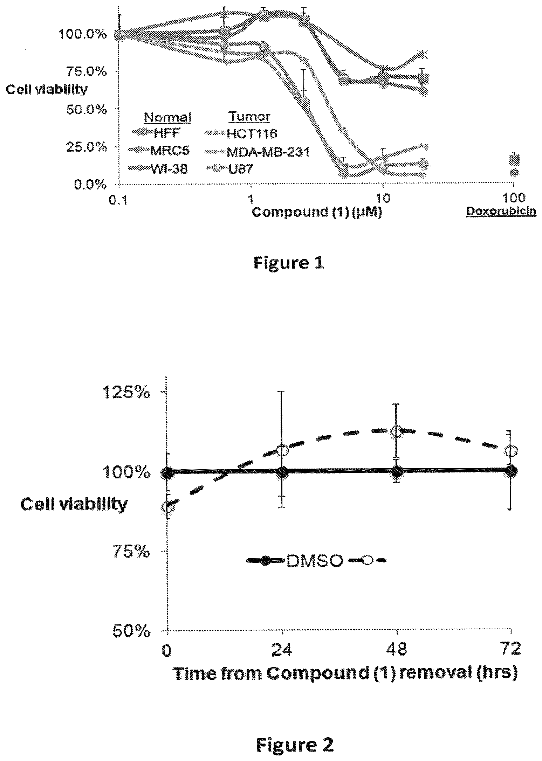

In some embodiments, the compound for useof the present invention have dose response relation in cancer cells that is different from dose response relation of the same the compositions and methods in normal cells. Figure 1, for example, illustrates the dose response relation in which effects of exemplary compound (**1**) on proliferation and cell death in normal and tumor cells. Figure 1 shows cell viability following treatment with exemplary compound (**1**) at indicated concentrations for 72 hours. The tumors tested included a human colon cancer cell line (HCT116), breast tumor cell line (MDA-MB-231), human primary glioblastoma cell line (U87). And the normal cells tested included human foreskin fibroblasts (HFF), human fetal lung fibroblast (MRC-5) cells, and human lung fibroblast cell line (WI-38). Doxorubicin was used as a positive control at 1 µg/mL in normal fibroblasts. As, shown in Figure 1, cell viability of normal cells tested is at least about 75% at about 1-5 mg/mL concentration of exemplary compound (**1**) whereas viability of tumor cells is significantly lower (e.g., at or below 50%) at the same concentration of exemplary compound (**1**). Moreover, as concentration of exemplary compound (**1**) is increased beyond about 5 mg/mL viability of tumor cells falls to below 25% whereas viability of normal cells remains at about 75%.

Figure 2 illustrates cell viability assay in human fetal lung fibroblast (MRC-5) cells following 72 hour treatment with exemplary compound (**1**) (5µM) or DMSO and the indicated recovery period in complete drug-free media after this treatment. The time points are given as time following removal of exemplary compound (**1**) after 72 hour treatment. As shown in Figure 2, cell recovery was seen with exemplary compound (**1**), but not with DMSO.

In some embodiments, the compound for use of the present invention has utility in treating cancer in a subject. In one embodiment, the compositions and methods of the present invention have utility in treating one or more of the diseases recited in the claims in a human subject. In one embodiment, the method of treatment comprises administering to a subject in need of such treatment: (i) a first therapeutic agent including a compound comprising compound (**1**) di-HCl in combination with (ii) a second therapeutic agent, wherein the first therapeutic agent and the second therapeutic agent are administered either simultaneously or sequentially. The second therapeutic agent can be any suitable therapeutic agent, including any of the pharmaceutically active agents disclosed in in this application. Compound (**1**) di-hydrochloride salt has the structure of compound (2):

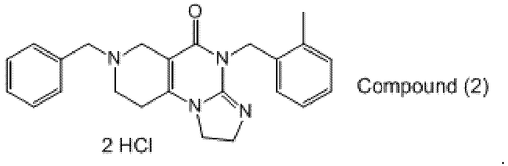

It is understood that compound (2) can be substitued for a compound (**1**) in any of the compositions or dosing regimens described hererin.

In some embodiments, the method of treatment of one or more of the diseases recited in the claims comprises administering to a subject in need of such treatment, a pharmaceutically effective amount of compound (**1**) di-HCl and a pharmaceutically acceptable carrier.

In some embodiments, the method of treatment of the present invention comprises administering a synergistic pharmaceutical combination, either simultaneously or sequentially, to a subject in need of such treatment, wherein the synergistic pharmaceutical combination comprising (i) a first therapeutic agent comprising compound (**1**) di-HCl; and (ii) a second therapeutic agent. In one embodiment, the method of treatment comprises administering to a subject in need of such treatment, either simultaneously or sequentially, therapeutically synergistic effective amounts of a first therapeutic agent comprising compound (**1**) di-HCl in combination with a second therapeutic agent. In one embodiment, the method of treatment comprises administering to a subject in need of such treatment an effective amount of a first therapeutic agent comprising compound (**1**) di-HCl in combination with an effective amount of a second therapeutic agent, wherein the combination provides a synergistic effect in the in vivo treatment of cancer sensitive to the combination, and wherein the first therapeutic agent and the second therapeutic agent are administered either simultaneously or sequentially. In one embodiment, the method of treatment comprises administering to a subject in need of such treatment an effective amount of a first therapeutic agent comprising compound (**1**) di-HCl in combination with an effective amount of a second therapeutic agent, wherein the combination provides a synergistic effect in the in vivo treatment of a minimal residual disease sensitive to the combination, and wherein the first therapeutic agent and the second therapeutic agent are administered either simultaneously or sequentially.

In some embodiments, the second drug can be given before or prior to compound (**1**) di-HCl.

In one embodiment, the method of treatment of the present invention targets cancer, wherein the cancer is selected from the group of diseases recited in the claims.

In one embodiment, the method of treatment of the present invention targets a solid tumor, wherein the solid tumor is selected from the group consisting of: Extracranial Germ Cell Tumor; Extragonadal Germ Cell Tumor; Germ Cell Tumor; Spinal Cord Tumors, Leptomeningeal Disease, Central Nervous System Embryonal Tumors, Chordoma, Embryonal Tumors of Central Nervous System, Ependymoblastoma, Ependymoma, Neuroblastoma; Pineal Parenchymal Tumors of Intermediate Differentiation, Pineoblastoma, Astrocytomas, Atypical Teratoid/Rhabdoid Tumor; Central Nervous System Atypical Teratoid/Rhabdoid Tumor, Craniopharyngioma, Glioma, Brain cancer, Medulloblastoma, Medulloepithelioma, Supratentorial Primitive Neuroectodermal Tumors, and Pituitary Tumor.

In one embodiment, the method of treatment of the present invention targets lymphoma, wherein the lymphoma is selected from the group consisting of: Central Nervous System Lymphoma and Primary Central Nervous System Lymphoma.

In one embodiment, the method of treatment of the present invention targets cancer, wherein the cancer is selected from the group consisting of Astrocytomas, and Atypical Teratoid/Rhabdoid Tumor.

In one embodiment, the method of treatment of the present invention targets cancer, wherein the cancer is selected from the group consisting of Brain Tumor, and Spinal Cord Tumors.

In one embodiment, the method of treatment of the present invention targets cancer, wherein the cancer is selected from the group consisting of Central Nervous System Atypical Teratoid/Rhabdoid Tumor, Central Nervous System Embryonal Tumors, Central Nervous System Lymphoma, Chordoma, and Craniopharyngioma.

In one embodiment, the method of treatment of the present invention targets cancer, wherein the cancer is selected from the group consisting of Embryonal Tumors of Central Nervous System, Ependymoblastoma, Ependymoma, Extracranial Germ Cell Tumor, and Extragonadal Germ Cell Tumor.

In one embodiment, the method of treatment of the present invention targets cancer, wherein the cancer is selected from the group consisting of Germ Cell Tumor, and Glioma.

The method of treating cancer according to claim 1, wherein the cancer is Primary Central Nervous System Lymphoma.

In one embodiment, the method of treatment of the present invention is useful for treating cancer, wherein the cancer is Neuroblastoma.

In one embodiment, the method of treatment of the present invention is useful for treating cancer, wherein the cancer is selected from the group consisting of Pineal Parenchymal Tumors of Intermediate Differentiation, Pineoblastoma and Supratentorial Primitive Neuroectodermal Tumors, Pituitary Tumor, and Primary Central Nervous System Lymphoma.

In one embodiment, the method of treatment of the present invention is useful for treating cancer, wherein the cancer is Supratentorial Primitive Neuroectodermal Tumors.

In some embodiments, treatment of cancer comprises prevention of tumor growth in a cancer subject. In some embodiments, treatment of cancer comprises prevention of formation of cancer metastases in a cancer subject. In some embodiments, treatment of cancer comprises targeted treatment of minimal residual disease in a cancer subject known to have the minimal residual disease in a cancer or a subject at risk for having minimal residual disease.

This might be indicated after treatment of the primary tumor by surgery and/or after chemotherapy (e.g. radiotherapy) has been initiated or determined to efficaceous. Disseminated tumor cells may be in their dormant state and often cannot be attacked by the chemotherapy (radiotherapy). A thus treated patient seemingly is in a healed state, which is also described as "minimal residual disease". Nevertheless, the dormant tumor cells have a potential of forming metastases if they become metastasising cells due to a growth stimulus also after a longer dormant state.

As used herein, "minimal residual disease" denotes a small number of cancer cells that remain in in a subject during treatment, or after treatment when the subject is in remission (exhibiting no symptoms or signs of the disease). The methods described herein are preferably applied to any form of the diseases listed herein, including adult and childhood forms of these diseases.

In one embodiment, the first therapeutic agent includes compound (1) di-HCl. In one embodiment, the first therapeutic agent is compound (**1**) in the form of a hydrochloride di-salt (i.e., di-hydrochloride salt).

In some embodiments of the method of treatment, the second therapeutic agent includes an anti-cancer agent. In some embodiments of the method of treatment, the second therapeutic agent is selected, without limitation, from acivicin, aclarubicin, acodazole, acronine, adozelesin, aldesleukin, alitretinoin, allopurinol, altretamine, ambomycin, ametantrone, amifostine, aminoglutethimide, amsacrine, anastrozole, anthramycin, arsenic trioxide, asparaginase, asperlin, azacitidine, azetepa, azotomycin, batimastat, benzodepa, bevacizumab, bicalutamide, bisantrene, bisnafide dimesylate, bizelesin, bleomycin, brequinar, bropirimine, busulfan, cactinomycin, calusterone, capecitabine, caracemide, carbetimer, carboplatin, carmustine, carubicin, carzelesin, cedefingol, celecoxib, chlorambucil, cirolemycin, cisplatin, cladribine, crisnatol mesylate, cyclophosphamide, cytarabine, dacarbazine, dactinomycin, daunorubicin, decitabine, dexormaplatin, dezaguanine, dezaguanine mesylate, diaziquone, docetaxel, doxorubicin, droloxifene, dromostanolone, duazomycin, edatrexate, eflomithine, elsamitrucin, enloplatin, enpromate, epipropidine, epirubicin, erbulozole, esorubicin, estramustine, etanidazole, etoposide, etoprine, fadrozole, fazarabine, fenretinide, floxuridine, fludarabine, fluorouracil, flurocitabine, fosquidone, fostriecin, fulvestrant, gemcitabine, hydroxyurea, idarubicin, ifosfamide, ilmofosine, interleukin II (IL-2, including recombinant interleukin II or rIL2), interferon alfa-2a, interferon alfa-2b, interferon alfa-n1, interferon alfa-n3, interferon beta-Ia, interferon gamma-Ib, iproplatin, irinotecan, lanreotide, letrozole, leuprolide, liarozole, lometrexol, lomustine, losoxantrone, masoprocol, maytansine, mechlorethamine hydrochlride, megestrol, melengestrol acetate, melphalan, menogaril, mercaptopurine, methotrexate, metoprine, meturedepa, mitindomide, mitocarcin, mitocromin, mitogillin, mitomalcin, mitomycin, mitosper, mitotane, mitoxantrone, mycophenolic acid, nelarabine, nocodazole, nogalamycin, ormnaplatin, oxisuran, paclitaxel, pegaspargase, peliomycin, pentamustine, peplomycin, perfosfamide, pipobroman, piposulfan, piroxantrone hydrochloride, plicamycin, plomestane, porfimer, porfiromycin, prednimustine, procarbazine, puromycin, pyrazofurin, riboprine, rogletimide, safingol, semustine, simtrazene, sparfosate, sparsomycin, spirogermanium, spiromustine, spiroplatin, streptonigrin, streptozocin, sulofenur, talisomycin, tamoxifen, tecogalan, tegafur, teloxantrone, temoporfin, teniposide, teroxirone, testolactone, thiamiprine, thioguanine, thiotepa, tiazofurin, tirapazamine, topotecan, toremifene, trestolone, triciribine, trimetrexate, triptorelin, tubulozole, uracil mustard, uredepa, vapreotide, verteporfin, vinblastine, vincristine sulfate, vindesine, vinepidine, vinglycinate, vinleurosine, vinorelbine, vinrosidine, vinzolidine, vorozole, zeniplatin, zinostatin, zoledronate, zorubicin and combinations thereof.

In some embodiments of the method of treatment, the second therapeutic agent is selected, without limitation, from hormone analogues and antihormones, aromatase inhibitors, LHRH agonists and antagonists, inhibitors of growth factors, growth factor antibodies, growth factor receptor antibodies, tyrosine kinase inhibitors; antimetabolites; antitumour antibiotics; platinum derivatives; alkylation agents; antimitotic agents; tubuline inhibitors; PARP inhibitors, topoisomerase inhibitors, serine/threonine kinase inhibitors, tyrosine kinase inhibitors, protein protein interaction inhibitors, MEK inhibitors, ERK inhibitors, IGF-1R inhibitors, ErbB receptor inhibitors, rapamycin analogs, amifostin, anagrelid, clodronat, filgrastin, interferon, interferon alpha, leucovorin, rituximab, procarbazine, levamisole, mesna, mitotane, pamidronate and porfimer, 2-chlorodesoxyadenosine, 2-fluorodesoxy-cytidine, 2-methoxyoestradiol, 2C4,3-alethine, 131-1-TM-601, 3CPA, 7-ethyl-10-hydroxycamptothecin, 16-aza-epothilone B, A 105972, A 204197, abiraterone, aldesleukin, alitretinoin, allovectin-7, altretamine, alvocidib, amonafide, anthrapyrazole, AG-2037, AP-5280, apaziquone, apomine, aranose, arglabin, arzoxifene, atamestane, atrasentan, auristatin PE, AVLB, AZ10992, ABX-EGF, AMG-479 (ganitumab), ARRY 162, ARRY 438162, ARRY-300, ARRY-142886/AZD-6244 (selumetinib), ARRY-704/AZD-8330, AR-12, AR-42, AS-703988, AXL-1717, AZD-8055, AZD-5363, AZD-6244, ARQ-736, ARQ 680, AS-703026 (primasertib), avastin, AZD-2014, azacytidine, azaepothilone B, azonafide, BAY-43-9006, BAY 80-6946, BBR-3464, BBR-3576, bevacizumab, BEZ-235, biricodar dicitrate, BCX-1777, BKM-120, bleocin, BLP-25, BMS-184476, BMS-247550, BMS-188797, BMS-275291, BMS-663513, BMS-754807, BNP-1350, BNP-7787, BIBW 2992 (afatinib, tomtovok), BIBF 1120 (vargatef), BI 836845, BI 2536, BI 6727, BI 836845, BI 847325, BI 853520, BUB-022, bleomycinic acid, bleomycin A, bleomycin B, brivanib, bryostatin-1, bortezomib, brostallicin, busulphan, BYL-719, CA-4 prodrug, CA-4, CapCell, calcitriol, canertinib, canfosfamide, capecitabine, carboxyphthalatoplatin, CCl-779, CC-115, CC-223, CEP-701, CEP-751, CBT-1 cefixime, ceflatonin, ceftriaxone, celecoxib, celmoleukin, cemadotin, CH4987655/RO-4987655, chlorotrianisene, cilengitide, ciclosporin, CDA-II, CDC-394, CKD-602, CKI-27, clofarabin, colchicin, combretastatin A4, COT inhibitors, CHS-828, CH-5132799, CLL-Thera, CMT-3 cryptophycin 52, CTP-37, CTLA-4 monoclonal antibodies, CP-461, CV-247, cyanomorpholinodoxorubicin, cytarabine, D 24851, decitabine, deoxorubicin, deoxyrubicin, deoxycoformycin, depsipeptide, desoxyepothilone B, dexamethasone, dexrazoxanet, diethylstilbestrol, diflomotecan, didox, DMDC, dolastatin 10, doranidazole, DS-7423, E7010, E-6201, edatrexat, edotreotide, efaproxiral, eflornithine, EGFR inhibitors, EKB-569, EKB-509, enzastaurin, enzalutamide, elsamitrucin, epothilone B, epratuzumab, ER-86526, erlotinib, ET-18-0CH3, ethynylcytidine, ethynyloestradiol, exatecan, exatecan mesylate, exemestane, exisulind, fenretinide, figitumumab, floxuridine, folic acid, FOLFOX, FOLFOX4, FOLFIRI, formestane, fotemustine, galarubicin, gallium maltolate, gefinitib, gemtuzumab, gimatecan, glufosfamide, GCS-100, GDC-0623, GDC-0941 (pictrelisib), GDC-0980, GDC-0032, GDC-0068, GDC-0349, GDC-0879, G17DT immunogen, GMK, GPX-100, gp100-peptide vaccines, GSK-5126766, GSK-690693, GSK-1120212 (trametinib), GSK-2118436 (dabrafenib), GSK-2126458, GSK-2132231A, GSK-2334470, GSK-2110183, GSK-2141795, GW2016, granisetron, herceptine, hexamethylmelamine, histamine, homoharringtonine, hyaluronic acid, hydroxyurea, hydroxyprogesterone caproate, ibandronate, ibritumomab, idatrexate, idenestrol, IDN-5109, IGF-1R inhibitors, IMC-1C11, IMC-A12 (cixutumumab), immunol, indisulam, interferon alpha-2a, interferon alpha-2b, pegylated interferon alpha-2b, interleukin-2, INK-1117, INK-128, INSM-18, ionafarnib, ipilimumab, iproplatin, irofulven, isohomohalichondrin-B, isoflavone, isotretinoin, ixabepilone, JRX-2, JSF-154, J-107088, conjugated oestrogens, kahalid F, ketoconazole, KW-2170, KW-2450, lobaplatin, leflunomide, lenograstim, leuprolide, leuporelin, lexidronam, LGD-1550, linezolid, lutetium texaphyrin, lometrexol, losoxantrone, LU 223651, lurtotecan, LY-S6AKT1, LY-2780301, mafosfamide, marimastat, mechloroethamine, MEK inhibitors, MEK-162, methyltestosteron, methylprednisolone, MEDI-573, MEN-10755, MDX-H210, MDX-447, MDX-1379, MGV, midostaurin, minodronic acid, mitomycin, mivobulin, MK-2206, MK-0646 (dalotuzumab), MLN518, motexaf in gadolinium, MS-209, MS-275, MX6, neridronate, neratinib, Nexavar, neovastat, nilotinib, nimesulide, nitroglycerin, nolatrexed, norelin, N-acetylcysteine, 06-benzylguanine, oblimersen, omeprazole, oncophage, oncoVEXGM-CSF, ormiplatin, ortataxel, OX44 antibodies, OSI-027, OSI-906 (linsitinib), 4-1BB antibodies, oxantrazole, oestrogen, panitumumab, patupilone, pegfilgrastim, PCK-3145, pegfilgrastim, PBI-1402, PBI-05204, PDO325901, PD-1 antibodies, PEG-paclitaxel, albumin-stabilized paclitaxel, PEP-005, PF-05197281, PF-05212384, PF-04691502, PHT-427, P-04, PKC412, P54, PI-88, pelitinib, pemetrexed, pentrix, perifosine, perillylalcohol, pertuzumab, PI3K inhibitors, PI3K/mTOR inhibitors, PG-TXL, PG2, PLX-4032/RO-5185426 (vemurafenib), PLX-3603/RO-5212054, PT-100, PWT-33597, PX-866, picoplatin, pivaloyloxymethylbutyrate, pixantrone, phenoxodiol O, PKI166, plevitrexed, plicamycin, polyprenic acid, porfiromycin, prednisone, prednisolone, quinamed, quinupristin, R115777, RAF-265, ramosetron, ranpirnase, RDEA-119/BAY 869766, RDEA-436, rebeccamycin analogues, receptor tyrosine kinase (RTK) inhibitors, revimid, RG-7167, RG-7304, RG-7421, RG-7321, RG 7440, rhizoxin, rhu-MAb, rinfabate, risedronate, rituximab, robatumumab, rofecoxib, RO-31-7453, RO-5126766, RO-5068760, RPR 109881A, rubidazone, rubitecan, R-flurbiprofen, RX-0201, S-9788, sabarubicin, SAHA, sargramostim, satraplatin, SB 408075, Se-015/Ve-015, SU5416, SU6668, SDX-101, semustin, seocalcitol, SM-11355, SN-38, SN-4071, SR-27897, SR-31747, SR-13668, SRL-172, sorafenib, spiroplatin, squalamine, suberanilohydroxamic acid, sutent, T 900607, T 138067, TAK-733, TAS-103, tacedinaline, talaporf in, Tarceva, tariquitar, tasisulam, taxotere, taxoprexin, tazarotene, tegafur, temozolamide, tesmilifene, testosterone, testosterone propionate, tesmilifene, tetraplatin, tetrodotoxin, tezacitabine, thalidomide, theralux, therarubicin, thymalfasin, thymectacin, tiazofurin, tipifarnib, tirapazamine, tocladesine, tomudex, toremofin, trabectedin, TransMID-107, transretinic acid, traszutumab, tremelimumab, tretinoin, triacetyluridine, triapine, triciribine, trimetrexate, TLK-286TXD 258, tykerb/tyverb, urocidin, valrubicin, vatalanib, vincristine, vinflunine, virulizin, WX-UK1, WX-554, vectibix, xeloda, XELOX, XL-147, XL-228, XL-281, XL-518/R-7420/GDC-0973, XL-765, YM-511, YM-598, ZD-4190, ZD-6474, ZD-4054, ZD-0473, ZD-6126, ZD-9331, ZD1839, ZSTK-474, zoledronat, zosuquidar, and combinations thereof.

In some embodiments of the method of treatment, the second therapeutic agent is selected from the group consisting of tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxy-progesterone, octreotide, and combinations thereof. In some embodiments of the method of treatment, the second therapeutic agent is selected, without limitation, from the group consisting of LHRH agonists and LHRH antagonists. In some embodiments of the method of treatment, the second therapeutic agent includes a LHRH agonist selected from the group consisting of goserelin acetate, luprolide acetate, triptorelin pamoate and combinations thereof. In some embodiments of the method of treatment, the second therapeutic agent includes a LHRH antagonist selected from the group consisting of wherein the LHRH antagonists are selected from the group consisting of Degarelix, Cetrorelix, Abarelix, Ozarelix, Degarelix combinations thereof. In some embodiments of the method of treatment, the second therapeutic agent includes an inhibitor of a growth factor. In some embodiments of the method of treatment, of the second therapeutic agent, the inhibitor of the growth factor is selected, without limitation, from the group consisting of inhibitors of: platelet derived growth factor (PDGF), fibroblast growth factor (FGF), vascular endothelial growth factor (VEGF), epidermal growth factor (EGF), insuline-like growth factors (IGF), human epidermal growth factor (HER), hepatocyte growth factor (HGF), and combinations thereof. In some embodiments of the method of treatment, the second therapeutic agent includes

In some embodiments of the method of treatment, the second therapeutic agent includes an inhibitor of a growth factor. In some embodiments of the method of treatment, the second therapeutic agent includes an inhibitor of the human epidermal growth factor (HER). In some embodiments of the method of treatment, the second therapeutic agent includes an inhibitor of a growth factor selected from the group consisting of: platelet derived growth factor (PDGF), fibroblast growth factor (FGF), vascular endothelial growth factor (VEGF), epidermal growth factor (EGF), insuline-like growth factors (IGF), human epidermal growth factor (HER) and hepatocyte growth factor (HGF). In some embodiments of the method of treatment, the second therapeutic agent includes n inhibitor of the human epidermal growth factor (HER). In some embodiments of the method of treatment, the second therapeutic agent includes an inhibitor of the human epidermal growth factor (HER) selected from the group consisting of HER2, HER3, and HER4. In some embodiments of the method of treatment, the second therapeutic agent includes a tyrosine kinase inhibitor. In some embodiments of the method of treatment, the second therapeutic agent includes a tyrosine kinase inhibitor selected, without limitation, from the group consisting of cetuximab, gefitinib, imatinib, lapatinib and trastuzumab, and combinations thereof. In some embodiments of the method of treatment, the second therapeutic agent includes an aromatase inhibitor. In some embodiments of the method of treatment, the second therapeutic agent includes an aromatase inhibitor selected from the group consisting of anastrozole, letrozole, liarozole, vorozole, exemestane, atamestane, and combinations thereof.

In some embodiments of the method of treatment, the second therapeutic agent includes an antimetabolite. In some embodiments of the method of treatment, the second therapeutic agent includes an antimetabolite that comprises an antifolate. In some embodiments of the method of treatment, the second therapeutic agent includes an antifolate selected from the group consisting of methotrexate, raltitrexed, pyrimidine analogues, and combinations thereof. In some embodiments of the method of treatment, the second therapeutic agent includes an antimetabolite that is pyrimidine analogue. In some embodiments of the method of treatment, the second therapeutic agent includes a pyrimidine analogue selected from the group consisting of 5-fluorouracil, capecitabin, gemcitabin, and combination thereof. In some embodiments of the method of treatment, the second therapeutic agent includes an antimetabolite that is a purine analogue or adenosine analogue. In some embodiments of the method of treatment, the second therapeutic agent includes a a purine analogue or adenosine analogue selected from the group consisting of mercaptopurine, thioguanine, cladribine and pentostatin, cytarabine, fludarabine, and combinations thereof. In some embodiments of the method of treatment, the second therapeutic agent includes an antitumour antibiotic. In some embodiments of the method of treatment, the antitumor antibiotic is selected from the group consisting of anthracyclins, doxorubicin, daunorubicin, epirubicin and idarubicin, mitomycin-C, bleomycin, dactinomycin, plicamycin, streptozocin and combinations thereof. In some embodiments of the method of treatment, the second therapeutic agent includes a platinum derivative. In some embodiments of the method of treatment, the platinum derivative is selected from the group consisting of cisplatin, oxaliplatin, carboplatin and combinations thereof. In some embodiments of the method of treatment, the second therapeutic agent includes an alkylation agent. In some embodiments of the method of treatment, the second therapeutic agent includes an alkylation agent selected from the group consisting of estramustin, meclorethamine, melphalan, chlorambucil, busulphan, dacarbazin, cyclophosphamide, ifosfamide, temozolomide, nitrosoureas, and combinations thereof. In some embodiments of the method of treatment, the second therapeutic agent includes a nitrosourea. In some embodiments of the method of treatment, the second therapeutic agent includes a nitrosourea selected from the group consisting of carmustin, lomustin, thiotepa, and combinations thereof. In some embodiments of the method of treatment, the second therapeutic agent includes an antimitotic agent. In some embodiments of the method of treatment, the second therapeutic agent includes an antimitotic agent selected from the group consisting of Vinca alkaloids and taxanes. In some embodiments of the method of treatment, the second therapeutic agent includes a taxane selected from the group consisting of paclitaxel, docetaxel, and combinations thereof. In some embodiments of the method of treatment, the second therapeutic agent includes a Vinca alkaloids are selected from the group consisting of vinblastine, vindesin, vinorelbin, vincristine, and combinations thereof. In some embodiments of the method of treatment, the second therapeutic agent includes a topoisomerase inhibitor. In some embodiments of the method of treatment, the second therapeutic agent includes a topoisomerase inhibitor which is an epipodophyllotoxin. In some embodiments of the method of treatment, the second therapeutic agent includes a topoisomerase inhibitor, which is a epipodophyllotoxins selected from the group consisting of etoposide and etopophos, teniposide, amsacrin, topotecan, irinotecan, mitoxantron, and combinations thereof. In some embodiments of the method of treatment, the second therapeutic agent includes a serine/threonine kinase inhibitor. In some embodiments of the method of treatment, the second therapeutic agent includes a serine/threonine kinase inhibitor selected from the group consisting of PDK 1 inhibitors, B-Raf inhibitors, mTOR inhibitors, mTORC1 inhibitors, PI3K inhibitors, dual mTOR/PI3K inhibitors, STK 33 inhibitors, AKT inhibitors, PLK 1 inhibitors, inhibitors of CDKs, Aurora kinase inhibitors, and combinations thereof. In some embodiments of the method of treatment, the second therapeutic agent includes a tyrosine kinase inhibitor. In some embodiments of the method of treatment, the second therapeutic agent includes a PTK2/FAK inhibitor. In some embodiments of the method of treatment, the second therapeutic agent includes a protein protein interaction inhibitor. In some embodiments of the method of treatment, the second therapeutic agent includes a protein protein interaction inhibitor selected from the group consisting of IAP, Mcl-1, MDM2/MDMX and combinations thereof. In some embodiments of the method of treatment, the second therapeutic agent includes a rapamycin analog. In some embodiments of the method of treatment, the second therapeutic agent includes a rapamycin analog selected from the group consisting of everolimus, temsirolimus, ridaforolimus, sirolimus, and combinations thereof. In some embodiments of the method of treatment, the second therapeutic agent is selected from the group consisting of amifostin, anagrelid, clodronat, filgrastin, interferon, interferon alpha, leucovorin, rituximab, procarbazine, levamisole, mesna, mitotane, pamidronate and porfimer, and combinations thereof. In some embodiments of the method of treatment, the second therapeutic agent is selected from the group consisting of 2-chlorodesoxyadenosine, 2-fluorodesoxy-cytidine, 2-methoxyoestradiol, 2C4,3-alethine, 131-1-TM-601, 3CPA, 7-ethyl-10-hydroxycamptothecin, 16-aza-epothilone B, A 105972, A 204197, abiraterone, aldesleukin, alitretinoin, allovectin-7, altretamine, alvocidib, amonafide, anthrapyrazole, AG-2037, AP-5280, apaziquone, apomine, aranose, arglabin, arzoxifene, atamestane, atrasentan, auristatin PE, AVLB, AZ10992, ABX-EGF, AMG-479 (ganitumab), ARRY 162, ARRY 438162, ARRY-300, ARRY-142886/AZD-6244 (selumetinib), ARRY-704/AZD-8330, AR-12, AR-42, AS-703988, AXL-1717, AZD-8055, AZD-5363, AZD-6244, ARQ-736, ARQ 680, AS-703026 (primasertib), avastin, AZD-2014, azacytidine, azaepothilone B, azonafide, BAY-43-9006, BAY 80-6946, BBR-3464, BBR-3576, bevacizumab, BEZ-235, biricodar dicitrate, BCX-1777, BKM-120, bleocin, BLP-25, BMS-184476, BMS-247550, BMS-188797, BMS-275291, BMS-663513, BMS-754807, BNP-1350, BNP-7787, BIBW 2992 (afatinib, tomtovok), BIBF 1120 (vargatef), BI 836845, BI 2536, BI 6727, BI 836845, BI 847325, BI 853520, BUB-022, bleomycinic acid, bleomycin A, bleomycin B, brivanib, bryostatin-1, bortezomib, brostallicin, busulphan, BYL-719, CA-4 prodrug, CA-4, CapCell, calcitriol, canertinib, canfosfamide, capecitabine, carboxyphthalatoplatin, CCl-779, CC-115, CC-223, CEP-701, CEP-751, CBT-1 cefixime, ceflatonin, ceftriaxone, celecoxib, celmoleukin, cemadotin, CH4987655/RO-4987655, chlorotrianisene, cilengitide, ciclosporin, CDA-II, CDC-394, CKD-602, CKI-27, clofarabin, colchicin, combretastatin A4, COT inhibitors, CHS-828, CH-5132799, CLL-Thera, CMT-3 cryptophycin 52, CTP-37, CTLA-4 monoclonal antibodies, CP-461, CV-247, cyanomorpholinodoxorubicin, cytarabine, D 24851, decitabine, deoxorubicin, deoxyrubicin, deoxycoformycin, depsipeptide, desoxyepothilone B, dexamethasone, dexrazoxanet, diethylstilbestrol, diflomotecan, didox, DMDC, dolastatin 10, doranidazole, DS-7423, E7010, E-6201, edatrexat, edotreotide, efaproxiral, eflornithine, EGFR inhibitors, EKB-569, EKB-509, enzastaurin, enzalutamide, elsamitrucin, epothilone B, epratuzumab, ER-86526, erlotinib, ET-18-0CH3, ethynylcytidine, ethynyloestradiol, exatecan, exatecan mesylate, exemestane, exisulind, fenretinide, figitumumab, floxuridine, folic acid, FOLFOX, FOLFOX4, FOLFIRI, formestane, fotemustine, galarubicin, gallium maltolate, gefinitib, gemtuzumab, gimatecan, glufosfamide, GCS-100, GDC-0623, GDC-0941 (pictrelisib), GDC-0980, GDC-0032, GDC-0068, GDC-0349, GDC-0879, G17DT immunogen, GMK, GPX-100, gp100-peptide vaccines, GSK-5126766, GSK-690693, GSK-1120212 (trametinib), GSK-2118436 (dabrafenib), GSK-2126458, GSK-2132231A, GSK-2334470, GSK-2110183, GSK-2141795, GW2016, granisetron, herceptine, hexamethylmelamine, histamine, homoharringtonine, hyaluronic acid, hydroxyurea, hydroxyprogesterone caproate, ibandronate, ibritumomab, idatrexate, idenestrol, IDN-5109, IGF-1R inhibitors, IMC-1C11, IMC-A12 (cixutumumab), immunol, indisulam, interferon alpha-2a, interferon alpha-2b, pegylated interferon alpha-2b, interleukin-2, INK-1117, INK-128, INSM-18, ionafarnib, ipilimumab, iproplatin, irofulven, isohomohalichondrin-B, isoflavone, isotretinoin, ixabepilone, JRX-2, JSF-154, J-107088, conjugated oestrogens, kahalid F, ketoconazole, KW-2170, KW-2450, lobaplatin, leflunomide, lenograstim, leuprolide, leuporelin, lexidronam, LGD-1550, linezolid, lutetium texaphyrin, lometrexol, losoxantrone, LU 223651, lurtotecan, LY-S6AKT1, LY-2780301, mafosfamide, marimastat, mechloroethamine, MEK inhibitors, MEK-162, methyltestosteron, methylprednisolone, MEDI-573, MEN-10755, MDX-H210, MDX-447, MDX-1379, MGV, midostaurin, minodronic acid, mitomycin, mivobulin, MK-2206, MK-0646 (dalotuzumab), MLN518, motexaf in gadolinium, MS-209, MS-275, MX6, neridronate, neratinib, Nexavar, neovastat, nilotinib, nimesulide, nitroglycerin, nolatrexed, norelin, N-acetylcysteine, 06-benzylguanine, oblimersen, omeprazole, oncophage, oncoVEXGM-CSF, ormiplatin, ortataxel, OX44 antibodies, OSI-027, OSI-906 (linsitinib), 4-1BB antibodies, oxantrazole, oestrogen, panitumumab, patupilone, pegfilgrastim, PCK-3145, pegfilgrastim, PBI-1402, PBI-05204, PDO325901, PD-1 antibodies, PEG-paclitaxel, albumin-stabilized paclitaxel, PEP-005, PF-05197281, PF-05212384, PF-04691502, PHT-427, P-04, PKC412, P54, PI-88, pelitinib, pemetrexed, pentrix, perifosine, perillylalcohol, pertuzumab, PI3K inhibitors, PI3K/mTOR inhibitors, PG-TXL, PG2, PLX-4032/RO-5185426 (vemurafenib), PLX-3603/RO-5212054, PT-100, PWT-33597, PX-866, picoplatin, pivaloyloxymethylbutyrate, pixantrone, phenoxodiol O, PKI166, plevitrexed, plicamycin, polyprenic acid, porfiromycin, prednisone, prednisolone, quinamed, quinupristin, R115777, RAF-265, ramosetron, ranpirnase, RDEA-119/BAY 869766, RDEA-436, rebeccamycin analogues, receptor tyrosine kinase (RTK) inhibitors, revimid, RG-7167, RG-7304, RG-7421, RG-7321, RG 7440, rhizoxin, rhu-MAb, rinfabate, risedronate, rituximab, robatumumab, rofecoxib, RO-31-7453, RO-5126766, RO-5068760, RPR 109881A, rubidazone, rubitecan, R-flurbiprofen, RX-0201, S-9788, sabarubicin, SAHA, sargramostim, satraplatin, SB 408075, Se-015/Ve-015, SU5416, SU6668, SDX-101, semustin, seocalcitol, SM-11355, SN-38, SN-4071, SR-27897, SR-31747, SR-13668, SRL-172, sorafenib, spiroplatin, squalamine, suberanilohydroxamic acid, sutent, T 900607, T 138067, TAK-733, TAS-103, tacedinaline, talaporf in, Tarceva, tariquitar, tasisulam, taxotere, taxoprexin, tazarotene, tegafur, temozolamide, tesmilifene, testosterone, testosterone propionate, tesmilifene, tetraplatin, tetrodotoxin, tezacitabine, thalidomide, theralux, therarubicin, thymalfasin, thymectacin, tiazofurin, tipifarnib, tirapazamine, tocladesine, tomudex, toremofin, trabectedin, TransMID-107, transretinic acid, traszutumab, tremelimumab, tretinoin, triacetyluridine, triapine, triciribine, trimetrexate, TLK-286TXD 258, tykerb/tyverb, urocidin, valrubicin, vatalanib, vincristine, vinflunine, virulizin, WX-UK1, WX-554, vectibix, xeloda, XELOX, XL-147, XL-228, XL-281, XL-518/R-7420/GDC-0973, XL-765, YM-511, YM-598, ZD-4190, ZD-6474, ZD-4054, ZD-0473, ZD-6126, ZD-9331, ZD1839, ZSTK-474, zoledronat, zosuquidar, and combinations thereof.

In some embodiments, the at least one other therapeutic agent comprises a steroid. Steroids include dexamethasone, prednisolone, methyl prednisolone, prednisone, hydrocortisone, triamcinolone, betamethasone, and cortivazol. In some embodiments, the at least one other therapeutic agent comprises an anti-emetic. Anti-emetics include 5-HT3 receptor agonists (such as dolasetron, granisetron, ondansetron, tropisetron, palonosetron, and mirtazapine), dopamine agonists (such as domperidone, olanzapine, droperidol, haloperidol, chlorpromazine, prochlorperazine, alizapride, prochlorperazine, and metoclopramide), NK1 receptor antagonists (such as aprepitant and casopitant), antihistamines (such as cyclizine, diphenhydramine, dimenhydrinate, doxylamine, meclizine, promethazine, hydroxyzine), cannabinoids (such as cannabis, dronabinol, nabilone, and sativex), benzodiazepines (such as midazolam and lorazepam), anticholinergics (such as hyoscine), trimethobenzamide, ginger, emetrol, propofol, peppermint, muscimol, and ajwain.

The pharmaceutical compositions may be administered to a subject via any suitable route of administration. The pharmaceutical composition may be administered to a subject orally, parenterally, transdermally or transmucosally. The pharmaceutical composition may be administered to a subject in a parenteral dosage form. The pharmaceutical composition may be administered to a subject as a parenterally. The pharmaceutical composition may be administered to a subject via a parenteral route of administration selected from the group consisting of intravenous (IV), subcutaneous (SC), and intramuscular (IM). The pharmaceutical compositionmay be administered to a subject via a route of administration selected from rectal (PR) and transdermal. The pharmaceutical composition may be administered to a subject in a dosage form selected from the group consisting of sterile solutions, suspensions, suppositories, tablets and capsules. The pharmaceutical composition may be administered to a subject in an oral dosage form selected from the group consisting of a tablet, caplet, capsule, lozenge, syrup, liquid, suspension and elixir. The pharmaceutical composition may be administered to a subject in an oral dosage form selected from the group consisting of tablets, hard shell capsules, soft gelatin capsules, beads, granules, aggregates, powders, gels, solids and semi-solids.

The pharmaceutical composition may be administered to a subject as a dosage form selected from the group consisting of sustained release forms, controlled release forms, delayed release forms and response release forms.

The pharmaceutical composition may be administered to a subject once daily. A pharmaceutical composition may be administered to a subject accoridng to an infrequent dosing regimen (e.g., administered once per week or less frequently). A pharmaceutical composition may be administered to a subject accoridng to a frequent dosing regimen (e.g., administered more than once per week). The pharmaceutical composition may be administered to a subject once weekly. The pharmaceutical composition may be administered to a subject once every four weeks. The pharmaceutical composition may be administered to a subject twice a week. The pharmaceutical composition may be administered to a subject once every two weeks. The pharmaceutical composition may be administered to a subject once every three weeks. The pharmaceutical composition may be administered to a subject in a repeated cycle of once weekly, once every two weeks, once every three weeks, once every four weeks or combinations thereof.

In one embodiment, the method of treatment comprises administering to a subject in need of such treatment: (i) a first therapeutic agent including a compound comprising compound (1) di-HCl in combination with (ii) a second therapeutic agent, wherein the first therapeutic agent and the second therapeutic agent are administered either simultaneously or sequentially; and further comprises assaying the expression of an endoplasmic reticulum (ER) stress response genes in a biological sample. In some embodiments the endoplasmic reticulum stress response gene is selected from the group that includes C/EBP-Homologous Protein (CHOP), Activating Transcription Factor 3 (ATF3) and both CHOP and ATF3. The biological sample may be tumor, peripheral blood mononuclear cells, or skin biopsy. The biological sample may be obtained before, during, or after drug administration. In some embodiments, the method of treatment further comprises adjusting a dose of compound (1) to achieve induction of about 50%, 75%, 100%, 125%, 150%, 175%, 200%, 225%, 250%, 275%, 300%, 325%, 350%, 375%, 400%, 425%, 450%, 475%, 500%, 525%, 550%, 575%, 600% ,or greater than 600% of one or more ER stress gene(s). In some embodiments, the method of treatment further comprises adjusting a dose of compound (1) to achieve induction of about 50% to about 100%, about 100% to about 150%, about 150% to about 200%, about 200% to about 250%, about 250% to about 300%, about 300% to about 350%, about 350% to about 400%, about 400% to about 450%, about 450% to about 500%, about 500% to about 550%, about 550% to about 600%, or greater than 600% of the ER stress gene. In some embodiments, the method of treatment further comprises adjusting a dose of compound (1) to achieve induction of about 50% to about 100%, about 100% to about 200%, about 200% to about 300%, about 300% to about 400%, about 400% to about 500%, about 500% to about 600%, or greater than 600% of the ER stress gene.

In one embodiment, the method of treatment comprises administering to a subject in need of such treatment: (i) a first therapeutic agent including a compound comprising compound (1) di-HCl in combination with (ii) a second therapeutic agent, wherein the first therapeutic agent and the second therapeutic agent are administered either simultaneously or sequentially; and further comprises assaying the expression of a proteasomal activity in a biological sample. In some embodiments the proteasomal activity may be chymotrysin-like, trypsin-like, and/or caspase-like activity. In some embodiments the biological sample may be tumor, peripheral blood mononuclear cells, or skin cells. The biological sample may be obtained before, during, or after drug administration. In some embodiments, the method of treatment further comprises adjusting the dose to achieve inhibition of about 20%, about 25%, about 30%, about 35%, about 40%, about 45%, about 50%, about 55%, about 60%, about 65%, about 70%, about 75%, about 80%, about 85%, about 90%, about 95%, or about 100% of the proteasomal activity. In some embodiments, the method of treatment further comprises adjusting the dose to achieve inhibition of at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of the proteasomal activity. In some embodiments, the method of treatment further comprises adjusting the dose to achieve inhibition of about 20% to about 30%, about 30% to about 40%, about 40% to about 50%, about 50% to about 60%, about 60% to about 70%, about 70% to about 80%, about 80% to about 90%, or greater than 90% of the proteasomal activity.

In an aspect, the present invention provides a method of treatment, which comprises administering to a subject in need of such treatment a combination of a first therapeutic agent including the following compound (**1**) and a second therapeutic agent, the method comprising: (i) administering to the subject the first therapeutic agent including compound (**1**): 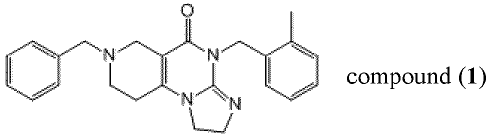 di-HCl;
(ii) waiting until a predetermined waiting time has elapsed after the time of administration of the first therapeutic agent to the subject; and / or until adverse events are resolved or resolving; and
(iii) administering the second therapeutic agent to the subject, wherein the predetermined waiting time is chosen so as to obtain a delayed therapeutic effect of the first therapeutic agent without an increased risk of possible combined toxic effects of the first and second therapeutic agents. In some embodiments of the method of treatment, the predetermined waiting time is determined based on the clearance rate of compound (**1**) di-HCl. In some embodiments of the method of treatment, the predetermined waiting time is determined by a quantitative assessment of renal function and parameters of renal. In some embodiments of the method of treatment, the predetermined waiting time is determined by an assays for the determination of renal function, wherein the assay is selected from the group consisting of serum level of compound (**1**) di-HCl; compound (**1**) di-HCl clearance rate; 24-hour urinary clearance of compound (**1**) di-HCl or a metabolite thereof.

In one embodiment of the method of treatment, the predetermined waiting time substantially equals to the time required for systemic clearance of compound (**1**) di-HClfrom the body of the subject. In one embodiment of the method of treatment, the predetermined waiting time substantially equals to the time required for renal clearance of compound (**1**) di-HCl from the body of the subject. In one embodiment of the method of treatment, the predetermined waiting time substantially equals to the time required for hepatic clearance of compound (**1**) di-HCl from the body of the subject. In one embodiment of the method of treatment, the predetermined waiting time substantially equals to the time required for total clearance of compound (**1**) di-HCl from the body of the subject. In one embodiment of the method of treatment, the predetermined waiting time is about 4 hours. In other embodimens the waiting time is 1 day. In some embodiments, the wait time is until Cmax of compound (**1**) has passed. In other embodiments, the waiting time is after most of the adverse events are resolved or are resolving. In one embodiment of the method of treatment, the predetermined waiting time is about 2 days. In one embodiment of the method of treatment, the predetermined waiting time is about 3 days. In one embodiment of the method of treatment, the predetermined waiting time is about 4 days. In one embodiment of the method of treatment, the predetermined waiting time is about 5 days. In one embodiment of the method of treatment, the predetermined waiting time is about 6 days. In one embodiment of the method of treatment, the predetermined waiting time is about 7 days. In one embodiment of the method of treatment, the predetermined waiting time is about 1-7 days. In one embodiment of the method of treatment, the predetermined waiting time is about 1-6 days. In one embodiment of the method of treatment, the predetermined waiting time is about 1-5 days. In one embodiment of the method of treatment, the predetermined waiting time is about 1-4 days. In one embodiment of the method of treatment, the predetermined waiting time is about 1-3 days. In one embodiment of the method of treatment, the predetermined waiting time is about 1 to 2 days. In some embodiments, the waiting time is up to 3 weeks. The preceeding are considered "therapeutic time priods."

When the order of administration is reveresed, timing for the administration of compound (**1**) can be after the Cmax of the first administered drug has passed. In some embodiments, administration of compound (**1**) can be after most or substantially all of the first administered drug has been eliminated from the body or the toxicity effects for the first administered drug are resolved or are resolving.

In some embodiments, the method of treatment further comprises monitoring level of compound (**1**) di-HCl or a metabolite thereof in the subject using pharmacokinetic profiling. In some such embodiments, monitoring level of compound (**1**) di-HCl or a metabolite thereof in the subject using pharmacokinetic profiling comprises constructing a pharmacokinetic profile of compound (**1**) di-HCl or a metabolite thereof for the subject using concentrations of compound (**1**) di-HCl or a metabolite thereof in at least two samples obtained from the subject at time points suitable to construct a pharmacokinetic profile. In some embodiments of the method, which include monitoring level of compound (**1**) di-HCl or a metabolite thereof in the subject using pharmacokinetic profiling, at least two samples are collected from the subject at point-of-care or point of use by sampling or self-sampling on point-of-care devices or point of use devices or on matrices suitable for storage of the at least two samples prior to quantitation in a laboratory. In some embodiments of the method of treatment, each of the point-of-care devices or point of use devices is capable of quantitating compound (**1**) di-HCl or a metabolite. In some embodiments of the method, which include monitoring level of compound (**1**) di-HCl or a metabolite thereof in the subject, one or more samples are collected from the subject at point-of-care or point of use by biopsy device for analysis at the point-of-care or point of use devices or for storage prior to analysis by a laboratory. In some embodiments of the method, a biopsy is taken after a time interval of 3-8 hours following administration of compound (**1**) di-HCl to the subject. In some embodiments of the method, a biopsy is taken after a time interval of 3-24 hours following administration of compound (**1**) di-HCl to the subject. In some embodiments of the method, a biopsy is taken after a time interval of 8-24 hours following administration of compound (**1**) di-HCl to the subject. In some embodiments of the method, a biopsy is taken after a time interval of 2 days following administration of compound (**1**) di-HCl to the subject. In some embodiments of the method, a biopsy is taken after a time interval of 3 days following administration of compound (**1**) di-HCl to the subject. In some embodiments of the method, a biopsy is taken after a time interval of 4 days following administration of compound (**1**) di-HCl to the subject. In some embodiments of the method, a biopsy is taken after a time interval of 1-7 days following administration of compound (**1**) di-HCl to the subject.

In some embodiments of the method of treatment, the pharmacokinetic profile includes pharmacokinetic parameters suitable for guiding dosing of compound (**1**) di-HCl for the subject being treated. In some embodiments of the method of treatment, maximum concentration of the first therapeutic agent in blood (whole blood, plasma, or serum) ("Cmax") of the subject following its administration to the subject ranges from about 1000 ng/dl to 1500 ng/dl for a therapeutic time period. In some embodiments, Cmax is less than 1500 ng/dl and greater than 85 ng/dl for a therapeutic time period. In some embodiments of the method of treatment, maximum concentration of the first therapeutic agent in blood (whole blood, plasma, or serum) ("Cmax") of the subject following its administration to the subject ranges from about 1000 ng/ml to 1500 ng/ml for a therapeutic time period. In some embodiments, Cmax is less than 1500 ng/ml and greater than 85 ng/ml for a therapeutic time period.

In some embodiments, maximum concentration of the first therapeutic agent in blood (whole blood, plasma, or serum) ("Cmax") of the subject following its administration to the subject is a Cmax of from about 1000 ng/dl to about 1500 ng/dl, from about 1010 ng/dl to about 1500 ng/dl, from about 1020 ng/dl to about 1500 ng/dl, from about 1030 ng/dl to about 1500 ng/dl, from about 1040 ng/dl to about 1500 ng/dl, from about 1050 ng/dl to about 1500 ng/dl, from about 1060 ng/dl to about 1500 ng/dl, from about 1070 ng/dl to about 1500 ng/dl, from about 1080 ng/dl to about 1500 ng/dl, from about 1090 ng/dl to about 1500 ng/dl, from about 1100 ng/dl to about 1500 ng/dl, from about 1110 ng/dl to about 1500 ng/dl, from about 1120 ng/dl to about 1500 ng/dl, from about 1130 ng/dl to about 1500 ng/dl, from about 1140 ng/dl to about 1500 ng/dl, from about 1150 ng/dl to about 1500 ng/dl, from about 1160 ng/dl to about 1500 ng/dl, from about 1170 ng/dl to about 1500 ng/dl, from about 1180 ng/dl to about 1500 ng/dl, from about 1190 ng/dl to about 1500 ng/dl, from about 1200 ng/dl to about 1500 ng/dl, from about 1210 ng/dl to about 1500 ng/dl, from about 1220 ng/dl to about 1500 ng/dl, from about 1230 ng/dl to about 1500 ng/dl, from about 1240 ng/dl to about 1500 ng/dl, from about 1250 ng/dl to about 1500 ng/dl, from about 1260 ng/dl to about 1500 ng/dl, from about 1270 ng/dl to about 1500 ng/dl, from about 1280 ng/dl to about 1500 ng/dl, from about 1290 ng/dl to about 1500 ng/dl, from about 1300 ng/dl to about 1500 ng/dl, from about 1310 ng/dl to about 1500 ng/dl, from about 1320 ng/dl to about 1500 ng/dl, from about 1330 ng/dl to about 1500 ng/dl, from about 1340 ng/dl to about 1500 ng/dl, from about 1350 ng/dl to about 1500 ng/dl, from about 1360 ng/dl to about 1500 ng/dl, from about 1370 ng/dl to about 1500 ng/dl, from about 1380 ng/dl to about 1500 ng/dl, from about 1390 ng/dl to about 1500 ng/dl, from about 1400 ng/dl to about 1500 ng/dl, from about 1410 ng/dl to about 1500 ng/dl, from about 1420 ng/dl to about 1500 ng/dl, from about 1430 ng/dl to about 1500 ng/dl, from about 1440 ng/dl to about 1500 ng/dl, from about 1450 ng/dl to about 1500 ng/dl, from about 1460 ng/dl to about 1500 ng/dl, from about 1470 ng/dl to about 1500 ng/dl, from about 1480 ng/dl to about 1500 ng/dl, or from about 1490 ng/dl to about 1500 ng/dl.

In some embodiments, maximum concentration of the first therapeutic agent in blood (whole blood, plasma, or serum) ("Cmax") of the subject following its administration to the subject is a Cmax of from about 1000 ng/ml to about 1500 ng/ml, from about 1010 ng/ml to about 1500 ng/ml, from about 1020 ng/ml to about 1500 ng/ml, from about 1030 ng/ml to about 1500 ng/ml, from about 1040 ng/ml to about 1500 ng/ml, from about 1050 ng/ml to about 1500 ng/ml, from about 1060 ng/ml to about 1500 ng/ml, from about 1070 ng/ml to about 1500 ng/ml, from about 1080 ng/ml to about 1500 ng/ml, from about 1090 ng/ml to about 1500 ng/ml, from about 1100 ng/ml to about 1500 ng/ml, from about 1110 ng/ml to about 1500 ng/ml, from about 1120 ng/ml to about 1500 ng/ml, from about 1130 ng/ml to about 1500 ng/ml, from about 1140 ng/ml to about 1500 ng/ml, from about 1150 ng/ml to about 1500 ng/ml, from about 1160 ng/ml to about 1500 ng/ml, from about 1170 ng/ml to about 1500 ng/ml, from about 1180 ng/ml to about 1500 ng/ml, from about 1190 ng/ml to about 1500 ng/ml, from about 1200 ng/ml to about 1500 ng/ml, from about 1210 ng/ml to about 1500 ng/ml, from about 1220 ng/ml to about 1500 ng/ml, from about 1230 ng/ml to about 1500 ng/ml, from about 1240 ng/ml to about 1500 ng/ml, from about 1250 ng/ml to about 1500 ng/ml, from about 1260 ng/ml to about 1500 ng/ml, from about 1270 ng/ml to about 1500 ng/ml, from about 1280 ng/ml to about 1500 ng/ml, from about 1290 ng/ml to about 1500 ng/ml, from about 1300 ng/ml to about 1500 ng/ml, from about 1310 ng/ml to about 1500 ng/ml, from about 1320 ng/ml to about 1500 ng/ml, from about 1330 ng/ml to about 1500 ng/ml, from about 1340 ng/ml to about 1500 ng/ml, from about 1350 ng/ml to about 1500 ng/ml, from about 1360 ng/ml to about 1500 ng/ml, from about 1370 ng/ml to about 1500 ng/ml, from about 1380 ng/ml to about 1500 ng/ml, from about 1390 ng/ml to about 1500 ng/ml, from about 1400 ng/ml to about 1500 ng/ml, from about 1410 ng/ml to about 1500 ng/ml, from about 1420 ng/ml to about 1500 ng/ml, from about 1430 ng/ml to about 1500 ng/ml, from about 1440 ng/ml to about 1500 ng/ml, from about 1450 ng/ml to about 1500 ng/ml, from about 1460 ng/ml to about 1500 ng/ml, from about 1470 ng/ml to about 1500 ng/ml, from about 1480 ng/ml to about 1500 ng/ml, or from about 1490 ng/ml to about 1500 ng/ml.

In some embodiments, maximum concentration of the first therapeutic agent in blood (whole blood, plasma, or serum) ("Cmax") of the subject following its administration to the subject is selected from 1000 ng/dl, about 1010 ng/dl, about 1020 ng/dl, about 1030 ng/dl, about 1040 ng/dl, about 1050 ng/dl, about 1060 ng/dl, about 1070 ng/dl, about 1080 ng/dl, about 1090 ng/dl, about 1100 ng/dl, about 1110 ng/dl, about 1120 ng/dl, about 1130 ng/dl, about 1140 ng/dl, about 1150 ng/dl, about 1160 ng/dl, about 1170 ng/dl, about 1180 ng/dl, about 1190 ng/dl, about 1200 ng/dl, about 1210 ng/dl, about 1220 ng/dl, about 1230 ng/dl, about 1240 ng/dl, about 1250 ng/dl, about 1260 ng/dl, about 1270 ng/dl, about 1280 ng/dl, about 1290 ng/dl, about 1300 ng/dl, about 1310 ng/dl, about 1320 ng/dl, about 1330 ng/dl, about 1340 ng/dl, about 1350 ng/dl, about 1360 ng/dl, about 1370 ng/dl, about 1380 ng/dl, about 1390 ng/dl, about 1400 ng/dl, about 1410 ng/dl, about 1420 ng/dl, about 1430 ng/dl, about 1440 ng/dl, about 1450 ng/dl, about 1460 ng/dl, about 1470 ng/dl, about 1480 ng/dl, and about 1490 ng/dl.

In some embodiments, maximum concentration of the first therapeutic agent in blood (whole blood, plasma, or serum) ("Cmax") of the subject following its administration to the subject is selected from 1000 ng/ml, about 1010 ng/ml, about 1020 ng/ml, about 1030 ng/ml, about 1040 ng/ml, about 1050 ng/ml, about 1060 ng/ml, about 1070 ng/ml, about 1080 ng/ml, about 1090 ng/ml, about 1100 ng/ml, about 1110 ng/ml, about 1120 ng/ml, about 1130 ng/ml, about 1140 ng/ml, about 1150 ng/ml, about 1160 ng/ml, about 1170 ng/ml, about 1180 ng/ml, about 1190 ng/ml, about 1200 ng/ml, about 1210 ng/ml, about 1220 ng/ml, about 1230 ng/ml, about 1240 ng/ml, about 1250 ng/ml, about 1260 ng/ml, about 1270 ng/ml, about 1280 ng/ml, about 1290 ng/ml, about 1300 ng/ml, about 1310 ng/ml, about 1320 ng/ml, about 1330 ng/ml, about 1340 ng/ml, about 1350 ng/ml, about 1360 ng/ml, about 1370 ng/ml, about 1380 ng/ml, about 1390 ng/ml, about 1400 ng/ml, about 1410 ng/ml, about 1420 ng/ml, about 1430 ng/ml, about 1440 ng/ml, about 1450 ng/ml, about 1460 ng/ml, about 1470 ng/ml, about 1480 ng/ml, and about 1490 ng/ml.

In some embodiments, maximum concentration of the first therapeutic agent in blood (whole blood, plasma, or serum) ("Cmax") of the subject following its administration to the subject is selected from about 85 ng/dl, about 95 ng/dl, about 105 ng/dl, about 115 ng/dl, about 125 ng/dl, about 135 ng/dl, about 145 ng/dl, about 155 ng/dl, about 165 ng/dl, about 175 ng/dl, about 185 ng/dl, about 195 ng/dl, about 205 ng/dl, about 215 ng/dl, about 225 ng/dl, about 235 ng/dl, about 245 ng/dl, about 255 ng/dl, about 265 ng/dl, about 275 ng/dl, about 285 ng/dl, about 295 ng/dl, about 305 ng/dl, about 315 ng/dl, about 325 ng/dl, about 335 ng/dl, about 345 ng/dl, about 355 ng/dl, about 365 ng/dl, about 375 ng/dl, about 385 ng/dl, about 395 ng/dl, about 405 ng/dl, about 415 ng/dl, about 425 ng/dl, about 435 ng/dl, about 445 ng/dl, about 455 ng/dl, about 465 ng/dl, about 475 ng/dl, about 485 ng/dl, about 495 ng/dl, about 505 ng/dl, about 515 ng/dl, about 525 ng/dl, about 535 ng/dl, about 545 ng/dl, about 555 ng/dl, about 565 ng/dl, about 575 ng/dl, about 585 ng/dl, about 595 ng/dl, about 605 ng/dl, about 615 ng/dl, about 625 ng/dl, about 635 ng/dl, about 645 ng/dl, about 655 ng/dl, about 665 ng/dl, about 675 ng/dl, about 685 ng/dl, about 695 ng/dl, about 705 ng/dl, about 715 ng/dl, about 725 ng/dl, about 735 ng/dl, about 745 ng/dl, about 755 ng/dl, about 765 ng/dl, about 775 ng/dl, about 785 ng/dl, about 795 ng/dl, about 805 ng/dl, about 815 ng/dl, about 825 ng/dl, about 835 ng/dl, about 845 ng/dl, about 855 ng/dl, about 865 ng/dl, about 875 ng/dl, about 885 ng/dl, about 895 ng/dl, about 905 ng/dl, about 915 ng/dl, about 925 ng/dl, about 935 ng/dl, about 945 ng/dl, about 955 ng/dl, about 965 ng/dl, about 975 ng/dl, about 985 ng/dl, about 995 ng/dl, about 1005 ng/dl, about 1015 ng/dl, about 1025 ng/dl, about 1035 ng/dl, about 1045 ng/dl, about 1055 ng/dl, about 1065 ng/dl, about 1075 ng/dl, about 1085 ng/dl, about 1095 ng/dl, about 1105 ng/dl, about 1115 ng/dl, about 1125 ng/dl, about 1135 ng/dl, about 1145 ng/dl, about 1155 ng/dl, about 1165 ng/dl, about 1175 ng/dl, about 1185 ng/dl, about 1195 ng/dl, about 1205 ng/dl, about 1215 ng/dl, about 1225 ng/dl, about 1235 ng/dl, about 1245 ng/dl, about 1255 ng/dl, about 1265 ng/dl, about 1275 ng/dl, about 1285 ng/dl, about 1295 ng/dl, about 1305 ng/dl, about 1315 ng/dl, about 1325 ng/dl, about 1335 ng/dl, about 1345 ng/dl, about 1355 ng/dl, about 1365 ng/dl, about 1375 ng/dl, about 1385 ng/dl, about 1395 ng/dl, about 1405 ng/dl, about 1415 ng/dl, about 1425 ng/dl, about 1435 ng/dl, about 1445 ng/dl, about 1455 ng/dl, about 1465 ng/dl, about 1475 ng/dl, about 1485 ng/dl, about 1495 ng/dl, and about 1500 ng/dl.

In some embodiments, maximum concentration of the first therapeutic agent in blood (whole blood, plasma, or serum) ("Cmax") of the subject following its administration to the subject is selected from about 85 ng/ml, about 95 ng/ml, about 105 ng/ml, about 115 ng/ml, about 125 ng/ml, about 135 ng/ml, about 145 ng/ml, about 155 ng/ml, about 165 ng/ml, about 175 ng/ml, about 185 ng/ml, about 195 ng/ml, about 205 ng/ml, about 215 ng/ml, about 225 ng/ml, about 235 ng/ml, about 245 ng/ml, about 255 ng/ml, about 265 ng/ml, about 275 ng/ml, about 285 ng/ml, about 295 ng/ml, about 305 ng/ml, about 315 ng/ml, about 325 ng/ml, about 335 ng/ml, about 345 ng/ml, about 355 ng/ml, about 365 ng/ml, about 375 ng/ml, about 385 ng/ml, about 395 ng/ml, about 405 ng/ml, about 415 ng/ml, about 425 ng/ml, about 435 ng/ml, about 445 ng/ml, about 455 ng/ml, about 465 ng/ml, about 475 ng/ml, about 485 ng/ml, about 495 ng/ml, about 505 ng/ml, about 515 ng/ml, about 525 ng/ml, about 535 ng/ml, about 545 ng/ml, about 555 ng/ml, about 565 ng/ml, about 575 ng/ml, about 585 ng/ml, about 595 ng/ml, about 605 ng/ml, about 615 ng/ml, about 625 ng/ml, about 635 ng/ml, about 645 ng/ml, about 655 ng/ml, about 665 ng/ml, about 675 ng/ml, about 685 ng/ml, about 695 ng/ml, about 705 ng/ml, about 715 ng/ml, about 725 ng/ml, about 735 ng/ml, about 745 ng/ml, about 755 ng/ml, about 765 ng/ml, about 775 ng/ml, about 785 ng/ml, about 795 ng/ml, about 805 ng/ml, about 815 ng/ml, about 825 ng/ml, about 835 ng/ml, about 845 ng/ml, about 855 ng/ml, about 865 ng/ml, about 875 ng/ml, about 885 ng/ml, about 895 ng/ml, about 905 ng/ml, about 915 ng/ml, about 925 ng/ml, about 935 ng/ml, about 945 ng/ml, about 955 ng/ml, about 965 ng/ml, about 975 ng/ml, about 985 ng/ml, about 995 ng/ml, about 1005 ng/ml, about 1015 ng/ml, about 1025 ng/ml, about 1035 ng/ml, about 1045 ng/ml, about 1055 ng/ml, about 1065 ng/ml, about 1075 ng/ml, about 1085 ng/ml, about 1095 ng/ml, about 1105 ng/ml, about 1115 ng/ml, about 1125 ng/ml, about 1135 ng/ml, about 1145 ng/ml, about 1155 ng/ml, about 1165 ng/ml, about 1175 ng/ml, about 1185 ng/ml, about 1195 ng/ml, about 1205 ng/ml, about 1215 ng/ml, about 1225 ng/ml, about 1235 ng/ml, about 1245 ng/ml, about 1255 ng/ml, about 1265 ng/ml, about 1275 ng/ml, about 1285 ng/ml, about 1295 ng/ml, about 1305 ng/ml, about 1315 ng/ml, about 1325 ng/ml, about 1335 ng/ml, about 1345 ng/ml, about 1355 ng/ml, about 1365 ng/ml, about 1375 ng/ml, about 1385 ng/ml, about 1395 ng/ml, about 1405 ng/ml, about 1415 ng/ml, about 1425 ng/ml, about 1435 ng/ml, about 1445 ng/ml, about 1455 ng/ml, about 1465 ng/ml, about 1475 ng/ml, about 1485 ng/ml, about 1495 ng/ml, and about 1500 ng/ml.

In some embodiments of the method of treatment, the maximum concentration of the first therapeutic agent in blood (whole blood, plasma, or serum) ("Cmax") of the subject following its administration to the subject ranges from about 85 ng/dl to 1500 ng/dl. In some embodiments, maximum concentration of the first therapeutic agent in blood (whole blood, plasma, or serum) ("Cmax") of the subject following its administration to the subject is selected from about 85 ng/dl to about 1500 ng/dl, from about 95 ng/dl to about 1500 ng/dl, from about 105 ng/dl to about 1500 ng/dl, from about 115 ng/dl to about 1500 ng/dl, from about 125 ng/dl to about 1500 ng/dl, from about 135 ng/dl to about 1500 ng/dl, from about 145 ng/dl to about 1500 ng/dl, from about 155 ng/dl to about 1500 ng/dl, from about 165 ng/dl to about 1500 ng/dl, from about 175 ng/dl to about 1500 ng/dl, from about 185 ng/dl to about 1500 ng/dl, from about 195 ng/dl to about 1500 ng/dl, from about 205 ng/dl to about 1500 ng/dl, from about 215 ng/dl to about 1500 ng/dl, from about 225 ng/dl to about 1500 ng/dl, from about 235 ng/dl to about 1500 ng/dl, from about 245 ng/dl to about 1500 ng/dl, from about 255 ng/dl to about 1500 ng/dl, from about 265 ng/dl to about 1500 ng/dl, from about 275 ng/dl to about 1500 ng/dl, from about 285 ng/dl to about 1500 ng/dl, from about 295 ng/dl to about 1500 ng/dl, from about 305 ng/dl to about 1500 ng/dl, from about 315 ng/dl to about 1500 ng/dl, from about 325 ng/dl to about 1500 ng/dl, from about 335 ng/dl to about 1500 ng/dl, from about 345 ng/dl to about 1500 ng/dl, from about 355 ng/dl to about 1500 ng/dl, from about 365 ng/dl to about 1500 ng/dl, from about 375 ng/dl to about 1500 ng/dl, from about 385 ng/dl to about 1500 ng/dl, from about 395 ng/dl to about 1500 ng/dl, from about 405 ng/dl to about 1500 ng/dl, from about 415 ng/dl to about 1500 ng/dl, from about 425 ng/dl to about 1500 ng/dl, from about 435 ng/dl to about 1500 ng/dl, from about 445 ng/dl to about 1500 ng/dl, from about 455 ng/dl to about 1500 ng/dl, from about 465 ng/dl to about 1500 ng/dl, from about 475 ng/dl to about 1500 ng/dl, from about 485 ng/dl to about 1500 ng/dl, from about 495 ng/dl to about 1500 ng/dl, from about 505 ng/dl to about 1500 ng/dl, from about 515 ng/dl to about 1500 ng/dl, from about 525 ng/dl to about 1500 ng/dl, from about 535 ng/dl to about 1500 ng/dl, from about 545 ng/dl to about 1500 ng/dl, from about 555 ng/dl to about 1500 ng/dl, from about 565 ng/dl to about 1500 ng/dl, from about 575 ng/dl to about 1500 ng/dl, from about 585 ng/dl to about 1500 ng/dl, from about 595 ng/dl to about 1500 ng/dl, from about 605 ng/dl to about 1500 ng/dl, from about 615 ng/dl to about 1500 ng/dl, from about 625 ng/dl to about 1500 ng/dl, from about 635 ng/dl to about 1500 ng/dl, from about 645 ng/dl to about 1500 ng/dl, from about 655 ng/dl to about 1500 ng/dl, from about 665 ng/dl to about 1500 ng/dl, from about 675 ng/dl to about 1500 ng/dl, from about 685 ng/dl to about 1500 ng/dl, from about 695 ng/dl to about 1500 ng/dl, from about 705 ng/dl to about 1500 ng/dl, from about 715 ng/dl to about 1500 ng/dl, from about 725 ng/dl to about 1500 ng/dl, from about 735 ng/dl to about 1500 ng/dl, from about 745 ng/dl to about 1500 ng/dl, from about 755 ng/dl to about 1500 ng/dl, from about 765 ng/dl to about 1500 ng/dl, from about 775 ng/dl to about 1500 ng/dl, from about 785 ng/dl to about 1500 ng/dl, from about 795 ng/dl to about 1500 ng/dl, from about 805 ng/dl to about 1500 ng/dl, from about 815 ng/dl to about 1500 ng/dl, from about 825 ng/dl to about 1500 ng/dl, from about 835 ng/dl to about 1500 ng/dl, from about 845 ng/dl to about 1500 ng/dl, from about 855 ng/dl to about 1500 ng/dl, from about 865 ng/dl to about 1500 ng/dl, from about 875 ng/dl to about 1500 ng/dl, from about 885 ng/dl to about 1500 ng/dl, from about 895 ng/dl to about 1500 ng/dl, from about 905 ng/dl to about 1500 ng/dl, from about 915 ng/dl to about 1500 ng/dl, from about 925 ng/dl to about 1500 ng/dl, from about 935 ng/dl to about 1500 ng/dl, from about 945 ng/dl to about 1500 ng/dl, from about 955 ng/dl to about 1500 ng/dl, from about 965 ng/dl to about 1500 ng/dl, from about 975 ng/dl to about 1500 ng/dl, from about 985 ng/dl to about 1500 ng/dl, from about 995 ng/dl to about 1500 ng/dl, from about 1005 ng/dl to about 1500 ng/dl, from about 1015 ng/dl to about 1500 ng/dl, from about 1025 ng/dl to about 1500 ng/dl, from about 1035 ng/dl to about 1500 ng/dl, from about 1045 ng/dl to about 1500 ng/dl, from about 1055 ng/dl to about 1500 ng/dl, from about 1065 ng/dl to about 1500 ng/dl, from about 1075 ng/dl to about 1500 ng/dl, from about 1085 ng/dl to about 1500 ng/dl, from about 1095 ng/dl to about 1500 ng/dl, from about 1105 ng/dl to about 1500 ng/dl, from about 1115 ng/dl to about 1500 ng/dl, from about 1125 ng/dl to about 1500 ng/dl, from about 1135 ng/dl to about 1500 ng/dl, from about 1145 ng/dl to about 1500 ng/dl, from about 1155 ng/dl to about 1500 ng/dl, from about 1165 ng/dl to about 1500 ng/dl, from about 1175 ng/dl to about 1500 ng/dl, from about 1185 ng/dl to about 1500 ng/dl, from about 1195 ng/dl to about 1500 ng/dl, from about 1205 ng/dl to about 1500 ng/dl, from about 1215 ng/dl to about 1500 ng/dl, from about 1225 ng/dl to about 1500 ng/dl, from about 1235 ng/dl to about 1500 ng/dl, from about 1245 ng/dl to about 1500 ng/dl, from about 1255 ng/dl to about 1500 ng/dl, from about 1265 ng/dl to about 1500 ng/dl, from about 1275 ng/dl to about 1500 ng/dl, from about 1285 ng/dl to about 1500 ng/dl, from about 1295 ng/dl to about 1500 ng/dl, from about 1305 ng/dl to about 1500 ng/dl, from about 1315 ng/dl to about 1500 ng/dl, from about 1325 ng/dl to about 1500 ng/dl, from about 1335 ng/dl to about 1500 ng/dl, from about 1345 ng/dl to about 1500 ng/dl, from about 1355 ng/dl to about 1500 ng/dl, from about 1365 ng/dl to about 1500 ng/dl, from about 1375 ng/dl to about 1500 ng/dl, from about 1385 ng/dl to about 1500 ng/dl, from about 1395 ng/dl to about 1500 ng/dl, from about 1405 ng/dl to about 1500 ng/dl, from about 1415 ng/dl to about 1500 ng/dl, from about 1425 ng/dl to about 1500 ng/dl, from about 1435 ng/dl to about 1500 ng/dl, from about 1445 ng/dl to about 1500 ng/dl, from about 1455 ng/dl to about 1500 ng/dl, from about 1465 ng/dl to about 1500 ng/dl, from about 1475 ng/dl to about 1500 ng/dl, from about 1485 ng/dl to about 1500 ng/dl, from about 1495 ng/dl to about 1500 ng/dl, and from about 1500 ng/dl to about 1500 ng/dl.

In some embodiments of the method of treatment, the maximum concentration of the first therapeutic agent in blood (whole blood, plasma, or serum) ("Cmax") of the subject following its administration to the subject ranges from about 85 ng/ml to 1500 ng/ml. In some embodiments, maximum concentration of the first therapeutic agent in blood (whole blood, plasma, or serum) ("Cmax") of the subject following its administration to the subject is selected from about 85 ng/ml to about 1500 ng/ml, from about 95 ng/ml to about 1500 ng/ml, from about 105 ng/ml to about 1500 ng/ml, from about 115 ng/ml to about 1500 ng/ml, from about 125 ng/ml to about 1500 ng/ml, from about 135 ng/ml to about 1500 ng/ml, from about 145 ng/ml to about 1500 ng/ml, from about 155 ng/ml to about 1500 ng/ml, from about 165 ng/ml to about 1500 ng/ml, from about 175 ng/ml to about 1500 ng/ml, from about 185 ng/ml to about 1500 ng/ml, from about 195 ng/ml to about 1500 ng/ml, from about 205 ng/ml to about 1500 ng/ml, from about 215 ng/ml to about 1500 ng/ml, from about 225 ng/ml to about 1500 ng/ml, from about 235 ng/ml to about 1500 ng/ml, from about 245 ng/ml to about 1500 ng/ml, from about 255 ng/ml to about 1500 ng/ml, from about 265 ng/ml to about 1500 ng/ml, from about 275 ng/ml to about 1500 ng/ml, from about 285 ng/ml to about 1500 ng/ml, from about 295 ng/ml to about 1500 ng/ml, from about 305 ng/ml to about 1500 ng/ml, from about 315 ng/ml to about 1500 ng/ml, from about 325 ng/ml to about 1500 ng/ml, from about 335 ng/ml to about 1500 ng/ml, from about 345 ng/ml to about 1500 ng/ml, from about 355 ng/ml to about 1500 ng/ml, from about 365 ng/ml to about 1500 ng/ml, from about 375 ng/ml to about 1500 ng/ml, from about 385 ng/ml to about 1500 ng/ml, from about 395 ng/ml to about 1500 ng/ml, from about 405 ng/ml to about 1500 ng/ml, from about 415 ng/ml to about 1500 ng/ml, from about 425 ng/ml to about 1500 ng/ml, from about 435 ng/ml to about 1500 ng/ml, from about 445 ng/ml to about 1500 ng/ml, from about 455 ng/ml to about 1500 ng/ml, from about 465 ng/ml to about 1500 ng/ml, from about 475 ng/ml to about 1500 ng/ml, from about 485 ng/ml to about 1500 ng/ml, from about 495 ng/ml to about 1500 ng/ml, from about 505 ng/ml to about 1500 ng/ml, from about 515 ng/ml to about 1500 ng/ml, from about 525 ng/ml to about 1500 ng/ml, from about 535 ng/ml to about 1500 ng/ml, from about 545 ng/ml to about 1500 ng/ml, from about 555 ng/ml to about 1500 ng/ml, from about 565 ng/ml to about 1500 ng/ml, from about 575 ng/ml to about 1500 ng/ml, from about 585 ng/ml to about 1500 ng/ml, from about 595 ng/ml to about 1500 ng/ml, from about 605 ng/ml to about 1500 ng/ml, from about 615 ng/ml to about 1500 ng/ml, from about 625 ng/ml to about 1500 ng/ml, from about 635 ng/ml to about 1500 ng/ml, from about 645 ng/ml to about 1500 ng/ml, from about 655 ng/ml to about 1500 ng/ml, from about 665 ng/ml to about 1500 ng/ml, from about 675 ng/ml to about 1500 ng/ml, from about 685 ng/ml to about 1500 ng/ml, from about 695 ng/ml to about 1500 ng/ml, from about 705 ng/ml to about 1500 ng/ml, from about 715 ng/ml to about 1500 ng/ml, from about 725 ng/ml to about 1500 ng/ml, from about 735 ng/ml to about 1500 ng/ml, from about 745 ng/ml to about 1500 ng/ml, from about 755 ng/ml to about 1500 ng/ml, from about 765 ng/ml to about 1500 ng/ml, from about 775 ng/ml to about 1500 ng/ml, from about 785 ng/ml to about 1500 ng/ml, from about 795 ng/ml to about 1500 ng/ml, from about 805 ng/ml to about 1500 ng/ml, from about 815 ng/ml to about 1500 ng/ml, from about 825 ng/ml to about 1500 ng/ml, from about 835 ng/ml to about 1500 ng/ml, from about 845 ng/ml to about 1500 ng/ml, from about 855 ng/ml to about 1500 ng/ml, from about 865 ng/ml to about 1500 ng/ml, from about 875 ng/ml to about 1500 ng/ml, from about 885 ng/ml to about 1500 ng/ml, from about 895 ng/ml to about 1500 ng/ml, from about 905 ng/ml to about 1500 ng/ml, from about 915 ng/ml to about 1500 ng/ml, from about 925 ng/ml to about 1500 ng/ml, from about 935 ng/ml to about 1500 ng/ml, from about 945 ng/ml to about 1500 ng/ml, from about 955 ng/ml to about 1500 ng/ml, from about 965 ng/ml to about 1500 ng/ml, from about 975 ng/ml to about 1500 ng/ml, from about 985 ng/ml to about 1500 ng/ml, from about 995 ng/ml to about 1500 ng/ml, from about 1005 ng/ml to about 1500 ng/ml, from about 1015 ng/ml to about 1500 ng/ml, from about 1025 ng/ml to about 1500 ng/ml, from about 1035 ng/ml to about 1500 ng/ml, from about 1045 ng/ml to about 1500 ng/ml, from about 1055 ng/ml to about 1500 ng/ml, from about 1065 ng/ml to about 1500 ng/ml, from about 1075 ng/ml to about 1500 ng/ml, from about 1085 ng/ml to about 1500 ng/ml, from about 1095 ng/ml to about 1500 ng/ml, from about 1105 ng/ml to about 1500 ng/ml, from about 1115 ng/ml to about 1500 ng/ml, from about 1125 ng/ml to about 1500 ng/ml, from about 1135 ng/ml to about 1500 ng/ml, from about 1145 ng/ml to about 1500 ng/ml, from about 1155 ng/ml to about 1500 ng/ml, from about 1165 ng/ml to about 1500 ng/ml, from about 1175 ng/ml to about 1500 ng/ml, from about 1185 ng/ml to about 1500 ng/ml, from about 1195 ng/ml to about 1500 ng/ml, from about 1205 ng/ml to about 1500 ng/ml, from about 1215 ng/ml to about 1500 ng/ml, from about 1225 ng/ml to about 1500 ng/ml, from about 1235 ng/ml to about 1500 ng/ml, from about 1245 ng/ml to about 1500 ng/ml, from about 1255 ng/ml to about 1500 ng/ml, from about 1265 ng/ml to about 1500 ng/ml, from about 1275 ng/ml to about 1500 ng/ml, from about 1285 ng/ml to about 1500 ng/ml, from about 1295 ng/ml to about 1500 ng/ml, from about 1305 ng/ml to about 1500 ng/ml, from about 1315 ng/ml to about 1500 ng/ml, from about 1325 ng/ml to about 1500 ng/ml, from about 1335 ng/ml to about 1500 ng/ml, from about 1345 ng/ml to about 1500 ng/ml, from about 1355 ng/ml to about 1500 ng/ml, from about 1365 ng/ml to about 1500 ng/ml, from about 1375 ng/ml to about 1500 ng/ml, from about 1385 ng/ml to about 1500 ng/ml, from about 1395 ng/ml to about 1500 ng/ml, from about 1405 ng/ml to about 1500 ng/ml, from about 1415 ng/ml to about 1500 ng/ml, from about 1425 ng/ml to about 1500 ng/ml, from about 1435 ng/ml to about 1500 ng/ml, from about 1445 ng/ml to about 1500 ng/ml, from about 1455 ng/ml to about 1500 ng/ml, from about 1465 ng/ml to about 1500 ng/ml, from about 1475 ng/ml to about 1500 ng/ml, from about 1485 ng/ml to about 1500 ng/ml, from about 1495 ng/ml to about 1500 ng/ml, and from about 1500 ng/ml to about 1500 ng/ml.

In some embodiments of the method the total drug exposure over time, measured as the area under the curve ("AUC") of a plot of the concentration of the drug in blood (whole blood, plasma, or serum) of the subject following administration of the drug against time after administration of the drug ranges from about 150 ng hr/ml to about 8000 ng hr/ml. In some embodiments, AUC is less than 8000 ng hr/ml and is greater than or equal to 150 ng hr/ml.

In some embodiments of the method the total drug exposure over time, measured as the area under the curve ("AUC") of a plot of the concentration of the drug in blood (whole blood, plasma, or serum) of the subject following administration against time is an AUC of from about 100 ng hr/ml to about 8000 ng hr/ml, from about 150 ng hr/ml to about 8000 ng hr/ml, from about 200 ng hr/ml to about 8000 ng hr/ml, from about 400 ng hr/ml to about 8000 ng hr/ml, from about 600 ng hr/ml to about 8000 ng hr/ml, from about 800 ng hr/ml to about 8000 ng hr/ml, from about 1000 ng hr/ml to about 8000 ng hr/ml, from about 1200 ng hr/ml to about 8000 ng hr/ml, from about 1400 ng hr/ml to about 8000 ng hr/ml, from about 1600 ng hr/ml to about 8000 ng hr/ml, from about 1800 ng hr/ml to about 8000 ng hr/ml, from about 2000 ng hr/ml to about 8000 ng hr/ml, from about 2200 ng hr/ml to about 8000 ng hr/ml, from about 2400 ng hr/ml to about 8000 ng hr/ml, from about 2600 ng hr/ml to about 8000 ng hr/ml, from about 2800 ng hr/ml to about 8000 ng hr/ml, from about 3000 ng hr/ml to about 8000 ng hr/ml, from about 3200 ng hr/ml to about 8000 ng hr/ml, from about 3400 ng hr/ml to about 8000 ng hr/ml, from about 3600 ng hr/ml to about 8000 ng hr/ml, from about 3800 ng hr/ml to about 8000 ng hr/ml, from about 4000 ng hr/ml to about 8000 ng hr/ml, from about 4200 ng hr/ml to about 8000 ng hr/ml, from about 4400 ng hr/ml to about 8000 ng hr/ml, from about 4600 ng hr/ml to about 8000 ng hr/ml, from about 4800 ng hr/ml to about 8000 ng hr/ml, from about 5000 ng hr/ml to about 8000 ng hr/ml, from about 5200 ng hr/ml to about 8000 ng hr/ml, from about 5400 ng hr/ml to about 8000 ng hr/ml, from about 5600 ng hr/ml to about 8000 ng hr/ml, from about 5800 ng hr/ml to about 8000 ng hr/ml, from about 6000 ng hr/ml to about 8000 ng hr/ml, from about 6200 ng hr/ml to about 8000 ng hr/ml, from about 6400 ng hr/ml to about 8000 ng hr/ml, from about 6600 ng hr/ml to about 8000 ng hr/ml, from about 6800 ng hr/ml to about 8000 ng hr/ml, from about 7000 ng hr/ml to about 8000 ng hr/ml, from about 7200 ng hr/ml to about 8000 ng hr/ml, from about 7400 ng hr/ml to about 8000 ng hr/ml, from about 7600 ng hr/ml to about 8000 ng hr/ml, or from about 7800 ng hr/ml to about 8000 ng hr/ml.

In some embodiments of the method the total drug exposure over time, measured as the area under the curve ("AUC") of a plot of the concentration of the drug in blood (whole blood, plasma, or serum) of the subject following administration against time is an AUC of from about 100 ng hr/ml to about 8000 ng hr/ml, from about 150 ng hr/ml to about 7800 ng hr/ml, from about 150 ng hr/ml to about 7600 ng hr/ml, from about 150 ng hr/ml to about 7400 ng hr/ml, from about 150 ng hr/ml to about 7200 ng hr/ml, from about 150 ng hr/ml to about 7000 ng hr/ml, from about 150 ng hr/ml to about 6800 ng hr/ml, from about 150 ng hr/ml to about 6600 ng hr/ml, from about 150 ng hr/ml to about 6400 ng hr/ml, from about 150 ng hr/ml to about 6200 ng hr/ml, from about 150 ng hr/ml to about 6000 ng hr/ml, from about 150 ng hr/ml to about 5800 ng hr/ml, from about 150 ng hr/ml to about 5600 ng hr/ml, from about 150 ng hr/ml to about 5400 ng hr/ml, from about 150 ng hr/ml to about 5200 ng hr/ml, from about 1500 ng hr/ml to about 5000 ng hr/ml, from about 1500 ng hr/ml to about 4800 ng hr/ml, from about 150 ng hr/ml to about 4600 ng hr/ml, from about 150 ng hr/ml to about 4400 ng hr/ml, from about 150 ng hr/ml to about 4200 ng hr/ml, from about 150 ng hr/ml to about 4000 ng hr/ml, from about 150 ng hr/ml to about 3800 ng hr/ml, from about 150 ng hr/ml to about 3600 ng hr/ml, from about 150 ng hr/ml to about 3400 ng hr/ml, from about 150 ng hr/ml to about 3200 ng hr/ml, from about 150 ng hr/ml to about 3000 ng hr/ml, from about 1500 ng hr/ml to about 2800 ng hr/ml, from about 150 ng hr/ml to about 2600 ng hr/ml, from about 150 ng hr/ml to about 2400 ng hr/ml, from about 150 ng hr/ml to about 2200 ng hr/ml, from about 150 ng hr/ml to about 2000 ng hr/ml, from about 150 ng hr/ml to about 1800 ng hr/ml, from about 150 ng hr/ml to about 1600 ng hr/ml, from about 150 ng hr/ml to about 1400 ng hr/ml, from about 150 ng hr/ml to about 1200 ng hr/ml, from about 150 ng hr/ml to about 1000 ng hr/ml, from about 150 ng hr/ml to about 800 ng hr/ml, from about 150 ng hr/ml to about 600 ng hr/ml, from about 150 ng hr/ml to about 400 ng hr/ml, from about 150 ng hr/ml to about 200 ng hr/ml, or from about 100 ng hr/ml to about 200 ng hr/ml.

In some embodiments of the method the total drug exposure over time, measured as the area under the curve ("AUC") of a plot of the concentration of the drug in blood (whole blood, plasma, or serum) of the subject following administration against time is selected from about 100 ng hr/ml, about 150 ng hr/ml, about 200 ng hr/ml, about 400 ng hr/ml, about 600 ng hr/ml, about 800 ng hr/ml, about 1000 ng hr/ml, about 1200 ng hr/ml, about 1400 ng hr/ml, about 1600 ng hr/ml, about 1800 ng hr/ml, about 2000 ng hr/ml, about 2200 ng hr/ml, about 2400 ng hr/ml, about 2600 ng hr/ml, about 2800, about 3000 ng hr/ml, about 3200 ng hr/ml, about 3400 ng hr/ml, about 3600 ng hr/ml, about 3800 ng hr/ml, about 4000 ng hr/ml, about 4200 ng hr/ml, about 4400 ng hr/ml, about 46000 ng hr/ml, about 4800 ng hr/ml, about 5000 ng hr/ml, about 5200 ng hr/ml, about 5400 ng hr/ml, about 5600 ng hr/ml, about 5800 ng hr/ml, about 6000 ng hr/ml, about 6200 ng hr/ml, about 6400 ng hr/ml, about 6600 ng hr/ml, about 6800 ng hr/ml, about 7000 ng hr/ml, about 7200 ng hr/ml, about 7400 ng hr/ml, about 7600 ng hr/ml, about 7800 ng hr/ml, and about 8000 ng hr/ml.

In another aspect, the present invention provides a method of treatment, or use of the composition to treat a disease defined in the claims, which comprises administering to a subject in need of such treatment a combination of a first therapeutic agent and a second therapeutic agent, the method comprising:
(i) administering to the subject the first therapeutic agent including compound **(1):** 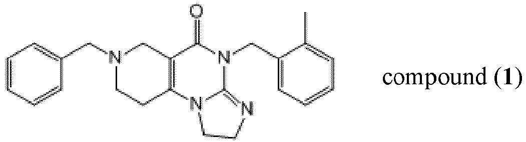 di-HCl;
(ii) monitoring level of compound **(1)** or a pharmaceutically acceptable salt thereof or a metabolite thereof in the subject using pharmacokinetic profiling; and
(iii) administering the second therapeutic agent conditional on the level of the first therapeutic agent in the subject. In some embodiments of the method, the monitoring step includes constructing a pharmacokinetic profile of compound **(1)** di-HCl or a metabolite thereof for the subject using concentrations of compound **(1)** di-HCl thereof or a metabolite thereof in at least two samples obtained from the subject at time points suitable to construct a pharmacokinetic profile. In some embodiments of the method, the at least two samples are collected at point-of-care or point of use by sampling or self-sampling on point-of-care devices or point of use devices or on matrices suitable for storage of the at least two samples prior to quantitation of compound **(1)** di-HCl or a metabolite by a laboratory. In some embodiments of the method, each point-of-care devices or point of use devices is capable of quantitating compound **(1)** di-HCl or a metabolite. In some embodiments of the method, the pharmacokinetic profile includes pharmacokinetic parameters suitable for guiding dosing of compound **(1)** di-HCl for the subject. In some embodiments of the method, the at least two samples include from 2-12 samples. In some embodiments of the method, the at least two samples are collected over a time period of up to 8 hours, up to 24 hours, up to 48 hours, or up to 72 hours. In some embodiments of the method, the pharmacokinetic parameters include at least one parameter selected from the group consisting of AUC, AUCinf, Tmax, Cmax, time above threshold, steady state concentration, absorption rate, clearance rate, distribution rate, terminal T-1/2 or parameters drawn from noncompartmental pharmacokinetic (PK) or compartmental PK analysis, including physiological model- based compartmental PK analysis. In some embodiments of the method, the method of treatment further comprises generating a report including the pharmacokinetic profile of the subject. In some embodiments of the method, the report includes a recommendation regarding dosing based on the pharmacokinetic profile of the subject. In some embodiments of the method, a reduction in dosage of compound **(1)** di-HCl is indicated to reduce risk of toxicity based on one or more pharmacokinetic parameters. In some embodiments of the method, the reduction in dosage of compound **(1)** di-HCl is indicated based on time above threshold, wherein the threshold is the drug concentration above which toxicity occurs, or one or more of AUC, AUCinf, mean residence time (MRT), exponentials defining the pharmacokinetic profile, volume of distribution at steady state (Vss), volume of distribution during the terminal phase (Vz) or combination of a group of pharmacokinetic variable to adequately describe the pharmacokinetic profile. In some embodiments of the method, a dose adjustment of compound **(1)** di-HCl is indicated to increase efficacy based on one or more pharmacokinetic parameters. In some embodiments of the method, an increase in dosage of compound **(1)** di-HCl is indicated based on one or more of AUC, AUCinf, MRT, exponentials defining the pharmacokinetic profile, steady state volume (Vss) of distribution, volume of distribution during the terminal phase (Vz) or combination of a group of pharmacokinetic variables to adequately describe the pharmacokinetic profile. In some embodiments of the method, the dose of compound **(1)** di-HCl is adjusted to within 5% to 25% of a desired target value. In some embodiments of the method, each of the at least two samples is applied to the point-of-care device or the point of use device for determining the concentration of the compound **(1)** di-HCl or a metabolite thereof, wherein the point-of-care device or the point of use device comprises a lateral flow strip having a construction and composition such that an application of one or more of the at least two samples to the lateral flow strip causes a fraction of the drug in the sample to bind to with a component of the lateral flow strip such that a detectable signal proportional to the concentration of the drug in the applied sample is produced. In some embodiments of the method, the at least two samples are applied to matrices suitable for storage of the at least two samples prior to quantitation by a laboratory. In some embodiments of the method, the at least two samples are stored as dried blood spots. In some embodiments of the method, drug concentrations are measured by ELISA, LC MS MS, LC UV or LCMS. In some embodiments of the method, the pharmacokinetic parameters include at least one of steady state concentration, absorption, and terminal T1/2. In some embodiments of the method, at least one of the at least two samples is whole blood.

### V. TNF-RELATED APOPTOSIS-INDUCING LIGAND ("TRAIL")

TRAIL protein can be assayed in a test sample obtained from a subject to detect TRAIL expression induced by compound **(1)** or a pharmaceutically acceptable salt thereof. Immunoassay methods can be used to assay TRAIL in a sample, including enzyme-linked immunosorbent assay (ELISA), enzyme-linked immunofiltration assay (ELIFA), flow cytometry, immunoblot, immunoprecipitation, immunohistochemistry, immunocytochemistry, luminescent immunoassay (LIA), fluorescent immunoassay (FIA), and radioimmunoassay. Assay methods may be used to obtain qualitative and/or quantitative results. Specific details of suitable assay methods for both qualitative and quantitative assay of a sample are described in standard references, illustratively including E. Harlow and D. Lane, Antibodies: A Laboratory Manual, Cold Spring Harbor Laboratory Press, 1988; F. Breitling and S. Diibel, Recombinant Antibodies, John Wiley & Sons, New York, 1999; H. Zola, Monoclonal Antibodies: Preparation and Use of Monoclonal Antibodies and Engineered Antibody Derivatives, Basics: From Background to Bench, BIOS Scientific Publishers, 2000; B. K. C. Lo, Antibody Engineering: Methods and Protocols, Methods in Molecular Biology, Humana Press, 2003; F. M. Ausubel et al., Eds., Short Protocols in Molecular Biology, Current Protocols, Wiley, 2002; S. Klussman, Ed., The Aptamer Handbook: Functional Oligonucleotides and Their Applications, Wiley, 2006; Ormerod, M. G., Flow Cytometry: a practical approach, Oxford University Press, 2000; Givan, A. L., Flow Cytometry: first principles, Wiley, New York, 2001; Gorczyca, W., Flow Cytometry in Neoplastic Hematology: morphologic-immunophenotypic correlation, Taylor & Francis, 2006; Crowther, J. R., The ELISA Guidebook (Methods in Molecular Biology), Humana Press, 2000; Wild, D., The Immunoassay Handbook, 3rd Edition, Elsevier Science, 2005.and J. Sambrook and D. W. Russell, Molecular Cloning: A Laboratory Manual, Cold Spring Harbor Laboratory Press, 3rd Ed., 2001.

Examples of protocols for assaying and analyzing a sample for TRAIL for the purpose of detection of an effect of a pharmaceutical composition of the present invention are described in U.S. Patent Application No. 2012/0276088 to Wafik S. El-deiry et al.

In some embodiments of the present invention, assays for TRAIL are used to monitor a subject. Thus, for example, a test sample is obtained from the subject before treatment with a pharmaceutical composition of the present invention and at one or more times during and/or following treatment in order to assess effectiveness of the treatment. In a further example, a test sample is obtained from the subject at various times in order to assess the course or progress of disease or healing. In one embodiment, death receptors can also be analyized from circulating tumor cells to see if the administration of compound (1) di-HCl increases the amount or type of death receptors.

Cancers treated using methods and compositions described herein are characterized by abnormal cell proliferation including pre-neoplastic hyperproliferation, cancer in-situ, neoplasms and metastasis. Methods and compositions of the present invention can be used for prophylaxis as well as amelioration of signs and/or symptoms of cancer. The terms "treating" and "treatment" used to refer to treatment of a cancer in a subject include: preventing, inhibiting or ameliorating the cancer in the subject, such as slowing progression of the cancer and/or reducing or ameliorating a sign or symptom of the cancer. Examples of cancers treated using the compound for use of the present invention are recited in the claims.

### VI. MULTIMODAL THERAPEUTIC METHODS

In one aspect, the present invention is directed to multimodal therapeutic methods of the diseases recited in the claims in which administration of compound **(1)** di-HCl to a subject in need of such treatment is supplemented by administration of other therapeutic modalities. In one embodiment, the multimodal therapeutic method of the present invention comprises administering to a subject a pharmaceutical composition comprising the compound **(1)** di-HCl in conjunction with radiation therapy or after radiation is determined to not have been efficacious. In one embodiment, the multimodal therapeutic method of the present invention comprises administering to a subject a pharmaceutical composition comprising compound **(1)** di-HCl in conjunction with radiation therapy, wherein the pharmaceutical composition comprising compound **(1)** di-HCl and the radiation therapy are administered concurrently or sequentially in any order. In one embodiment, the multimodal therapeutic method comprises administering to a subject a pharmaceutical composition comprising compound **(1)** di-HCl in conjunction with radiation therapy in a sequential arrangement. In one embodiment, the multimodal therapeutic method comprises administering to a subject in need of such treatment a pharmaceutical composition comprising compound **(1)** di-HCl concurrently with radiation therapy. In one embodiment, the multimodal therapeutic method of the present invention is used for the treatment of cancer. In one embodiment, the multimodal therapeutic method includes administering to a cancer subject in need of such treatment a pharmaceutical composition comprising compound **(1)** di-HCl and irradiating cancer cells with a radiation beam. In one embodiment, the multimodal therapeutic method uses the technique of conformal radiotherapy (CRT) to deliver a dose volume histogram (DVH) prescribed to a cancer subject. In one embodiment, the multimodal therapeutic method uses the technique of intensity modulated radiation therapy (IMRT) to deliver radiation to cancer cells. In one embodiment, the multimodal therapeutic method uses a techniques compensates for motion of tumors in the subject during treatment (e.g., where doses of radiation must be administered to a thoracic tumor which moves as the patient breathes). In one embodiment, the multimodal therapeutic method use Four Dimensional Computed Tomography (4D CT) scanning techniques to adjust the delivered radiation field to compensate for tumor motion over the breathing cycle.

Any suitable type of radiation, including gamma radiation which is given fractionated, IMRT (intensity modulated radiation therapy), gamma knife, proton therapy and brachytherapy can be used with the multimodal therapeutic method of the present invention. Radiation therapy and compound **(1)** di-HCl can be used to treat brain tumors such as glioblastoma or disease that has metastasized to the brain from lung cancer. The multimodal therapeutic method of the present invention can be used to treat the lymphomas recited in the claims. The gamma knife is used frequently to treat brain metastases. In one embodiment, the multimodal therapeutic method of the present invention includes use of proton therapy to treat cancer, including brain tumors, prostate cancer and any tumor proximate vital organs where it is very important to minimize toxicity to nearby normal tissue.

In one embodiment, the multimodal therapeutic method of the present invention eliminates minimal residual disease without adding to any toxicity resulting from treatment compound **(1)** di-HCl. In one embodiment, the multimodal therapeutic method of the present invention improves prognosis and/or reduces adverse side-effects associated with a disease state or condition in a subject undergoing treatment.

### VII. SYNTHESIS OF A SALT OF COMPOUND (1) AND RELATED ANALOGS

The compound represented by the above compound **(1)** can be prepared by the synthetic process illustrated in Scheme 1 below.

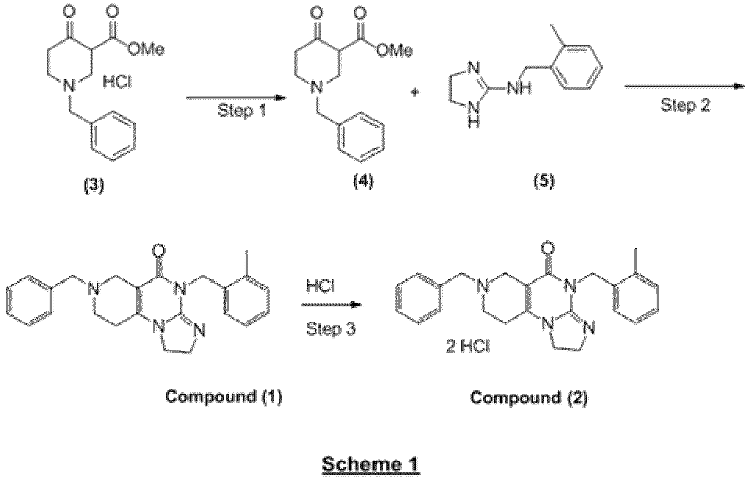

The process for making dihydrochloride salt of compound **(1)** commences with the intermediary compound of (3), also known as N-Benzyl-3-carbomethoxy-4-piperidone hydrochloride, which is commercially available. The synthetic process includes neutralizing the intermediary compound of (3) with a base (Step 1) to produce the compound of (4), a free base. The synthetic process includes neutralizing the intermediary compound of (3) with an inorganic base to produce the compound of (4). The synthetic process includes neutralizing the intermediary compound of (3) with an organic base to produce the compound of (4). The intermediary compound of (3) is neutralized in the presence of an alcohol. In one such embodiment, the intermediary compound of (3) is neutralized in the presence of n-butanol. The intermediary compound of (3) is neutralized in the presence of at least one organic solvent. In one such embodiment, the intermediary compound of (3) is neutralized in the presence of n-butanol and/or ethyl acetate. The intermediary compound of (3) is neutralized in the presence of a base and at least one organic solvent. In one such embodiment, the intermediary compound of (3) is neutralized in the presence of NaHCO3 and n-butanol. The intermediary compound of (3) is neutralized in the presence of n-butanol and triethyl amine (Et3N).

The synthetic process includes reacting the compound of (4) with the compound of (5) (Step 2) to produce intermediary compound of **(1).** The reaction in Step 2 includes heating the compound of (4) with the compound of (5). The reaction in Step 2 includes refluxing heating the compound of (4) and the compound of (5) in the presence of a solvent. The reaction in Step 2 includes use of Dean-stark trap to remove water and/or methanol (MeOH) formed in the reaction.

The synthetic process includes forming a dihydrochloride salt of the compound of **(1)** (Step 3). The reaction in Step 3 includes treating compound of **(1)** with HCl in dioxane. The reaction in Step 3 includes treating compound (3) with 4N HCl in dioxane.

The synthetic process optionally includes recrystallization of the di-salt of compound **(1).**

In one preferred embodiment, the synthetic process for the preparation of the dihydrochloride salt of compound **(1)** is as illustrated in the following Scheme 2.

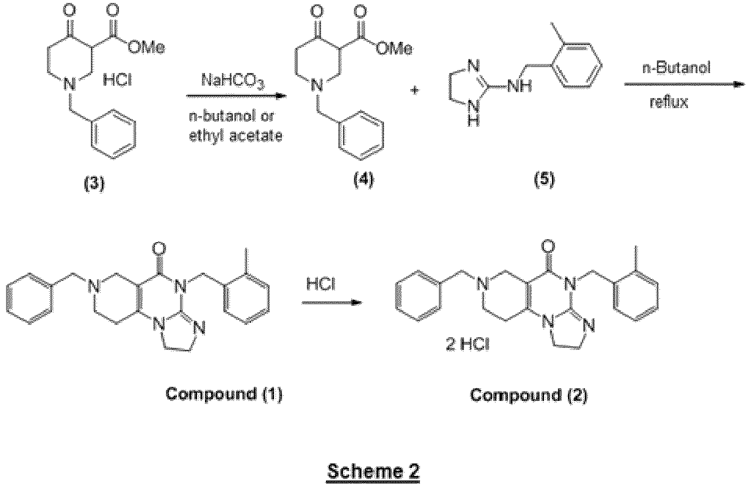

### VIII. DERIVATIVES AND ANALOGS OF AND SALTS OF COMPOUND (1) AND RELATED COMPOUNDS

Described herein are analogs and related salts of compound **(1)** and processes of making the same. Persons skilled in the art will understand that the same general principles and concepts described above in conjunction with compound **(1)** and salts thereof, including principles and concepts related to methods and pharmaceutical compositions, apply with equal force to derivatives and analogs of and salts of compound **(1)** and salts thereof.

Described herein, the compounds related to compound **(1)** have the structure of compound (10): 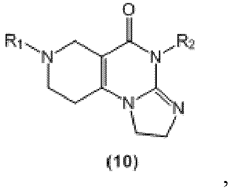 wherein R₁ and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. The compounds related to compound **(1)** have the structure of compound (10), wherein R₁ and R₂ may be independently selected from the group consisting of H, C₁₋₄alkyl, C₁₋₄alkylphenyl, C₁₋₄alkylphenylketone, C₁₋₄benzylpiperazine, and C₁₋₄alkylthienyl wherein C₁₋₄alkyl, C₁₋₄alkylphenyl, C₁₋₄alkylphenylketone, and C₁₋₄benzyl-piperazine are optionally substituted with C₁₋₄alkyl, hydroxyl, or halo. The compounds related to compound (1) have the structure of compound (10), wherein R₁ and R₂ may be independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph. When R₁ represents CH₂Ph, R₂ may not represent CH₂-((2-CH₃)-Ph.

As illustrated in Schemes 3 and 4, compound **(10)** can be synthesized starting either with methyl 1-R₁-4-oxo-3-piperidinecarboxylate **(6)** or by reacting compound **(12)** with compound **(6).**

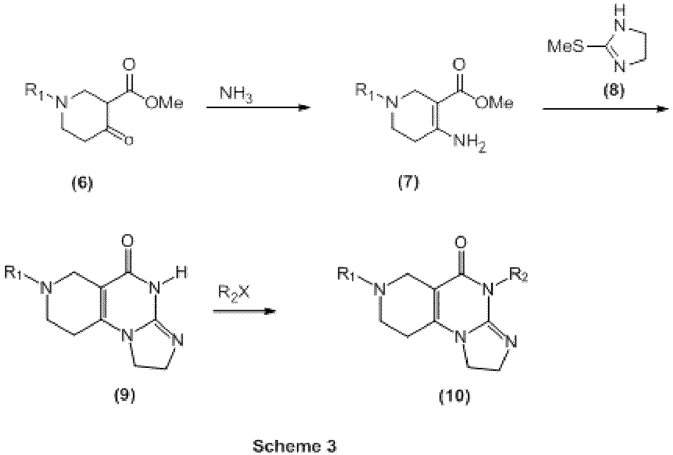

Scheme 3 illustrates the synthesis of compound **(10)** starting from compound **(6).** In one embodiment, as illustrated in Scheme 3, compound **(6)** was converted into 4-amino-3-pyridinecarboxylic acid ester methyl ester (7) (or methyl 4-amino-1-R₁-1,2,5,6-tetrahydro-3-pyridinecarboxylate) by a reaction with ammonia. In one embodiment, compound (7) (or 4-amino-3-pyridinecarboxylic acid ester methyl ester **(7))** was treated with 2-(Methylsulfanyl)-4,5-dihydro-1H-imidazole **(8)** to make compound **(9),** which when alkylated R₂X, wherein R₂ is as defined above and X is a halogen or an equivalent leaving group, produced compound **(10)** with different values for the R₂ substituent.

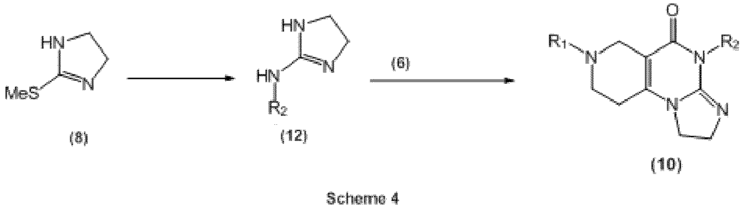

Scheme 4 illustrates the synthesis of compound **(10)** starting from compound **(6)** and compound **(12).** In one embodiment, as illustrated in Scheme 4, compound **(12)** is prepared from compound **(8).** In one embodiment, compound **(12)** was treated with compound **(6)** to produce compound **(10)** with different values for the R₂ substituent.

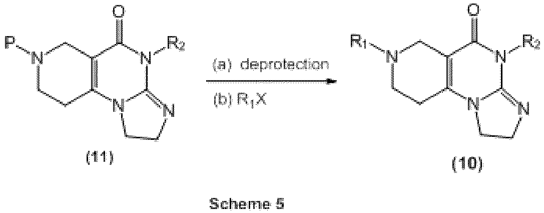

Scheme 5 illustrates the synthesis of compound **(10)** starting from compound **(11).** In one embodiment, as illustrated in Scheme 5, compound **(11),** having a nitrogen protecting group (P) at the N atom at ring position 7, was first deprotected and then akylated with R₁X, wherein R₁ is as defined above and X is a halogen or an equivalent leaving group, to produce compound **(10)** with different values for the R₁ substituent. In some embodiments compound (10) can be prepared as a salt, for example a 2TFA salt or 2HCl salt. In some embodiments, compound (10) can be prepared as a 2HCl salt by following a scheme analagous to Scheme 2 described above.

### EXAMPLES OF COMPOUND (10)

| **No.** | **ONC Number** | **R₁** | **R₂** |
|---|---|---|---|
| **1** | ONC201 | CH₂Ph | CH₂-((2-CH₃)-Ph) |
| **13 (reference)** | | CH₂Ph | CH₃ |
| **14 (reference)** | ONC902 | CH₂Ph | CH₂-((2-Cl)-Ph) |
| **15 (reference)** | ONC903 | CH₂Ph | CH₂-(2-thienyl) |
| **16 (reference)** | ONC904 | CH₂Ph | CH₂CH₂Ph |
| **17 (reference)** | ONC905 | CH₂Ph | CH₂CH₂(4-N-benzyl-piperazine) |
| **18 (reference)** | ONC906 | CH₂Ph | CH₂-(2,4-di F-Ph) |
| **19 (reference)** | ONC907 | H | CH₂-((2-CH₃)-Ph) |
| **20 (reference)** | ONC908 | CH₃ | CH₂-((2-CH₃)-Ph) |
| **21 (reference)** | ONC909 | CH₂CH₂Ph | CH₂-((2-CH₃)-Ph) |
| **22 (reference)** | | CH₂CH₂-(4-N-benzyl-piperizine) | CH₂-((2-CH₃)-Ph) |
| **23 (reference)** | | CH₂CHOHPh | CH₂-((2-CH₃)-Ph) |
| **24 (reference)** | | (CH₂)₃CO-4F-Ph | CH₂-((2-CH₃)-Ph) |
| **32 (reference)** | ONC910 (PV 03171) | CH₂CH₂NHCOOC(CH₃)₃ | CH₂-((2-CH₃)-Ph) |
| **33 (reference)** | ONC911 (PV 03172) | CH₂CH₂CH₂NH₂ | CH₂-((2-CH₃)-Ph) |

Not encompassed by the literal wording of the claims, are analogs having the structure of compound **(25):** 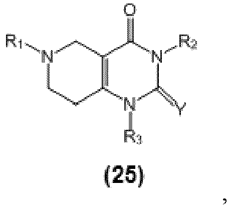 wherein Y represents NR₄ or O, and wherein R₁, R₂, R₃, and R₄ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁, R₂, R₃, and R₄ may be optionally substituted. Some or all hydrogens in R₁, R₂, R₃, and R₄ may be substituted by deuterium. The analogs have the structure of compound **(25),** wherein R₁, R₂, R₃, and R₄ may be independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs have the structure of compound **(25),** wherein R₁, R₂, R₃, and R₄ may be independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

Not encompassed by the literal wording of the claims, are analogs having the structure of compound **(26):** 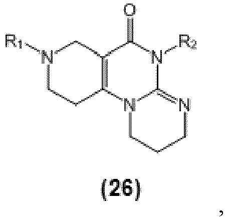 wherein R₁ and R₂, independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ and R₂ may beoptionally substituted. Some or all hydrogens in R₁ and R₂ may be substituted by deuterium. The analogs may have the structure of compound **(26),** wherein R₁ and R₂ are independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs may have the structure of compound **(26),** wherein R₁ and R₂ are independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-(2,3,4-tri F-Ph), CH₂-(2,3,4,5-quadra F-Ph), CH₂-(penta F-Ph) CH₂-((2-CH₃)-Ph), CH₂CHOHPh, and (CH₂)₃CO-4F-Ph. The analogs may have the structure of compound **(26),** wherein R₁ and R₂ are independently selected from the group consisting of C₆H₄(CH₃), C₆H₃(CH₃)₂, C₆H₂(CH₃)₃, C₆H(CH₃)₄, C₆(CH₃)₅, CH₂-C₆H₄(CH₃), CH₂-C₆H₃(CH₃)₂, CH₂-C₆H₂(CH₃)₃, CH₂-C₆H(CH₃)₄, CH₂-C₆(CH₃)₅. The analogs may have the structure of compound **(26),** wherein R₁ and R₂ are independently selected from the group consisting of C₆H₄(OH), C₆H₃(OH)₂, C₆H₂(OH)₃, C₆H(OH)₄, C₆(OH)₅, CH₂-C₆H₄(OH), CH₂-C₆H₃(OH)₂, CH₂-C₆H₂(OH)₃, CH₂-C₆H(OH)₄, CH₂-C₆(OH)₅.

Not encompassed by the literal wording of the claims, are analogs having the structure of compound **(27):** 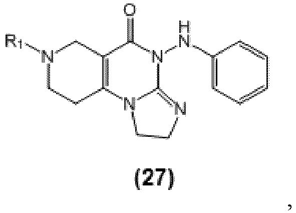 wherein R₁ represents hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ may be optionally substituted Some or all hydrogens in R₁ may be substituted by deuterium. The analogs may be the structure of compound **(27),** wherein R₁ is selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzyl-piperazine are optionally substituted with C₁₋₄alkyl, hydroxyl, or halo. The analogs may have the structure of compound **(27),** wherein R₁ is selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

Not encompassed by the literal wording of the claims, are analogs having the structure of compound **(28):** 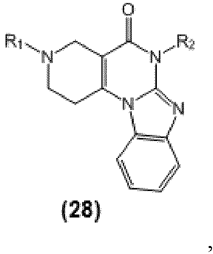 wherein R₁, and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ and R₂ may be optionally substituted Some or all hydrogens in R₁ and R₂ may be substituted by deuterium. The analogs may have the structure of compound **(28),** wherein R₁ and R₂ are independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs may have the structure of compound **(28),** wherein R₁ and R₂ are independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

Not encompassed by the literal wording of the claims are analogs having the structure of compound (29): 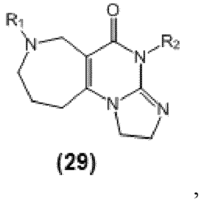 wherein R₁, and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ and R₂ may be optionally substituted Some or all hydrogens in R₁ and R₂ may be substituted by deuterium. The analogs may have the structure of compound **(29),** wherein R₁ and R₂ are independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs may have the structure of compound **(29),** wherein R₁ and R₂ are independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

Not encompassed by the literal wording of the claims are analogs having the structure of compound **(30):** 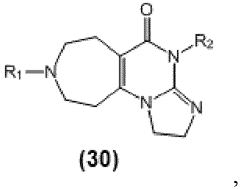 wherein R₁, and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ and R₂ are optionally substituted Some or all hydrogens in R₁ and R₂ may be substituted by deuterium. The analogs may have the structure of compound **(30),** wherein R₁ and R₂ are independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs have the structure of compound **(30),** wherein R₁ and R₂ are independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

Not encompassed by the literal wording of the claims are analogs having the structure of compound **(31):** 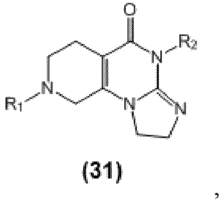 wherein R₁, and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals. R₁ and R₂ may be optionally substituted Some or all hydrogens in R₁ and R₂ may be substituted by deuterium. The analogs have the structure of compound **(31),** wherein R₁ and R₂ may be independently selected from the group consisting of H, C₁₋₄alkyl, C₁₋₄alkylphenyl, C₁₋₄alkylphenylketone, C₁₋₄benzyl-piperazine, and C₁₋₄alkylthienyl wherein C₁₋₄alkyl, C₁₋₄alkylphenyl, C₁₋₄alkylphenylketone, and C₁₋₄benzylpiperazine are optionally substituted with C₁₋₄alkyl, hydroxyl, or halo. The analogs have the structure of compound (31), wherein R₁ and R₂ may be independently selected from the group consisting of H, CH₃, CH₂Ph, CH₂-((2-Cl)-Ph), CH₂-(2-thienyl), CH₂CH₂Ph, CH₂CH₂(4-N-benzyl-piperazine), CH₂-(2,4-di F-Ph), CH₂-((2-CH₃)-Ph), CH₂CHOHPh, and (CH₂)₃CO-4F-Ph.

### IX. EXAMPLES

It should be understood that the description and specific examples provided below are intended for purposes of illustration only and are not intended to limit the scope of the present disclosure. Examples 1 to 2 illustrate synthesis of dihydrochloride salt of compound **(1)** starting from compound **(1).** In these examples and throughout the application the dihydrochloride salt of compound **(1)** is referred to as compound **(2).** The following examples are intended to illustrate the embodiments disclosed and are not to be construed as being limitations thereto. Additional compounds, other than those described below, may be prepared using the following reaction schemes described above or appropriate variations or modifications thereof.

### Example 1. Synthesis of 2-Chlorobenzylamino-2-imidazoline hydriodide

To a stirred solution of 2-methylthio-2-imidazoline hydriodide (244 mg, 1.00 mMol) in dry dioxane (2.0 mL) was added 2- chlorobenzylamine (141 mg, 1.0 mMol). The reaction mixture was stirred for 90 min at 70 C. under an atmosphere of argon. The solution was cooled to room temperature, filtered on a sintered funnel, washed with cold dioxane (2 mL) and dried under vacuum. The white solid compound 4•HI (R₂=2-chlorobenzyl) was obtained (242 mg, 72%) and used without further purification.

### Example 2. Synthesis of 2-Chlorobenzylamino-2-imidazoline

To a stirred solution of 2-chlorobenzylamino-2-imidazoline hydriodide (242 mg, 0.72 mMol) in water (3 mL), was added 1.0 N sodium hydroxide (2 mL) at 7 °C. The reaction mixture was stirred for 30 min at 7 °C under argon. After that methylene chloride (5 mL) was added and the mixture stirred for another 5 min. The reaction mixture was extracted with methylene chloride (2X 2.5 mL), The organic layer was dried over anhydrous Na₂SO₄, filtered and evaporated. The resulting free base (150 mg, 100%) was obtained as a viscous liquid and was used for the next reaction without any further purification. MS(ESI) 210(M+H).

### Reference Example 3. Synthesis of Methyl-1-benzyl 4-oxo-3-piperidine carboxylate (Compound (6)).

To a stirred methyl-1-benzyl 4-oxo-3-piperidine carboxylate hydrochloride (5.7 g, 20 mMol) in ethyl acetate (50 mL), was added triethylamine (6 mL) at 7 °C. The reaction mixture was stirred for 30 min at 7 °C under atmosphere of argon. The reaction mixture was extracted with ethyl acetate (2x50 mL) washed with water (50mL). The organic layer was dried over anhydrous Na₂SO₄, filtered and evaporated. The resulting free base residue (5, Ri=benzyl) as a viscous oil was used in the next reaction without any further purification MS(ESI) 248(M+H)

### Reference Example 4. Synthesis of ONC902 (Compound (14))

To a solution of 2-chlorobenzylamino-2- imidazoline (150 mg, 0.72 mMol ), methyl 1-benzyl 4- oxo-3-piperidine carboxylate (5, Ri=benzyl) (195 mg, 0.79 mMol ) in 1-butanol (2 mL ) was added PPTS (10 mg) and the mixture was stirred at room temperature for 48 h. After that the reaction mixture was refluxed at 125 °C to 130 °C for 2h. The solvents were removed under vacuum, extracted with ethyl acetate (10 mL), washed with saturated sodium bicarbonate solution (2 x10 mL) and water (10 mL). The organic layer was dried over anhydrous Na₂SO₄, filtered and evaporated. The crude free base was purified by RP HPLC (10%-40% acetonitrile/water) to give ONC902 TFA salt as a white solid (228 mg, 50% yield) MS(ESI) 407 (M+H).

The same process was used starting with different benzylamines to prepare various analogs, e.g., ONC903, 904, 905, and 906.

### Reference Example 5. Synthesis of ONC907 (Compound (19))

To a suspension of 60% sodium hydride (3.5 g, 88 mMol) in dry toluene (50 mL), dimethyl carbonate (4.32 g, 48.0 mMol) was added dropwise in 0.5 h at room temperature under an atmosphere of nitrogen. After addition of a few drops of methanol, a solution of 1-tert-butoxycarbonyl-4-piperidone (4.8 g, 24 mMol) dissolved in dry toluene (20 mL) was added dropwise to the reaction mixture while stirring at 80 °C over 1h. The reaction mixture was stirred for 3 h at the same temperature and then cooled to 0 °C (ice bath) and adjusted to pH 6-6.5 with acetic acid. The resulting cold mixture was diluted with water (10mL) and adjusted to pH 8 with 5% sodium hydroxide solution. The toluene layer was separated and the aqueous layer was extracted with toluene (20 mL). The combined organic layer was dried over anhydrous sodium sulfate, and concentrated under reduced pressure The compound was dried in vacuum to give methyl-1-tert-butoxycarbonyl- 4-oxo-3-piperidine carboxylate (5.0 g, 80%). The compound obtained was carried to next reaction without any further purification.

2-methybenzylamino-2-imidazoline (190 mg, 1 mMol ), methyl 1-tert-butoxycarbonyl- 4-oxo-3-piperidine carboxylate (315 mg, 1.1 mMol ) in 1-butanol (2 mL ) was added PPTS (10.0 mg) and the mixture was stirred at room temperature for 48 h. After that the reaction mixture was refluxed at 125 °C to 130 °C for 2h. The solvents were removed under vacuum, extracted with ethyl acetate (10 mL), washed with saturated sodium bicarbonate solution (2x10 mL) and water (10 mL). The organic layer was dried over anhydrous Na₂SO₄, filtered and evaporated. The crude free base was cleaved with 10% trifluoroacetic acid in dichloromethane, purified by RP HPLC (10%-40% acetonitrile/water) to give ONC907 (262 mg, 50%) TFA salt as a white solid MS(ESI) 297 (M+H).

### Reference Example 6. Synthesis of ONC909 (Compound 21)

A mixture of ONC907 (100 mg, 0.2 mMol), phenylethyl bromide (55.0 mg, 0.28mMol) and potassium carbonate (150 mg, 1.0 mMol ) in N,N-dimethylformamide (3 mL) was heated to 70 °C for 12 h. The solvents were removed under vacuum, extracted with ethyl acetate (10 mL), washed with water (5 mL). The organic layer was dried over anhydrous Na₂SO₄, filtered and evaporated. The crude free base was purified by RP HPLC (10%-40% acetonitrile/water) to give ONC909 (62 mg, 50%) TFA salt as a white solid MS(ESI) 401 (M+H).

### Reference Example 7. Synthesis of ONC908 (Compound 20)

To a solution of 2-methylbenzylamino-2-imidazoline (190.0 mg, 1.0 mmol), methyl 1-methyl 4-oxo-3-piperidine carboxylate (185.0 mg, 1.0 mMol ) in 1-butanol (2.0 mL) was added PPTS (10.0 mg) and the mixture was stirred at room temperature for 48 h. After that the reaction mixture was refluxed at 125 °C to 130 °C for 2h. The solvents were removed under vacuum, extracted with ethyl acetate (10 mL), washed with saturated sodium bicarbonate solution (2 x10 ml) and water (10 mL). The organic layer was dried over anhydrous Na₂SO₄, filtered and evaporated. The crude free base was purified by HPLC 10%-40% acetonitrile and water to give ONC908 (270.0 mg., 50%) TFA salt as a white solid MS(ESI) 311 (M+H).

### Example 8. Synthesis of ONC201 (Compound 1)

To a stirred 800 mL saturated NaHCO₃ in a 2 L round bottom flask, compound **(3)** (239.7 g, 0.845 mol, 1.6 equiv) was added in portions. n-Butanol (500 mL) was added to the resulting mixture and the mixture was stirred for 30 min and the**n** transferred to a separating funnel. The organic phase, containing compound **(4),** was separated and transferred to a 2 L three-neck round bottom flas**k** equipped with mechanical stirring, N₂ inlet, a thermocouple, a condenser and a Dean-Stark trap. Compound **(5)** (100 g, 0.528 mol, 1 equiv) and pyridinium p-toluenesulfonate (PPTS) (6.63 gm 0.026 mol, 5 mol%) were added to the contents of the flask. The resulting mixture was heated to reflux for 6 hours. Water in the **reacti**on mixture was separated into the Dean-Stark trap as necessary. Refluxing temperature increased from 93 ^{°}C to 118 ^{°}C. Reaction progress was monitored by HPLC. When the peak area of compound **(1)** on HPLC remained constant with the reaction time, the reaction was stopped.

### Example 9. Synthesis of Di-Salt of ONC201 (Compound (2))

Without isolation of the compound **(1),** the reaction mixture from EXAMPLE 8 was washed with 500 mL of water and diluted with methyl tert-butyl ether (MTBE) (800 mL). The organic phase was washed with water (500 mL × 2) and transferred to a 3 L three-neck round bottom flask equipped with mechanical stirring, N2 inlet, a thermocouple, a condenser and a Dean-Stark trap. While agitating the reaction mixture, 1 N HCl in dioxane-MTBE solution was added dropwise (4 N HCl in dioxane: 300 mL, 1.2 mol, 2.27 equiv; MTBE: 1200 mL) until no more solid precipitated out of the reaction mixture upon addition of HCl. The reaction mixture was heated to reflux at 60-65 °C for 2 hours. Water was separated into the Dean-Stark trap as necessary. Upon cooling to room temperature, the solid precipitate was filtered through a sintered glass funnel and washed with n-butanol-MTBE (1: 2, 600 mL) and MTBE (600 mL) respectively. The solid was dried in the vacuum oven at 65°C overnight (16 hours) to afford 200 g yellow solid.

To a 2 L three-neck round bottom flask equipped with mechanical stirring, N2 inlet, a thermocouple and a condenser, the above solid (200 g) was added, followed by ethanol (1000 mL). The mixture was heated to reflux at 78°C for 2 hours. Upon cooling to room temperature, the solid was filtered through a sintered glass funnel and washed with ethanol (200 mL × 3). The wet solid was dried in the vacuum oven at 85°C for 3 days until the residual solvent met specification. 120 g of compound (2) was obtained as a white solid in a yield of 49%, with HPLC purity 99.7%.

It will be appreciated by those skilled in the art that changes could be made to the exemplary embodiments shown and described above. It is understood, therefore, that this invention is not limited to the exemplary embodiments shown and described, but it is intended to cover modifications within the scope of the present invention as defined by the claims.

## Claims

1. A compound: 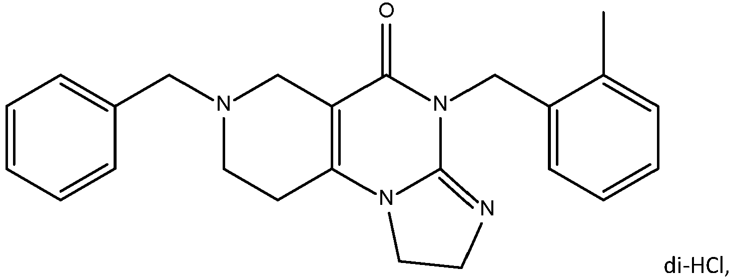 for use in the treatment of one or more diseases selected from the group consisting of brain cancer, glioblastoma, astrocytoma, atypical teratoid tumors, atypical rhabdoid tumors, brain tumors, spinal cord tumors, central nervous system atypical teratoid tumors, central nervous system atypical rhabdoid tumors, leptomeningeal disease, central nervous system embryonal tumors, central nervous system lymphoma, chordoma, craniopharyngioma, embryonal tumors of central nervous system, ependymoblastoma, ependymoma, extracranial germ cell tumors, extragonadal germ cell tumors, germ cell tumors, glioma, medulloblastoma, medulloepithelioma, neuroblastoma, pineal parenchymal tumors of intermediate differentiation, pineoblastoma, supratentorial primitive neuroectodermal tumors, pituitary tumors, and primary central nervous system lymphoma.

2. The compound for use of claim 1, wherein the treatment is for one or more diseases selected from the group consisting of brain cancer, glioblastoma, astrocytoma, atypical teratoid tumors, atypical rhabdoid tumors, brain tumors, craniopharyngioma, ependymoblastoma, glioma, and medulloblastoma.

3. The compound for use of claim 1 or claim 2, wherein the disease is a brain tumor.

4. The compound for use of any one of claims 1 - 3, wherein the treatment is for an adult patient.

5. The compound for use of any one of claims 1 - 4, wherein the treatment is for a pediatric patient.

## Patentansprüche

1. Verbindung: 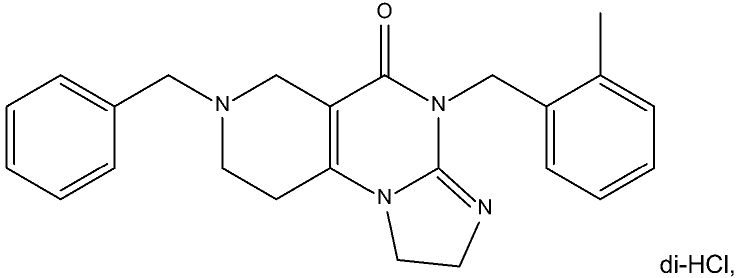 zur Verwendung bei der Behandlung einer oder mehrerer Krankheiten, ausgewählt aus der Gruppe besehend aus Gehirnkrebs, Glioblastom, Astrozytom, atypischen teratoiden Tumoren, atypischen rhabdoiden Tumoren, Gehirntumoren, Rückenmarkstumoren, atypischen teratoiden Tumoren des Zentralnervensystems, atypischen rhabdoiden Tumoren des Zentralnervensystems, leptomeningealer Erkrankung, embryonalen Tumoren des Zentralnervensystems, Lymphom des Zentralnervensystems, Chordom, Kraniopharyngeom, embryonalen Tumoren des Zentralnervensystems, Ependymoblastom, Ependymom, extrakraniellen Keimzelltumoren, extragonadalen Keimzelltumoren, Keimzelltumoren, Gliom, Medulloblastom, Medulloepitheliom, Neuroblastom, Pinealisparenchymtumoren intermediärer Differenzierung, Pineoblastom, supratentoriellen primitiven neuroektodermalen Tumoren, Hypophysentumoren und primärem Lymphom des Zentralnervensystems.

2. Verbindung zur Verwendung nach Anspruch 1, wobei die Behandlung für eine oder mehrere Krankheiten ist, ausgewählt aus der Gruppe bestehend aus Gehirnkrebs, Glioblastom, Astrozytom, atypischen teratoiden Tumoren, atypischen rhabdoiden Tumoren, Gehirntumoren, Kraniopharyngeom, Ependymoblastom, Gliom und Medulloblastom.

3. Verbindung zur Verwendung nach Anspruch 1 oder 2, wobei die Erkrankung ein Gehirntumor ist.

4. Verbindung zur Verwendung nach einem der Ansprüche 1 bis 3, wobei die Behandlung für einen erwachsenen Patienten ist.

5. Verbindung zur Verwendung nach einem der Ansprüche 1 bis 4, wobei die Behandlung für einen pädiatrischen Patienten ist.

## Revendications

1. Composé : 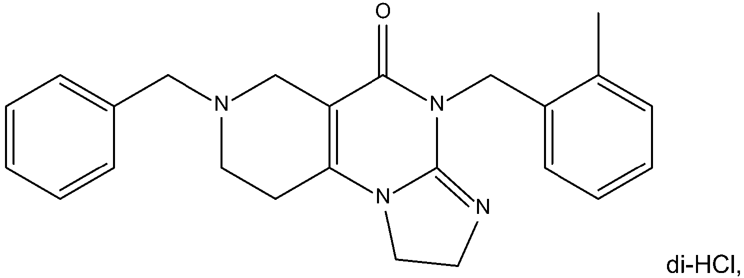 destiné à être utilisé dans le traitement d'une ou de plusieurs maladies choisies dans le groupe constitué de cancer du cerveau, glioblastome, astrocytome, tumeurs tératoïdes atypiques, tumeurs rhabdoïdes atypiques, tumeurs cérébrales, tumeurs de la moelle épinière, tumeurs tératoïdes atypiques du système nerveux central, tumeurs rhabdoïdes atypiques du système nerveux central, maladie leptoméningée, tumeurs embryonnaires du système nerveux central, lymphome du système nerveux central, chordome, craniopharyngiome, tumeurs embryonnaires du système nerveux central, épendymoblastome, épendymome, tumeurs germinales extracrâniennes, tumeurs germinales extragonadiques, tumeurs germinales, gliome, médulloblastome, médulloépithéliome, neuroblastome, tumeurs du parenchyme pinéal à différenciation intermédiaire, pinéoblastome, tumeurs neuroectodermiques primitives sus-tentorielles, tumeurs hypophysaires et lymphome primitif du système nerveux central.

2. Composé destiné à être utilisé selon la revendication 1, dans lequel le traitement est destiné à une ou plusieurs maladies choisies dans le groupe constitué de cancer du cerveau, glioblastome, astrocytome, tumeurs tératoïdes atypiques, tumeurs rhabdoïdes atypiques, tumeurs cérébrales, craniopharyngiome, épendymoblastome, gliome et médulloblastome.

3. Composé destiné à être utilisé selon la revendication 1 ou la revendication 2, dans lequel la maladie est une tumeur cérébrale.

4. Composé destiné à être utilisé selon l'une quelconque des revendications 1 à 3, dans lequel le traitement est destiné à un patient adulte.

5. Composé destiné à être utilisé selon l'une quelconque des revendications 1 à 4, dans lequel le traitement est destiné à un patient de pédiatrie.
